# FASCICULE DE BREVET EUROPEEN

(11) **EP 3 152 228 B1**
(45) Date de publication et mention de la délivrance du brevet: **04.05.2022**
(21) Numéro de dépôt: 15736537.0
(22) Date de dépôt: 03.06.2015
(51) Int. Cl.: C07K 14/415, C12N 15/82

(54) **UTILISATION DE MICROPEPTIDES POUR FAVORISER LA SYMBIOSE MYCORHIZIENNE**
VERWENDUNG VON MIKROPEPTIDEN ZUR STIMULIERUNG VON MYKORRHIZALER SYMBIOSE
USE OF MICROPEPTIDES IN ORDER TO STIMULATE MYCORRHIZAL SYMBIOSIS

(30) Priorité: 03.06.2014 FR 1455047; 24.03.2015 FR 1552462
(43) Date de publication de la demande: 12.04.2017
(73) Titulaire: Université Toulouse III-Paul Sabatier, 31062 Toulouse Cedex 9 (FR); Centre National de la Recherche Scientifique, 75794 Paris Cedex 16 (FR)
(72) Inventeur: COMBIER, Jean-Philippe, 31320 Castanet Tolosan (FR); LAURESSERGUES, Dominique, 31000 Toulouse (FR); BECARD, Guillaume, 31450 Odars (FR)
(74) Mandataire: Grosset-Fournier, Chantal Catherine
(86) Numéro de dépôt international: PCT/FR2015/051473
(87) Numéro de publication internationale: WO 2015/185862

(56) Documents cités:
- WO-A1-2015/063431
- XUE-YI XUE ET AL: "Interaction between Two Timing MicroRNAs Controls Trichome Distribution in Arabidopsis", PLOS GENETICS, vol. 10, no. 4, 3 avril 2014 (2014-04-03), page e1004266, XP055167292, DOI: 10.1371/journal.pgen.1004266
- DOMINIQUE LAURESSERGUES ET AL: "The microRNA miR171h modulates arbuscular mycorrhizal colonization of Medicago truncatula by targeting NSP2", THE PLANT JOURNAL, vol. 72, no. 3, 30 août 2012 (2012-08-30), pages 512-522, XP055128000, ISSN: 0960-7412, DOI: 10.1111/j.1365-313X.2012.05099.x
- BARI ASSYL ET AL: "miR156-and miR171-Binding Sites in the Protein-Coding Sequences of Several Plant Genes", BIOMED RESEARCH INTERNATIONAL, 2013, XP002735648,
- SUNKAR RAMANJULU ET AL: "Identification of novel and candidate miRNAs in rice by high throughput sequencing", BMC PLANT BIOLOGY, BIOMED CENTRAL, LONDON, GB, vol. 8, no. 1, 29 février 2008 (2008-02-29), page 25, XP021033845, ISSN: 1471-2229
- BARDOU F ET AL: "Dual RNAs in plants", BIOCHIMIE, MASSON, PARIS, FR, vol. 93, no. 11, 1 novembre 2011 (2011-11-01), pages 1950-1954, XP002734738, ISSN: 0300-9084, DOI: 10.1016/J.BIOCHI.2011.07.028 [extrait le 2011-07-31]
- JEROEN CRAPPÉ ET AL: "Little things make big things happen: A summary of micropeptide encoding genes", EUPA OPEN PROTEONOMICS, ELSEVIER BV, NL, vol. 3, 1 juin 2014 (2014-06-01), pages 128-137, XP002734739, ISSN: 2212-9685, DOI: 10.1016/J.EUPROT.2014.02.006 [extrait le 2014-03-06]
- DE CONINCK B ET AL: "Mining the genome of Arabidopsis thaliana as a basis for the identification of novel bioactive peptides involved in oxidative stress tolerance", JOURNAL OF EXPERIMENTAL BOTANY, OXFORD UNIVERSITY PRESS, GB, vol. 64, no. 17, 1 décembre 2013 (2013-12-01), pages 5297-5307, XP002734740, ISSN: 0022-0957, DOI: 10.1093/JXB/ERT295 [extrait le 2013-09-16]

### SEQUENCE LISTING

<110> Universit Toulouse III - Paul Sabatier Centre National de la Recherche Scientifique - CNRS
<120> UTILISATION DE MICROPEPTIDES POUR FAVORISER LA SYMBIOSE MYCORHIZIENNE
<130> WOB 15 TTT M171
<150> FR 14 55047
   <151> 2014-06-03
<150> FR 15 52462
   <151> 2015-03-24
<160> 16
<170> PatentIn version 3.5
<210> 1
   <211> 21
   <212> RNA
   <213> Medicago truncatula
<400> 1
   ugauugagcc gcgucaauau c 21
<210> 2
   <211> 20
   <212> PRT
   <213> Medicago truncatula
<400> 2
   Met Leu Leu His Arg Leu Ser Lys Phe Cys Lys Ile Glu Arg Asp Ile
   1               5                   10                  15
   Val Tyr Ile Ser
               20
<210> 3
   <211> 63
   <212> DNA
   <213> Medicago truncatula
<400> 3
   atgcttcttc ataggctctc caaattttgc aaaattgaaa gaggcatagt atatatatct       60
   tag                                                                      63
<210> 4
   <211> 728
   <212> DNA
   <213> Medicago truncatula
<400> 4
   attggtcaaa catacataca gtagcactag ctggtttcat tattccacta tgcttcttca       60
   taggctctcc aaattttgca aaattgaaag aggcatagta tatatatctt agcaaggaga      120
   aattcaggat attgaggatg aagattgaag agtaatcagt gatgaagaaa gcaagcaagg      180
   tattggcgcg cctcaatttg aatacatggc tataaaaatg catcatatca gccatgtagt      240
   ttgattgaac cgcgtcaata tcttgtttcc atctccaaat ttaccaatct catcaaatca      300
   aattaacacc acaatcaagt caaaataggt tgaccttatc atcgaagaaa ttgttttctc      360
   attcctatcg aagttggact tgctgaaaat gctcgaaagc atgtgtttta gttcgacagg      420
   cgaaaaagtt accgaaggac aatttggttg tggttcggat aaggtcaagc aacggatatt      480
   ttcaagacac gttcgaaatt caagtcaaat ggataagtat cgttagttta ctgcagttat      540
   agttttaaat tcaaatctag gcagttattt ctatttgtat aaatagtagt ttttccctag      600
   ggaaaaaggg tcgcaattca atcatacaaa aaacttacaa tcaaattatc cgcatggaag      660
   agagaaacga gtcacaagtt gcaatgtatg aacatgtgta ccaatttaca ttcaatcagt      720
   acaatttt                                                               728
<210> 5
   <211> 21
   <212> RNA
   <213> Solanum lycopersicum
<400> 5
   uugagccgcg ucaauaucuc u 21
<210> 6
   <211> 19
   <212> PRT
   <213> Solanum lycopersicum
<400> 6
   Met Lys Leu Gly Asn Ile Glu Gly Thr Tyr Phe Ile Ile Cys Leu Gly
   1               5                   10                  15
   Arg Tyr Ile
<210> 7
   <211> 60
   <212> DNA
   <213> Solanum lycopersicum
<400> 7
   atgaagttgg gaaatattga aggtacgtac tttataatat gtttaggaag atatatatag 60
<210> 8
   <211> 1000
   <212> DNA
   <213> Solanum lycopersicum
<400> 8
   gtctttgagg gcatgaatag tgtaatatgg acccattcgt gtgtggaaaa attgtacaat      60
   attccaagaa aaaacgtacc tgtccacatt aattagttag gtaagtggat tatactactc     120
   ataaaatatt atattacagc caaaggaatc caacttctct aaaataaaaa taaaaaacac     180
   caaattaatg tttgtttcaa tattttgatg tacattactt tagagcaagt attagcaaga     240
   tacattaggc tattttttgt tttttcggaa tcacatcaag aaatgaaaga tctttggtca     300
   caacatgatg aagatacata gttgaaactt gaaagatata atagttttga tttttcgtat     360
   tgaaaattat tctgcaaatt gatggatagc tagctaattc aaagatgaag ttgggaaata     420
   ttgaaggtac gtactttata atatgtttag gaagatatat atagatattg atgcggttca     480
   atctgaaaga catggttaga tatgtaatta gccttgtaat tttggattga gccgcgtcaa     540
   tatctctctt cctattttca attagtttat aagtaacttg aactttattt aattactcgt     600
   tggtaatact tgtcttgttt catgttttcc tcttggccat gcatctttaa tgtttttttt     660
   tccactaatt ttctggtttt taattagttt ttaaatttct tcttaatttc atctttgaca     720
   ccattaattt cattcgttgc cgttcgttga tagacgtttt tgaattagat agttaatcat     780
   aattaataca tgaatgaatc gatcaagagc tagcatggaa attaatgatt gtgttgaatg     840
   attttttttt gtattatttt ttgcttctta aggtaattgc aacctacttt tgcttatata     900
   ttttctttta ttatttgaat ttcgaggtat tttcactacc tcaaataatt acctttcttc     960
   ctgatttcaa ttttctttca taaaaaagcg tctttctaaa                          1000
<210> 9
   <211> 21
   <212> RNA
   <213> Lotus japonicus
<400> 9
   ugauugagcc gcgucaauau c 21
<210> 10
   <211> 22
   <212> PRT
   <213> Lotus japonicus
<400> 10
   Met Tyr His Arg Ser Lys Ala Lys Leu Cys Gln Thr Asp Gly Asp Asp
   1               5                   10                  15
   Gly Gly Gly Ser Asp Met
               20
<210> 11
   <211> 69
   <212> DNA
   <213> Lotus japonicus
<400> 11
   atgtatcatc gaagcaaagc aaaactatgt caaactgatg gtgatgatgg aggaggaagt      60
   gatatgtga                                                              69
<210> 12
   <211> 1220
   <212> DNA
   <213> Lotus japonicus
<400> 12
   gagaaataca aaaacccaac aaaacccaac tcaaaatatt attctccacc aagcagaaac      60
   cagatttcat ctcgttacag tatcaccata gtccacgccc aactccaaaa gccatgccac     120
   aagccaaaac aaaacgtcag gtacagatca caggacaaaa tagctttttt ccaatcagcc     180
   caagtataca aagcaaagat gatataaagt ggtccatgat ggtgggtgta attcgtatta     240
   acaacttaat taatatgtac attaatgtac catctaacgg gtatatggaa attcagtctc     300
   tttcatgacc ttatatttcg cctcaatgcc tcattatagg catccatcca tgcagatgaa     360
   attccaataa caacctcccc gtcttaattc aattgagaca atattttgtt gggatttgca     420
   aggcacttag aaaataattt ttgaaacaca aacaaagcca cttgatagag tcatagaggt     480
   agtagaacag aaacacttgg cgattgagtt ttgttttttt aaagtatttt tcgaaattga     540
   ggaggaaaac taattaatat ttgcgctcat tcgtgggaaa agtggtcgtt ggtcaaagag     600
   tcgcacaaat attatttcag cacttgtggt ttggtttaat ggttttgatg aattcattcc     660
   actttgctta ttcccccagt ctgcaacaca aaatgatgtg ttttccaaaa ttaaagacag     720
   atctcaaaag caggtgttag ttaatttcct catgtatcat cgaagcaaag caaaactatg     780
   tcaaactgat ggtgatgatg gaggaggaag tgatatgtga agcacaaatt aacaaggtat     840
   tgacgcgtct caatttgaag acatggctgg ctaacatgaa aaccaatcat gtagtttgat     900
   tgagccgcgt caatatcttg cttttgcgta cttctttcca tgtccatctg gccaccaatc     960
   tcaactcaaa cgcccaccgg tatgtataat cattattgaa tgcttaattg tgttctctta    1020
   attttgtata atctatattc atagtactgt ttctctttct gcagaaaatc aaaatgcgtt    1080
   atacatcaga agtaggtaaa attgacgtat caggtcagcc atcacagctt gcaccaatca    1140
   ttattatatt gtattatctg atatgcacaa attaatggaa attataaaat cctttaaatg    1200
   gtatagtata ggtgtggaaa                                                1220
<210> 13
   <211> 21
   <212> RNA
   <213> Oryza sativa
<400> 13
   ggauugagcc gcgucaauau c 21
<210> 14
   <211> 31
   <212> PRT
   <213> Oryza sativa
<400> 14
   Met Ile Ala Arg Tyr Ile Glu Arg Glu Met Thr Ser Lys Leu Gly Arg
   1               5                   10                  15
   Gly Arg Lys Arg Ala Ala Arg Leu Val Ala Val Phe Leu Leu Gly
               20                  25                  30
<210> 15
   <211> 96
   <212> DNA
   <213> Oryza sativa
<400> 15
   atgatagcta gatatatcga gagggagatg accagtaagc ttggaagagg aagaaagaga        60
   gctgctaggc ttgttgcggt ctttttgctt gggtaa                                  96
<210> 16
   <211> 790
   <212> DNA
   <213> Oryza sativa
<400> 16
   agaagaatgg ttgtgattga ttgagaggag gaggtaggtg aagaaatagc ttcattttag      60
   gacaagacac tgtgctaaaa atagctatat tttaggacgg agagagtaga taagctagtc     120
   ccgacccccc tcctctcctc tcttgccccg cctatataat ccccaaacat cgctttctct     180
   tggagtagga gaagggtagt agtttaagct atagctctaa agacatcacc atgcaacatt     240
   acttgcgtta ttactatacc tatccaccac ctacacattt tgtcatctcc ctctctcttt     300
   ctctctgatc tgtctcagat gtttatgcac atatacaagt taatagttct gtggatctag     360
   caatcccggc ttgtttgttt ttgctgcttt ggtttgatga tagctagata tatcgagagg     420
   gagatgacca gtaagcttgg aagaggaaga aagagagctg ctaggcttgt tgcggtcttt     480
   ttgcttgggt aaaaagaggt attggcgtgc ctcaatccga aggcatggct gattacaggc     540
   acctcgaccg atctagcgca tgcagccatg tttcttggat tgagccgcgt caatatctct     600
   ccttgcttcc ttacttcatg tactgtgtca tgctcaagca tatgtcccct ctccgatctt     660
   cctacctcgt cgagttcgtc ggatcagttc ccaaattaaa ggtgttatat atatatatat     720
   atatatatat atatatatat atatatatat atatatatag taaatttgtt tggtgctcca     780
   tggtgcccgg                                                            790

## Description

La présente invention concerne l'utilisation de micropeptides (peptides codés par des microARNs ou « miPEPs ») pour favoriser la symbiose mycorhizienne entre une plante et un champignon.

La symbiose mycorhizienne est un processus biologique ancien et fréquent dans la nature. La symbiose mycorhizienne à arbuscules (AM) date en effet de plus de 400 millions d'années et associe la plupart des plantes terrestres et des champignons Gloméromycètes. Cette symbiose améliore la nutrition hydrique et minérale des plantes, mais également leur résistance à différents stress biotiques et abiotiques.

De manière générale, les champignons mycorhiziens, incapables de photosynthèse, sont dépendants de la plante qu'ils colonisent pour récupérer des substances carbonées. En retour, les champignons mycorhiziens fournissent à la plante des substances minérales et de l'eau qu'ils sont capables de capter dans le sol.

Les symbioses mycorhiziennes peuvent donc profiter à la croissance et à la protection des plantes contre divers stress biotiques et abiotiques. La symbiose mycorhizienne peut également stimuler la croissance végétale, tout en réduisant considérablement les besoins en engrais des cultures. Le mycélium constitue d'ailleurs une extension du système racinaire des plantes qui permet à celles-ci de mieux exploiter l'eau et les minéraux d'un volume de sol étendu. De telles plantes mieux nourries et en meilleure santé résistent davantage aux stress environnementaux tels que la sécheresse, et survivent mieux aux attaques de pathogènes.

Les microARNs (miRs) sont des petits ARNs non codants, d'environ 21 nucléotides après maturation, qui contrôlent l'expression de gènes cibles au niveau post-transcriptionnel, en dégradant l'ARNm cible ou en inhibant sa traduction. Les miRs sont notamment rencontrés chez les plantes.

Les gènes ciblés par les miRs sont souvent des gènes clés de processus développementaux. Par exemple, le miR171b cible des facteurs de transcription de la famille GRAS, spécifiques des plantes, qui sont connus pour intervenir dans le fonctionnement des méristèmes (Stuurman et al., Genes Dev, 16:2213-8, 2002 ; Engstrom et al., Plant Signal Behav, 20116:850-4). Par ailleurs, cette famille comprend des gènes régulateurs clés du développement racinaire comme les gènes *Scarecrow* et *Shortroot.*

La mycorhization étant intimement liée au développement racinaire, il a d'ailleurs été mis en évidence récemment que le miR171h régule la mycorhization à arbuscules chez *Medicago truncatula* par le biais d'une régulation négative du gène *NSP2,* limitant la surcolonisation des racines par les champignons mycorhiziens (Lauressergues et al., Plant journal, 75:512-22, 2012).

La régulation de l'expression des miRs est très peu connue, mais il a été démontré que celle-ci fait intervenir, à l'instar de la plupart des gènes codants, une ARN polymerase II : cette enzyme produit un transcrit primaire, appelé « *pri*-*miR* », qui est ensuite maturé par un complexe protéique contenant notamment les enzymes type Dicer. Cette maturation conduit tout d'abord à la formation d'un précurseur de miR appelé « *pre-miR* », ayant une structure secondaire en forme tige-boucle contenant le *miR* et sa séquence *miR*^{∗} complémentaire. Puis le précurseur est maturé, ce qui conduit à la formation d'un ARN double brin plus court contenant le miR et le miR^{∗}. Le miR est ensuite pris en charge par le complexe RISC qui clive l'ARNm du gène cible ou bien inhibe sa traduction.

Jusqu'à présent, les miRs, et par extension leur transcrit primaire, ont toujours été considérés, de par leur mode d'action particulier, comme des ARNs régulateurs non codants et ne produisant aucun peptide. Or, les Inventeurs ont récemment mis en évidence dans la demande de brevet FR 13/60727 l'existence de micropeptides (ou « miPEPs », *microRNA encoded PEPtides)* capables de moduler l'accumulation des miRs.

Dans ce contexte, la présente invention a pour but de proposer de nouveaux outils efficaces et écologiques pour favoriser la symbiose entre une plante et un champignon.

L'un des aspects de l'invention est de proposer une nouvelle utilisation des miPEPs pour favoriser symbiose mycorhizienne entre une plante et un champignon.

Un autre aspect de l'invention concerne également un nouveau procédé de culture de plantes en symbiose avec un champignon.

Un autre aspect de l'invention est de proposer une composition de miPEPs permettant de favoriser symbiose mycorhizienne entre une plante et un champignon.

L'un des autres aspects de l'invention est également de proposer une plante transgénique et des parties de plantes transgéniques, et leur procédé de production.

L'un des autres aspects de l'invention est également de proposer des organes, cellules et semences de plantes transgéniques.

L'un des autres aspects de l'invention est de proposer des plantes modifiées écologiquement.

L'un des autres aspects de l'invention est de proposer des inocula de champignons mycorhiziens.

L'invention concerne donc l'utilisation d'un peptide pour favoriser la symbiose mycorhizienne entre une plante et un champignon, et en particulier la symbiose mycorhizienne à arbuscules (AM), ledit peptide étant introduit dans la plante, ledit peptide ayant une séquence d'acides aminés comprenant ou consistant en une séquence identique à celle d'un miPEP naturellement présent dans ladite plante,
ledit miPEP naturellement présent dans ladite plante étant un peptide de 3 à 100 acides aminés, notamment de 4 à 100 acides aminés, dont la séquence est codée par un cadre ouvert de lecture situé sur le transcrit primaire d'un miR, ledit miPEP étant capable de moduler l'accumulation dudit miR dans ladite plante, lequel miR régule l'expression d'au moins un gène impliqué dans la symbiose mycorhizienne chez ladite plante.

De manière surprenante et inattendue, les Inventeurs ont constaté que l'utilisation de peptides dont la séquence comprend ou consiste en une séquence identique à celle de miPEPs codés sur les transcrits primaires de miRs, permet de favoriser la symbiose mycorhizienne entre une plante et un champignon.

Dans l'invention, les termes *« microARN », « microARN non codant »* et *« miR »* sont équivalents et peuvent être utilisés l'un pour l'autre. Ils définissent des petites molécules d'ARN d'environ 21 nucléotides, qui ne sont pas traduites et ne conduisent pas à un peptide ou une protéine. Cependant, sous cette forme mature, les miRs assurent une fonction de régulation de certains gènes *via* des mécanismes post-transcriptionnels, comme par exemple par l'intermédiaire du complexe RISC.

Le « *transcrit primaire de miR »* (ou *« pri-miR »)* correspond lui à la molécule d'ARN directement obtenue à partir de la transcription de la molécule d'ADN. Généralement, ce transcrit primaire subit une ou plusieurs modifications post-transcriptionnelles, qui entraînent par exemple une structure particulière de l'ARN ou un clivage de certaines parties de l'ARN par des phénomènes d'épissage, et qui conduisent à la forme précurseur du miR ou (*« pre-miR »),* puis à la forme mature du miR.

Les termes *« micropeptides »* et *« miPEPs » (microRNA encoded PEPtides)* sont équivalents et peuvent être utilisés l'un pour l'autre. Ils définissent un peptide qui est codé par un cadre ouvert de lecture présent sur le transcrit primaire d'un miR, et qui est capable de moduler l'accumulation dudit miR. Les miPEPs au sens de la présente invention ne doivent être entendus comme étant nécessairement des peptides de petite taille, dans la mesure où « micro » ne correspond pas à la taille du peptide.

Comme indiqué dans la demande de brevet FR 13/60727, dont le contenu doit être considéré comme faisant partie de la présente demande, les miPEPs sont des peptides :
- de 4 à 100 acides aminés, de préférence de 4 à 60 acides aminés, notamment de 4 à 59 acides aminés,
- codés par un cadre ouvert de lecture contenu dans le transcrit primaire d'un miR, de préférence dans la partie 5' du transcrit primaire dudit miR, et
- capables de moduler l'accumulation dudit miR dans une cellule eucaryote.

Les termes « *cadre ouvert de lecture »* ou *« ORF » (open readingframe)* sont équivalents et peuvent être utilisés l'un pour l'autre. Ils correspondent à une séquence de nucléotides dans une molécule d'ADN ou d'ARN pouvant potentiellement coder un peptide ou une protéine: ledit cadre ouvert de lecture débute par un codon start (le codon start codant généralement une méthionine), suivi d'une série de codons (chaque codon codant un acide aminé), et se termine par un codon stop (le codon stop n'étant pas traduit).

Dans l'invention, les ORFs peuvent être dénommés de manière spécifique « *miORFs »* lorsque ceux-ci sont présents sur les transcrits primaires de miR.

Les miORFs tels que définis dans l'invention peuvent avoir une taille de 15 à 303 nucléotides. Un acide aminé étant codé par un codon de 3 nucléotides, les miORFs de 15 à 303 nucléotides codent des miPEPS de 4 à 100 acides aminés.

En particulier, les miORFs ont une taille de :
15, 18, 21, 24, 27, 30, 33, 36, 39, 42, 45, 47, 51, 54, 57, 60, 63, 66, 69, 72, 75, 78, 81, 84, 87, 90, 93, 96, 99, 102, 105, 108, 111, 114, 117, 120, 123, 126, 129, 132, 135, 138, 141, 144, 147, 150, 153, 156, 159, 162, 165, 168, 171, 174, 177, 180, 183, 186, 189, 192, 195, 198, 201, 204, 207, 210, 213, 216, 219, 222, 225, 228, 231, 234, 237, 240, 243, 246, 249, 252, 255, 258, 261, 264, 267, 270, 273, 276, 279, 282, 285, 288, 291, 294, 297, 300 ou 303 nucléotides, et codent respectivement des miPEPs ayant une taille de : 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 ou 100 acides aminés.

Un miPEP peut également avoir une taille de 3 acides aminés.

Compte tenu de la dégénérescence du code génétique, un même miPEP peut être codé par plusieurs séquences nucléotidiques. De telles séquences nucléotidiques, différentes entre elles d'au moins un nucléotide mais codant un même peptide, sont appelées « *séquences dégénérées ».*

Dans l'invention, le terme « *plante* » fait référence de manière générale à tout ou partie d'une plante quel que soit son stade de développement (y compris la plante sous forme de graine ou de jeune pousse), à un ou plusieurs organes de la plante (comme par exemple les feuilles, les racines, la tige, les fleurs), à une ou plusieurs cellules de la plante, ou encore à un amas de cellules de la plante.

L'expression « *symbiose mycorhizienne* » fait référence à l'association symbiotique entre un champignon mycorhizien et les racines d'une plante. Dans l'invention, cette association symbiotique peut également être nommée « *mycorhization ».*

L'expression « *symbiose mycorhizienne à arbuscules* » fait référence à l'association symbiotique entre un champignon Gloméromycète et une plante. Les champignons mycorhiziens à arbuscules sont capables de pénétrer dans les racines de la plante et peuvent former des structures cellulaires particulières ramifiées dans les cellules végétales. De telles structures sont appelées arbuscules en raison de leur forme qui rappelle celle d'un petit arbre.

De manière non limitative, les paramètres permettant de déterminer et quantifier la symbiose mycorhizienne entre une plante et un champignon peuvent être notamment :
- la longueur et le nombre de racines de la plante,
- le pourcentage de colonisation d'une plante par le champignon,
- le nombre, la taille et l'aire des arbuscules,
- ou encore la croissance racinaire ou aérienne de la mycorhization.

Par ailleurs, dans l'invention, l'expression « *favoriser la* symbiose mycorhizienne», ou *« améliorer la symbiose mycorhizienne»,* indique :
- soit une accélération de la mycorhization (comme par exemple un pourcentage de colonisation plus important pour une plante à un instant donné par rapport à une plante de référence),
- soit à un accroissement de la mycorhization (comme par exemple un pourcentage de colonisation plus important pour une plante qui ne serait pas atteinte par une plante de référence cultivée dans les mêmes conditions),
- soit à une accélération et un accroissement de la mycorhization.

Il est important de noter que l'utilisation selon l'invention possède l'avantage d'être écologique, car le miPEP est un peptide qui est présent naturellement chez la plante.

L'invention concerne également l'utilisation d'un miPEP introduit par voie exogène dans une plante pour favoriser la symbiose mycorhizienne entre ladite plante et un champignon, en particulier la symbiose mycorhizienne à arbuscules,
ledit miPEP introduit par voie exogène étant un peptide comprenant, ou consistant en, une séquence identique à celle d'un miPEP naturellement présent dans ladite plante,
lequel miPEP naturellement présent est un peptide de 3 à 100 acides aminés, notamment de 4 à 100 acides aminés, dont la séquence est codée par un cadre ouvert de lecture situé en 5' sur le transcrit primaire d'un miR,
ledit miPEP étant capable de moduler l'accumulation dudit miR dans ladite plante, lequel miR régule l'expression d'au moins un gène impliqué dans la symbiose mycorhizienne,
la somme de la quantité dudit miPEP introduit par voie exogène et de celle dudit miPEP naturellement présent étant strictement supérieure à la quantité dudit miPEP naturellement présent.

Dans l'invention, l'expression « *miPEP introduit par voie exogène* » fait référence à un miPEP introduit artificiellement dans la plante que celui-ci existe ou non naturellement dans la plante.

Si le miPEP existe naturellement chez la plante, il s'agit d'un *« miPEP d'origine endogène ».*

Si le miPEP n'existe pas naturellement chez la plante, il s'agit d'un « *miPEP d'origine exogène ».*

Lorsqu'il est introduit dans la plante un « *miPEP d'origine exogène* », il est alors nécessaire d'introduire également le miR correspondant et son transcrit primaire.

L'introduction d'un miPEP par voie exogène dans la plante implique donc une étape technique, laquelle étape n'est pas un phénomène naturel et ne correspond ni à un croisement, ni à une sélection.

Le miPEP introduit par voie exogène peut être soit un peptide produit hors de la plante (comme par exemple un peptide isolé et/ou purifié, un peptide synthétique ou un peptide recombinant), soit un peptide produit dans la plante suite à l'introduction non naturelle d'un acide nucléique codant ledit miPEP dans ladite plante.

La plante dans laquelle le miPEP n'a pas été introduit possède une quantité basale dudit miPEP, qui correspond à celle dudit miPEP naturellement présent. L'utilisation d'un miPEP comprenant, ou consistant en, une séquence identique à celle dudit miPEP entraîne une augmentation de la quantité totale de miPEP, ce qui module l'accumulation du miR dont le transcrit primaire contient la séquence codant ledit miPEP.

Par ailleurs, le miPEP introduit se retrouve dans la plante et son introduction n'a pas d'impact sur sa stabilité.

Dans l'invention, par *« accumulation* », on entend la production d'une molécule, telle qu'un miR ou un miPEP, dans la cellule.

Ainsi, la *« modulation de l'accumulation* » d'une molécule dans une cellule correspond à une modification de la quantité de cette molécule dans la cellule.

Dans un mode de réalisation, l'invention concerne l'utilisation telle que définie ci-dessus, dans lequel la modulation de l'accumulation dudit miR est une diminution ou une augmentation de l'accumulation dudit miR, en particulier une augmentation.

Une *« diminution de l'accumulation du miR»* correspond à une baisse de la quantité de ladite molécule dans la cellule.

A l'inverse, une *« augmentation de l'accumulation du miR»* correspond à une hausse de la quantité de ladite molécule dans la cellule.

Dans un mode de réalisation, l'invention concerne l'utilisation telle que définie ci-dessus dans laquelle ledit gène impliqué dans la symbiose mycorhizienne code un facteur de transcription de la famille GRAS.

Dans un mode de réalisation, l'invention concerne l'utilisation telle que définie ci-dessus, dans laquelle ledit gène, impliqué dans la symbiose mycorhizienne, est choisi parmi le groupe consistant en: *HAM1* (Accession n°MTGI9-TC114268) et *HAM2* (Accession n°MTGI9-TC120850) (numéros d'accession selon la banque de données *Medicago truncatula* Gene Expression Atlas « MtGEA »).

Dans un mode de réalisation, l'invention concerne l'utilisation telle que définie ci-dessus, dans laquelle ledit miARN est le miR171b, en particulier, dans laquelle ledit miR171b possède une séquence nucléotidique consistant en SEQ ID NO : 1.

En particulier, l'invention concerne l'utilisation telle que définie ci-dessus, dans laquelle ledit miR171b possède une séquence nucléotidique présentant au moins 90 % d'identité, avec la séquence nucléotidique SEQ ID NO : 1.

Dans un mode de réalisation, l'invention concerne l'utilisation telle que définie ci-dessus, dans laquelle ledit miPEP est le miPEP171b, en particulier, dans laquelle ledit miPEP171b possède une séquence d'acides aminés consistant en SEQ ID NO : 2.

En particulier, l'invention concerne l'utilisation telle que définie ci-dessus, dans laquelle ledit miPEP171b possède une séquence d'acides aminés présentant au moins 80% d'identité, de préférence au moins 90 % d'identité, avec la séquence d'acides aminés SEQ ID NO : 2.

Dans un mode de réalisation, l'invention concerne l'utilisation telle que définie ci-dessus, dans laquelle ledit miARN est le slmiR171e, en particulier, dans laquelle ledit slmiR171e possède une séquence nucléotidique consistant en SEQ ID NO : 5.

En particulier, l'invention concerne l'utilisation telle que définie ci-dessus, dans laquelle ledit slmiR171e possède une séquence nucléotidique présentant au moins 90 % d'identité, avec la séquence nucléotidique SEQ ID NO : 5. Dans un mode de réalisation, l'invention concerne l'utilisation telle que définie ci-dessus, dans laquelle ledit miPEP est le slmiPEP171e, en particulier, dans laquelle ledit slPEP171e possède une séquence d'acides aminés consistant en SEQ ID NO : 6.

En particulier, l'invention concerne l'utilisation telle que définie ci-dessus, dans laquelle ledit slmiPEP171e possède une d'acides aminés présentant au moins 80% d'identité, de préférence au moins 90 % d'identité, avec la séquence d'acides aminés SEQ ID NO : 6.

Dans un mode de réalisation, l'invention concerne l'utilisation telle que définie ci-dessus, dans laquelle ledit miARN est le ljmiR171b, en particulier, dans laquelle ledit ljmiR171b possède une séquence nucléotidique consistant en SEQ ID NO : 9.

En particulier, l'invention concerne l'utilisation telle que définie ci-dessus, dans laquelle ledit ljmiR171b possède une séquence nucléotidique présentant au moins 80% d'identité, de préférence au moins 90 % d'identité, avec la séquence nucléotidique SEQ ID NO : 9.

Dans un mode de réalisation, l'invention concerne l'utilisation telle que définie ci-dessus, dans laquelle ledit miPEP est le ljmiPEP171b, en particulier, dans laquelle ledit ljPEP171b possède une séquence d'acides aminés consistant en SEQ ID NO : 10.

En particulier, l'invention concerne l'utilisation telle que définie ci-dessus, dans laquelle ledit ljmiPEP171b possède une d'acides aminés présentant au moins 80% d'identité, de préférence au moins 90 % d'identité, avec la séquence d'acides aminés SEQ ID NO : 10.

Dans un mode de réalisation, l'invention concerne l'utilisation telle que définie ci-dessus, dans laquelle ledit miARN est le osmiR171i, en particulier, dans laquelle ledit osmiR171i possède une séquence nucléotidique consistant en SEQ ID NO : 13.

En particulier, l'invention concerne l'utilisation telle que définie ci-dessus, dans laquelle ledit osmiR171i possède une séquence nucléotidique présentant au moins 80% d'identité, de préférence au moins 90 % d'identité, avec la séquence nucléotidique SEQ ID NO : 13.

Dans un mode de réalisation, l'invention concerne l'utilisation telle que définie ci-dessus, dans laquelle ledit miPEP est le osmiPEP171i, en particulier, dans laquelle ledit osmiPEP171i possède une séquence d'acides aminés consistant en SEQ ID NO : 14.

En particulier, l'invention concerne l'utilisation telle que définie ci-dessus, dans laquelle ledit osmiPEP171i possède une séquence d'acides aminés présentant au moins 80% d'identité, de préférence au moins 90 % d'identité, avec la séquence d'acides aminés SEQ ID NO : 14.

Dans un mode de réalisation, l'invention concerne l'utilisation telle que définie ci-dessus, dans laquelle ladite plante est une plante Monocotylédone telle que Oryza sativa (riz), une plante Dicotylédone, une plante légumineuse telle que *Glycine max* (soja), *Medicago truncatula* et *Medicago sativa* (luzerne) ou une plante solanacée telle que *Nicotiana benthamiana* (tabac), *Solanum tuberosum* (pomme de terre), *Solanum lycopersicum* (tomate) ou *Solanum melongena* (aubergine).

Dans un mode de réalisation, l'invention concerne l'utilisation telle que définie ci-dessus, dans laquelle ladite plante est *Medicago truncatula, Medicago sativa* (luzerne), *Solanum lycopersicum* (tomate), *Lotus japonicus* (lotier) ou *Oryza sativa* (riz).

Dans un mode de réalisation, l'invention concerne l'utilisation telle que définie ci-dessus, dans laquelle ladite plante est choisie parmi *Medicago truncatula, M. sativa* et *Glycine max* (soja).

Dans un mode de réalisation, l'invention concerne l'utilisation telle que définie ci-dessus, dans laquelle ladite plante est *Medicago truncatula.*

Dans un mode de réalisation, l'invention concerne l'utilisation telle que définie ci-dessus, dans laquelle ledit champignon est un champignon endomycorhizien ou ectomycorhizien, de préférence endomycorhizien.

Dans un mode de réalisation, l'invention concerne l'utilisation telle que définie ci-dessus, dans lequel ledit champignon est un champignon Gloméromycète ou Basidiomycète ou Ascomycète, de préférence Gloméromycète.

De manière non limitative, un champignon mycorhizien peut par exemple être choisi parmi la liste indiquée ci-dessous :
- champignons ectomycorhiziens :
   - *Tuber sp.*
   - *Boletus sp.*
   - *Lactarius sp.*
   - *Cantharellus sp.*
- champignons endomycorhiziens (Gloméromycètes) :
   - *Glomus sp.*
   - *Rhizophagus sp.*
   - *Gigaspora sp.*
   - *Acaulospora sp.*
   - *Scutellospora sp.*

Dans un mode de réalisation, l'invention concerne l'utilisation telle que définie ci-dessus, dans laquelle ledit champignon est un gloméromycète.

Dans un mode de réalisation, ledit champignon est *Rhizophagus irregularis.*

Dans un mode de réalisation, l'invention concerne l'utilisation telle que définie ci-dessus, pour favoriser la symbiose mycorhizienne à arbuscules entre une plante *Medicago truncatula* et un champignon gloméromycète, dans laquelle le miPEP171b est introduit par voie exogène dans ladite plante *M. truncatula,* ledit miPEP171b étant également naturellement présent dans ladite plante *M. truncatula,*
ledit miPEP171b introduit par voie exogène étant un peptide dont la séquence comprend ou consiste en une séquence identique à celle dudit miPEP171b naturellement présent, ladite séquence du miPEP171b naturellement présent étant codée par un cadre ouvert de lecture situé en 5' sur le transcrit primaire du miR171b, lequel miR171b régule l'expression d'au moins un gène impliqué dans la symbiose mycorhizienne chez *M. truncatula,*
la somme de la quantité dudit miPEP171b introduit par voie exogène et de celle dudit miPEP171b naturellement présent étant strictement supérieure à la quantité dudit miPEP 171b naturellement présent chez ladite plante *Medicago truncatula.*

Dans un mode de réalisation, l'invention concerne l'utilisation telle que définie ci-dessus, pour favoriser la symbiose mycorhizienne à arbuscules entre une plante *Solanum lycopersicum* et un champignon gloméromycète, dans laquelle le slmiPEP171e est introduit par voie exogène dans ladite plante *Solanum lycopersicum,* ledit slmiPEP171e étant également naturellement présent dans ladite plante *Solanum lycopersicum,*
ledit slmiPEP171e introduit par voie exogène étant un peptide dont la séquence comprend ou consiste en une séquence identique à celle dudit slmiPEP171e naturellement présent, ladite séquence du slmiPEP171e naturellement présent étant codée par un cadre ouvert de
lecture situé en 5' sur le transcrit primaire du slmiR171e, lequel slmiR171e régule l'expression d'au moins un gène impliqué dans la symbiose mycorhizienne chez *Solanum lycopersicum,*
la somme de la quantité dudit slmiPEP171e introduit par voie exogène et de celle dudit slmiPEP171e naturellement présent étant strictement supérieure à la quantité dudit slmiPEP171e naturellement présent chez ladite plante *Solanum lycopersicum.*

Dans un mode de réalisation, l'invention concerne l'utilisation telle que définie ci-dessus, pour favoriser la symbiose mycorhizienne à arbuscules entre une plante *Lotus japonicus* et un champignon gloméromycète, dans laquelle le ljmiPEP171b est introduit par voie exogène dans ladite plante *Lotus japonicus,* ledit ljmiPEP171b étant également naturellement présent dans ladite plante *Lotus japonicus,*
ledit ljmiPEP171b introduit par voie exogène étant un peptide dont la séquence comprend ou consiste en une séquence identique à celle dudit ljmiPEP171b naturellement présent, ladite séquence du ljmiPEP171b naturellement présent étant codée par un cadre ouvert de lecture situé en 5' sur le transcrit primaire du ljmiR171b, lequel ljmiR171b régule l'expression d'au moins un gène impliqué dans la symbiose mycorhizienne chez *Lotus japonicus,*
la somme de la quantité dudit ljmiPEP171b introduit par voie exogène et de celle dudit ljmiPEP171b naturellement présent étant strictement supérieure à la quantité dudit ljmiPEP171b naturellement présent chez ladite plante *Lotus japonicus.*

Dans un mode de réalisation, l'invention concerne l'utilisation telle que définie ci-dessus, pour favoriser la symbiose mycorhizienne à arbuscules entre une plante *Oryza sativa* et un champignon gloméromycète, dans laquelle le osmiPEP171i est introduit par voie exogène dans ladite plante *Oryza sativa,* ledit osmiPEP171i étant également naturellement présent dans ladite plante *Oryza sativa,*
ledit osmiPEP171i introduit par voie exogène étant un peptide dont la séquence comprend ou consiste en une séquence identique à celle dudit osmiPEP171i naturellement présent, ladite séquence du osmiPEP171i naturellement présent étant codée par un cadre ouvert de lecture situé en 5' sur le transcrit primaire du osmiR171i, lequel miR171i régule l'expression d'au moins un gène impliqué dans la symbiose mycorhizienne chez *Oryza sativa,*
la somme de la quantité dudit osmiPEP171i introduit par voie exogène et de celle dudit osmiPEP171i naturellement présent étant strictement supérieure à la quantité dudit osmiPEP171i naturellement présent chez ladite plante *Oryza sativa.*

Dans un mode de réalisation, l'invention concerne l'utilisation telle que définie ci-dessus, dans laquelle ledit miPEP est introduit par voie externe dans la plante, de préférence par arrosage, par pulvérisation ou par l'ajout d'un engrais, d'un terreau, d'un substrat de culture ou d'un support en contact avec la plante.

Dans un mode de réalisation, l'invention concerne l'utilisation telle que définie ci-dessus, dans laquelle ledit miPEP est introduit par voie externe dans une graine ou une semence, de préférence par arrosage, par pulvérisation ou par l'ajout d'un engrais, d'un terreau, d'un substrat de culture ou d'un support en contact avec la graine ou la semence.

Dans un mode de réalisation, l'invention concerne l'utilisation telle que définie ci-dessus, dans laquelle ledit miPEP est introduit par arrosage, notamment par pulvérisation.

Dans un mode de réalisation, l'invention concerne l'utilisation telle que définie ci-dessus, dans laquelle ledit miPEP est introduit par arrosage et par l'ajout d'un engrais.

Dans un mode de réalisation, l'invention concerne l'utilisation telle que définie ci-dessus, dans laquelle ledit miPEP est introduit par l'ajout d'un engrais.

Dans un mode de réalisation, l'invention concerne l'utilisation telle que définie ci-dessus, dans laquelle ledit miPEP est introduit, par arrosage, et par l'ajout d'un engrais.

Les Inventeurs ont en effet constaté de manière inattendue qu'il est possible d'appliquer directement une composition comprenant un miPEP sur la plante pour moduler l'accumulation du miR correspondant dans la plante, ce qui indique que le miPEP est capté par la plante.

Dans un mode de réalisation, l'invention concerne l'utilisation telle que définie ci-dessus, dans laquelle la plante est traitée avec une composition comprenant 10⁻⁹ M à 10⁻⁴ M dudit miPEP, notamment 10⁻⁹ M, 10⁻⁸ M, 10⁻⁷ M, 10⁻⁶ M, 10⁻⁵ M ou 10⁻⁴ M dudit miPEP.

De préférence, les compositions ont une concentration de 10⁻⁸ M à 10⁻⁵ M pour une application par arrosage ou par pulvérisation sur la plante.

De manière complémentaire, des compositions plus ou moins concentrées peuvent être envisagées pour traiter la plante avec le miPEP. Par exemple, et de manière non limitative, des compositions plus concentrées comprenant 10⁻¹ M à 10⁻³ M, notamment 10⁻² M de miPEP, peuvent être utilisées dans le cas où le miPEP introduit par voie exogène est administré à la plante par épandage.

Les propriétés de solubilité des miPEPs sont déterminées notamment par leur composition en acides aminés. Les miPEPs hydrophiles peuvent être solubilisés et conditionnés dans des solutions aqueuses, telles que l'eau. Les miPEPs hydrophobes peuvent être solubilisés et conditionnés dans des solvants, tels que les solvants organiques.

Pour un traitement des plantes par les miPEPs, les solvants organiques sont des solvants non toxiques pour les plantes en faibles quantités, c'est-à-dire qu'ils n'ont pas d'effet délétères sur le développement de la plante. De manière non limitative, les solvants organiques peuvent être choisis parmi l'acétonitrile et l'acide acétique.

Les miPEPs peuvent également être solubilisés et conditionnés dans des mélanges de solvants organiques, comme par exemple un mélange d'acétonitrile et d'acide acétique. En particulier, les miPEPs peuvent être solubilisés dans une solution comprenant 50 % d'acétonitrile, 10% d'acide acétique et 40% d'eau (volume/volume/volume).

En particulier, le miPEP171b est solubilisé dans une solution comprenant 50 % d'acétonitrile, 10% d'acide acétique et 40% d'eau (volume/volume/volume).

En particulier, le miPEP171b solubilisé dans une solution comprenant 50 % d'acétonitrile, 10% d'acide acétique et 40% d'eau (volume/volume/volume) est dilué à une concentration de 10⁻⁹ M à 10⁻⁴ M avec de l'eau.

En particulier, le slmiPEP171e est solubilisé dans une solution comprenant 50% d'acétonitrile et 50 % d'eau (volume/volume).

En particulier, le slmiPEP171e solubilisé dans une solution comprenant 50% d'acétonitrile et 50 % d'eau (volume/volume) est dilué à une concentration de 10⁻⁹ M à 10⁻⁴ M avec de l'eau.

En particulier, le ljmiPEP171b est solubilisé dans de l'eau.

En particulier, le ljmiPEP171b est à une concentration de 10⁻⁹ M à 10⁻⁴ M dans de l'eau.

En particulier, le osmiPEP171i est solubilisé dans une solution comprenant 50% d'acétonitrile et 50 % d'eau (volume/volume).

En particulier, le osmiPEP171i solubilisé dans une solution comprenant 50% d'acétonitrile et 50 % d'eau (volume/volume) est dilué à une concentration de 10⁻⁹ M à 10⁻⁴ M avec de l'eau.

Dans un mode de réalisation, l'invention concerne l'utilisation telle que définie ci-dessus, dans laquelle ledit miPEP est introduit dans la plante par le biais d'un acide nucléique codant ledit miPEP, ledit acide nucléique étant introduit dans la plante.

Dans un mode de réalisation, l'invention concerne l'utilisation telle que définie ci-dessus, dans laquelle le pourcentage de colonisation par ledit champignon est augmenté chez la plante dans laquelle a été introduit ledit miPEP par rapport au pourcentage de colonisation par ledit champignon d'une plante identique et de même âge dans laquelle aucun miPEP n'a été introduit, ou bien par rapport au pourcentage de colonisation par ledit champignon d'une plante identique et de même âge dans laquelle ledit miPEP n'a pas été introduit.

Dans un mode de réalisation, l'invention concerne l'utilisation telle que définie ci-dessus, dans laquelle le nombre d'arbuscules dans les zones racinaires est augmenté chez la plante dans laquelle a été introduit ledit miPEP par rapport au nombre d'arbuscules dans les zones racinaires d'une plante identique et de même âge dans laquelle aucun miPEP n'a été introduit, ou bien par rapport au nombre d'arbuscules dans les zones racinaires d'une plante identique et de même âge dans laquelle ledit miPEP n'a pas été introduit.

Dans un mode de réalisation, l'invention concerne l'utilisation telle que définie ci-dessus, dans laquelle l'aire des arbuscules est augmentée chez la plante dans laquelle a été introduit ledit miPEP par rapport à l'aire des arbuscules d'une plante identique et de même âge dans laquelle aucun miPEP n'a été introduit, ou bien par rapport à l'aire des arbuscules d'une plante identique et de même âge dans laquelle ledit miPEP n'a pas été introduit.

L'augmentation des paramètres permettant de déterminer et quantifier la symbiose mycorhizienne la plante dans laquelle a été introduit le miPEP (tels que le pourcentage de colonisation, le nombre d'arbuscules ou encore l'aire des arbuscules) est de préférence mis en évidence par comparaison avec une plante identique (c'est-à-dire une plante de la même espèce et/ou variété), de même âge et cultivée dans les mêmes conditions mais dans laquelle aucun miPEP n'a été introduit.

L'invention concerne également l'utilisation d'un miPEP introduit par voie exogène dans une plante pour favoriser la symbiose mycorhizienne entre ladite plante et un champignon, ledit miPEP étant codé par le transcrit primaire, introduit artificiellement dans la plante, d'un miR,
ledit transcrit primaire, ledit miR et ledit miPEP étant absents naturellement chez la plante, ledit miPEP étant capable de moduler l'accumulation dudit miR dans ladite plante, lequel miR régule l'expression d'au moins un gène impliqué dans la symbiose mycorhizienne chez ladite plante.

Dans un mode de réalisation particulier, ledit transcrit primaire du miR, le miR et ledit miPEP sont introduits dans la plante à l'aide d'un vecteur.

Dans un autre aspect, l'invention concerne un procédé pour favoriser la symbiose mycorhizienne entre une plante et un champignon, et en particulier la symbiose mycorhizienne à arbuscules (AM), comprenant une étape d'introduction d'un miPEP dans une plante par voie exogène, ledit miPEP étant également présent naturellement dans ladite plante,
ledit miPEP introduit par voie exogène étant un peptide de 3 à 100 acides aminés, notamment de 4 à 100 acides aminés, dont la séquence comprend ou consiste en une séquence identique à celle dudit miPEP naturellement présent, laquelle séquence du miPEP naturellement présent est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la symbiose mycorhizienne chez ladite plante,
la somme de la quantité dudit miPEP introduit par voie exogène et de celle dudit miPEP naturellement présent étant strictement supérieure à la quantité dudit miPEP naturellement présent.

Dans un mode de réalisation, l'invention concerne un procédé tel que défini ci-dessus dans laquelle ledit gène impliqué dans la symbiose mycorhizienne code un facteur de transcription de la famille GRAS.

Dans un mode de réalisation, l'invention concerne un procédé tel que défini ci-dessus, dans lequel ledit gène, impliqué dans la symbiose mycorhizienne, est choisi parmi le groupe consistant en : *HAM1* (Accession n°MTGI9-TC114268) et *HAM2* (Accession n°MTGI9-TC120850).

Dans un mode de réalisation, l'invention concerne un procédé tel que défini ci-dessus, dans lequel ledit miARN est le miR171b, en particulier, dans laquelle ledit miR171b possède une séquence nucléotidique consistant en SEQ ID NO : 1.

Dans un mode de réalisation, l'invention concerne un procédé tel que défini ci-dessus, dans lequel ledit miPEP est le miPEP171b, en particulier, dans laquelle ledit miPEP171b possède une séquence d'acides aminés consistant en SEQ ID NO : 2.

Dans un mode de réalisation, l'invention concerne un procédé tel que défini ci-dessus, dans lequel ledit miARN est le slmiR171e, en particulier, dans laquelle ledit slmiR171e possède une séquence nucléotidique consistant en SEQ ID NO : 5.

Dans un mode de réalisation, l'invention concerne un procédé tel que défini ci-dessus, dans lequel ledit miPEP est le slmiPEP171e, en particulier, dans laquelle ledit slmiPEP171e possède une séquence d'acides aminés consistant en SEQ ID NO : 6.

Dans un mode de réalisation, l'invention concerne un procédé tel que défini ci-dessus, dans lequel ledit miARN est le ljmiR171b, en particulier, dans laquelle ledit ljmiR171b possède une séquence nucléotidique consistant en SEQ ID NO : 9.

Dans un mode de réalisation, l'invention concerne un procédé tel que défini ci-dessus, dans lequel ledit miPEP est le ljmiPEP171b, en particulier, dans laquelle ledit ljmiPEP171b possède une séquence d'acides aminés consistant en SEQ ID NO : 10.

Dans un mode de réalisation, l'invention concerne un procédé tel que défini ci-dessus, dans lequel ledit miARN est le osmiR171i, en particulier, dans laquelle ledit osmiR171i possède une séquence nucléotidique consistant en SEQ ID NO : 13.

Dans un mode de réalisation, l'invention concerne un procédé tel que défini ci-dessus, dans lequel ledit miPEP est le osmiPEP171i, en particulier, dans laquelle ledit osmiPEP171i possède une séquence d'acides aminés consistant en SEQ ID NO : 14.

Dans un mode de réalisation, l'invention concerne un procédé tel que défini ci-dessus, dans lequel ladite plante est une plante Monocotylédone telle que Oryza sativa (riz), une plante Dicotylédone, une plante légumineuse telle que *Glycine max* (soja), *Medicago truncatula* et *Medicago sativa* (luzerne) ou une plante solanacée telle que *Nicotiana benthamiana* (tabac), *Solanum tuberosum* (pomme de terre), *Solanum lycopersicum* (tomate) ou *Solanum melongena* (aubergine).

Dans un mode de réalisation, l'invention concerne un procédé tel que défini ci-dessus, dans lequel ladite plante est *Medicago truncatula, Medicago sativa* (luzerne), *Solanum lycopersicum* (tomate), *Lotus japonicus* (lotier) ou *Oryza sativa* (riz).

Dans un mode de réalisation, l'invention concerne un procédé tel que défini ci-dessus, dans lequel ladite plante est choisie parmi *Medicago truncatula, M. sativa* et *Glycine Max* (soja).

Dans un mode de réalisation, l'invention concerne un procédé tel que défini ci-dessus, dans lequel ladite plante est *Medicago truncatula.*

Dans un mode de réalisation, l'invention concerne un procédé tel que défini ci-dessus, dans lequel ladite plante est *Solanum lycopersicum.*

Dans un mode de réalisation, l'invention concerne un procédé tel que défini ci-dessus, dans lequel ladite plante est *Lotus japonicus.*

Dans un mode de réalisation, l'invention concerne un procédé tel que défini ci-dessus, dans lequel ladite plante est *Oryza sativa.*

Dans un mode de réalisation, l'invention concerne un procédé tel que défini ci-dessus, dans lequel ledit champignon est un champignon endomycorhizien ou ectomycorhizien, de préférence endomycorhizien.

Dans un mode de réalisation, l'invention concerne un procédé tel que défini ci-dessus, dans lequel ledit champignon est un champignon Gloméromycète ou Basidiomycète ou Ascomycète, de préférence Gloméromycète.

Dans un mode de réalisation, ledit champignon est *Rhizophagus irregularis.*

Dans un mode de réalisation, l'invention concerne un procédé tel que défini ci-dessus, pour favoriser la symbiose mycorhizienne à arbuscules entre une plante *Medicago truncatula* et un champignon Gloméromycète, dans lequel le miPEP171b est introduit par voie exogène dans ladite plante *M. truncatula,* ledit miPEP171b étant également naturellement présent dans ladite plante *M. truncatula,*
ledit miPEP171b introduit par voie exogène étant un peptide comprenant ou consistant en une séquence identique à celle dudit miPEP171b naturellement présent, lequel miPEP171b naturellement présent est un peptide de 3 à 100 acides aminés, notamment de 4 à 100 acides aminés dont la séquence est codée par un cadre ouvert de lecture situé en 5' sur le transcrit primaire du miR171b,
ledit miPEP171b étant capable d'augmenter l'accumulation dudit miR171b, lequel miR171b régule l'expression d'au moins un gène impliqué dans la symbiose mycorhizienne chez *M. truncatula,*
la somme de la quantité dudit miPEP171b introduit par voie exogène et de celle dudit miPEP171b naturellement présent étant strictement supérieure à la quantité dudit miPEP171b naturellement présent.

Dans un mode de réalisation, l'invention concerne un procédé tel que défini ci-dessus, pour favoriser la symbiose mycorhizienne à arbuscules entre une plante *Solanum lycopersicum* et un champignon Gloméromycète, dans lequel le slmiPEP171e est introduit par voie exogène dans ladite plante *Solanum lycopersicum,* ledit slmiPEP171e étant également naturellement présent dans ladite plante *Solanum lycopersicum,*
ledit slmiPEP171e introduit par voie exogène étant un peptide comprenant ou consistant en une séquence identique à celle dudit slmiPEP171e naturellement présent, lequel slmiPEP171e naturellement présent est un peptide de 3 à 100 acides aminés, notamment de 4 à 100 acides aminés, dont la séquence est codée par un cadre ouvert de lecture situé en 5' sur le transcrit primaire du slmiR171e,
ledit slmiPEP171e étant capable d'augmenter l'accumulation dudit slmiR171e, lequel slmiR171e régule l'expression d'au moins un gène impliqué dans la symbiose mycorhizienne chez *Solanum lycopersicum,*
la somme de la quantité dudit slmiPEP171e introduit par voie exogène et de celle dudit slmiPEP171e naturellement présent étant strictement supérieure à la quantité dudit slmiPEP171e naturellement présent.

Dans un mode de réalisation, l'invention concerne un procédé tel que défini ci-dessus, pour favoriser la symbiose mycorhizienne à arbuscules entre une plante *Lotus japonicus* et un champignon Gloméromycète, dans lequel le ljmiPEP171b est introduit par voie exogène dans ladite plante *Lotus japonicus,* ledit ljmiPEP171b étant également naturellement présent dans ladite plante *Lotus japonicus,*
ledit ljmiPEP171b introduit par voie exogène étant un peptide comprenant ou consistant en une séquence identique à celle dudit ljmiPEP171b naturellement présent, lequel ljmiPEP171b naturellement présent est un peptide de 3 à 100 acides aminés, notamment de 4 à 100 acides aminés, dont la séquence est codée par un cadre ouvert de lecture situé en 5' sur le transcrit primaire du ljmiR171b,
ledit ljmiPEP171b étant capable d'augmenter l'accumulation dudit ljmiR171b, lequel ljmiR171b régule l'expression d'au moins un gène impliqué dans la symbiose mycorhizienne chez *Lotus japonicus,*
la somme de la quantité dudit ljmiPEP171b introduit par voie exogène et de celle dudit ljmiPEP171b naturellement présent étant strictement supérieure à la quantité dudit ljmiPEP171b naturellement présent.

Dans un mode de réalisation, l'invention concerne un procédé tel que défini ci-dessus, pour favoriser la symbiose mycorhizienne à arbuscules entre une plante *Oriza sativa* et un champignon Gloméromycète, dans lequel le osmiPEP171i est introduit par voie exogène dans ladite plante *Oriza sativa,* ledit osmiPEP171i étant également naturellement présent dans ladite plante *Oriza sativa,*
ledit osmiPEP171i introduit par voie exogène étant un peptide comprenant ou consistant en une séquence identique à celle dudit osmiPEP171i naturellement présent, lequel osmiPEP171i naturellement présent est un peptide de 3 à 100 acides aminés, notamment de 4 à 100 acides aminés, dont la séquence est codée par un cadre ouvert de lecture situé en 5' sur le transcrit primaire du osmiR171i,
ledit osmiPEP171i étant capable d'augmenter l'accumulation dudit osmiR171i, lequel osmiR171i régule l'expression d'au moins un gène impliqué dans la symbiose mycorhizienne chez *Oriza sativa,*
la somme de la quantité dudit osmiPEP171i introduit par voie exogène et de celle dudit osmiPEP171i naturellement présent étant strictement supérieure à la quantité dudit osmiPEP171i naturellement présent.

Dans un mode de réalisation, l'invention concerne un procédé tel que défini ci-dessus, dans lequel ledit miPEP est introduit par voie externe dans la plante, de préférence par arrosage, par pulvérisation ou par l'ajout d'un engrais, d'un terreau, d'un substrat de culture ou d'un support en contact avec la plante.

Dans un mode de réalisation, l'invention concerne un procédé tel que défini ci-dessus, dans lequel ledit miPEP est introduit par voie externe dans une graine ou une semence, de préférence par arrosage, par pulvérisation ou par l'ajout d'un engrais, d'un terreau, d'un substrat de culture ou d'un support en contact avec la graine ou la semence.

Dans un mode de réalisation, l'invention concerne un procédé tel que défini ci-dessus, dans lequel ledit miPEP est administré à la plante sous la forme d'une composition comprenant 10⁻⁹ M à 10⁻⁴ M dudit miPEP, en particulier 10⁻⁹, 10⁻⁸, 10⁻⁷, 10⁻⁶, 10⁻⁵ ou 10⁻⁴ M dudit miPEP.

Dans un mode de réalisation, l'invention concerne un procédé tel que défini ci-dessus, dans lequel ledit miPEP est introduit dans la plante par le biais d'un acide nucléique codant ledit miPEP, ledit acide nucléique étant introduit dans la plante.

Dans un mode de réalisation, l'invention concerne un procédé tel que défini ci-dessus, dans laquelle le pourcentage de colonisation par le dit champignon est augmentée chez la plante dans laquelle a été introduit ledit miPEP par rapport au pourcentage de colonisation par ledit champignon d'une plante identique et de même âge dans laquelle aucun miPEP n'a été introduit, ou bien par rapport au pourcentage de colonisation par ledit champignon d'une plante identique et de même âge dans laquelle ledit miPEP n'a pas été introduit. Dans un mode de réalisation, l'invention concerne un procédé tel que défini ci-dessus, dans laquelle le nombre d'arbuscules dans les zones racinaires est augmenté chez la plante dans laquelle a été introduit ledit miPEP par rapport au nombre d'arbuscules dans les zones racinaires d'une plante identique et de même âge dans laquelle aucun miPEP n'a été introduit, ou bien par rapport au nombre d'arbuscules dans les zones racinaires d'une plante identique et de même âge dans laquelle ledit miPEP n'a pas été introduit.

Dans un mode de réalisation, l'invention concerne un procédé tel que défini ci-dessus, dans laquelle l'aire des arbuscules est augmentée chez la plante dans laquelle a été introduit ledit miPEP par rapport à l'aire des arbuscules d'une plante identique et de même âge dans laquelle aucun miPEP n'a été introduit, ou bien par rapport à l'aire des arbuscules d'une plante identique et de même âge dans laquelle ledit miPEP n'a pas été introduit.

Dans un autre aspect, l'invention concerne une plante dans laquelle a été introduit un miPEP selon l'utilisation ou le procédé pour favoriser la symbiose mycorhizienne décrits ci-dessus.

Dans un autre aspect, l'invention concerne un procédé de production d'une plante transgénique comprenant:
**a)** une étape d'introduction d'un acide nucléique codant un miPEP de 3 à 100 acides aminés, notamment de 4 à 100 acides aminés dans une plante, ou dans au moins une cellule de ladite plante, dans des conditions permettant l'expression dudit miPEP, ledit miPEP étant également naturellement présent dans ladite plante, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR dans la plante, lequel miR régule l'expression d'au moins un gène impliqué dans la symbiose mycorhizienne, en particulier la symbiose mycorhizienne à arbuscules, et
**b)** une étape de culture de la plante, ou d'au moins une cellule de ladite plante, obtenues à l'étape a) dans des conditions permettant l'obtention d'une plante transgénique.

Dans un mode de réalisation, l'invention concerne un procédé de production d'une plante transgénique tel que défini ci-dessus, dans lequel ladite plante transgénique obtenue à l'étape b) est plus apte à former une symbiose mycorhizienne par rapport à une plante identique dans laquelle ledit acide nucléique n'a pas été introduit.

Dans un mode de réalisation, l'invention concerne un procédé de production d'une plante transgénique tel que défini ci-dessus, dans lequel l'étape a) est réalisée à l'aide d'un vecteur contenant ledit acide nucléique, de préférence un plasmide.

Dans un mode de réalisation, l'invention concerne un procédé de production d'une plante transgénique tel que défini ci-dessus, dans lequel ledit acide nucléique ne comprend pas la séquence complète dudit miR.

Dans un mode de réalisation, l'invention concerne un procédé de production d'une plante transgénique tel que défini ci-dessus, dans lequel l'expression dudit acide nucléique de l'étape a) est placée sous le contrôle d'un promoteur fort, de préférence un promoteur fort constitutif tel que le promoteur 35S.

Dans un mode de réalisation, l'invention concerne un procédé de production d'une plante transgénique tel que défini ci-dessus, dans lequel ledit gène impliqué dans la symbiose mycorhizienne code un facteur de transcription de la famille GRAS.

Dans un mode de réalisation, l'invention concerne un procédé de production d'une plante transgénique tel que défini ci-dessus, dans lequel ledit gène, impliqué dans le développement des parties végétatives ou reproductrices de la plante, est choisi parmi le groupe consistant en: *HAM1* (Accession n°MTGI9-TC114268) et *HAM2* (Accession n°MTGI9-TC120850).

Dans un mode de réalisation, l'invention concerne un procédé de production d'une plante transgénique tel que défini ci-dessus, dans lequel ledit miARN est le miR171b, en particulier, dans lequel ledit miR171b possède une séquence nucléotidique consistant en SEQ ID NO : 1.

Dans un mode de réalisation, l'invention concerne un procédé de production d'une plante transgénique tel que défini ci-dessus, dans lequel ledit miPEP est le miPEP171b, en particulier, dans lequel ledit miPEP171b possède une séquence d'acides aminés consistant en SEQ ID NO : 2.

Dans un mode de réalisation, l'invention concerne un procédé de production d'une plante transgénique tel que défini ci-dessus, dans lequel ledit acide nucléique introduit à l'étape a) comprend une séquence nucléotidique consistant en SEQ ID NO : 3.

Dans un mode de réalisation, l'invention concerne un procédé de production d'une plante transgénique tel que défini ci-dessus, dans lequel ledit miARN est le slmiR171e, en particulier, dans lequel ledit slmiR171e possède une séquence nucléotidique consistant en SEQ ID NO : 5.

Dans un mode de réalisation, l'invention concerne un procédé de production d'une plante transgénique tel que défini ci-dessus, dans lequel ledit miPEP est le slmiPEP171e, en particulier, dans lequel ledit slmiPEP171e possède une séquence d'acides aminés consistant en SEQ ID NO : 6.

Dans un mode de réalisation, l'invention concerne un procédé de production d'une plante transgénique tel que défini ci-dessus, dans lequel ledit acide nucléique introduit à l'étape a) comprend une séquence nucléotidique consistant en SEQ ID NO : 7.

Dans un mode de réalisation, l'invention concerne un procédé de production d'une plante transgénique tel que défini ci-dessus, dans lequel ledit miARN est le ljmiR171b, en particulier, dans lequel ledit ljmiR171b possède une séquence nucléotidique consistant en SEQ ID NO : 9.

Dans un mode de réalisation, l'invention concerne un procédé de production d'une plante transgénique tel que défini ci-dessus, dans lequel ledit miPEP est le ljmiPEP171b, en particulier, dans lequel ledit ljmiPEP171b possède une séquence d'acides aminés consistant en SEQ ID NO : 10.

Dans un mode de réalisation, l'invention concerne un procédé de production d'une plante transgénique tel que défini ci-dessus, dans lequel ledit acide nucléique introduit à l'étape a) comprend une séquence nucléotidique consistant en SEQ ID NO : 11.

Dans un mode de réalisation, l'invention concerne un procédé de production d'une plante transgénique tel que défini ci-dessus, dans lequel ledit miARN est le osmiR171i, en particulier, dans lequel ledit osmiR171i possède une séquence nucléotidique consistant en SEQ ID NO : 13.

Dans un mode de réalisation, l'invention concerne un procédé de production d'une plante transgénique tel que défini ci-dessus, dans lequel ledit miPEP est le osmiPEP171i, en particulier, dans lequel ledit osmiPEP171i possède une séquence d'acides aminés consistant en SEQ ID NO : 14.

Dans un mode de réalisation, l'invention concerne un procédé de production d'une plante transgénique tel que défini ci-dessus, dans lequel ledit acide nucléique introduit à l'étape a) comprend une séquence nucléotidique consistant en SEQ ID NO : 15.

Dans un mode de réalisation, l'invention concerne un procédé tel que défini ci-dessus, dans lequel ladite plante est une plante Monocotylédone telle que Oryza sativa (riz), une plante Dicotylédone, une plante légumineuse telle que *Glycine max* (soja), *Medicago truncatula* et *Medicago sativa* (luzerne) ou une plante solanacée telle que *Nicotiana benthamiana* (tabac), *Solanum tuberosum* (pomme de terre), *Solanum lycopersicum* (tomate) ou *Solanum melongena* (aubergine).

Dans un mode de réalisation, l'invention concerne un procédé de production d'une plante transgénique tel que défini ci-dessus, dans lequel ladite plante est *Medicago truncatula, Medicago sativa* (luzerne), *Solanum lycopersicum* (tomate), *Lotus japonicus* (lotier) ou *Oryza sativa* (riz).

Dans un mode de réalisation, l'invention concerne un procédé de production d'une plante transgénique tel que défini ci-dessus, dans lequel ladite plante est choisie parmi *Medicago truncatula, M. sativa* et *Glycine max* (soja).

Dans un mode de réalisation, l'invention concerne un procédé de production d'une plante transgénique tel que défini ci-dessus, dans lequel ladite plante transgénique est *Medicago truncatula.*

Dans un mode de réalisation, l'invention concerne un procédé de production d'une plante transgénique tel que défini ci-dessus, dans lequel ladite plante transgénique est *Solanum lycopersicum.*

Dans un mode de réalisation, l'invention concerne un procédé de production d'une plante transgénique tel que défini ci-dessus, dans lequel ladite plante transgénique est *Lotus japonicus.*

Dans un mode de réalisation, l'invention concerne un procédé de production d'une plante transgénique tel que défini ci-dessus, dans lequel ladite plante transgénique est *Oryza sativa.*

Dans un mode de réalisation, l'invention concerne un procédé de production d'une plante transgénique tel que défini ci-dessus, comprenant :
**a)** une étape d'introduction d'un acide nucléique contenant la séquence nucléotidique SEQ ID NO : 3, codant le miPEP171b consistant en la séquence d'acides aminés SEQ ID NO : 2, dans une plante *M. truncatula,* ou dans au moins une cellule de ladite plante *M*. *truncatula,* dans des conditions permettant l'expression du miPEP171b,
   ledit miPEP171b étant également naturellement présent dans ladite plante *M. truncatula,* ledit miPEP naturellement présent étant un peptide dont la séquence est codée par un cadre ouvert de lecture situé en 5' sur le transcrit primaire du miR171b, ledit miPEP171b étant capable de moduler l'accumulation dudit miR171, lequel miR171b régule l'expression d'au moins un gène impliqué dans la symbiose mycorhizienne chez *M. truncatula,* et
**b)** une étape de culture de la plante, ou d'au moins une cellule de ladite plante, obtenues à l'étape a) dans des conditions permettant l'obtention d'une plante *M. truncatula* transgénique.

Dans un mode de réalisation, l'invention concerne un procédé de production d'une plante transgénique tel que défini ci-dessus, comprenant :
**a)** une étape d'introduction d'un acide nucléique contenant la séquence nucléotidique SEQ ID NO : 7, codant le slmiPEP171e consistant en la séquence d'acides aminés SEQ ID NO : 6, dans une plante *Solanum lycopersicum,* ou dans au moins une cellule de ladite plante *Solanum lycopersicum,* dans des conditions permettant l'expression du slmiPEP171e,
   ledit slmiPEP171e étant également naturellement présent dans ladite plante *Solanum lycopersicum,* ledit slmiPEPe naturellement présent étant un peptide dont la séquence est codée par un cadre ouvert de lecture situé en 5' sur le transcrit primaire du slmiR171e, ledit slmiPEP171e étant capable de moduler l'accumulation dudit slmiR171e, lequel slmiR171e régule l'expression d'au moins un gène impliqué dans la symbiose mycorhizienne chez *Solanum lycopersicum,* et
**b)** une étape de culture de la plante, ou d'au moins une cellule de ladite plante, obtenues à l'étape a) dans des conditions permettant l'obtention d'une plante *Solanum lycopersicum* transgénique.

Dans un mode de réalisation, l'invention concerne un procédé de production d'une plante transgénique tel que défini ci-dessus, comprenant :
**a)** une étape d'introduction d'un acide nucléique contenant la séquence nucléotidique SEQ ID NO: 11, codant le ljmiPEP171b consistant en la séquence d'acides aminés SEQ ID NO : 10, dans une plante *Lotus japonicus,* ou dans au moins une cellule de ladite plante *Lotus japonicus* dans des conditions permettant l'expression du ljmiPEP171b,
   ledit ljmiPEP171b étant également naturellement présent dans ladite plante *Lotus japonicus,* ledit ljmiPEPb naturellement présent étant un peptide dont la séquence est codée par un cadre ouvert de lecture situé en 5' sur le transcrit primaire du ljmiR171b, ledit ljmiPEP171b étant capable de moduler l'accumulation dudit ljmiR171b, lequel ljmiR171b régule l'expression d'au moins un gène impliqué dans la symbiose mycorhizienne chez *Lotus japonicus* et
**b)** une étape de culture de la plante, ou d'au moins une cellule de ladite plante, obtenues à l'étape a) dans des conditions permettant l'obtention d'une plante *Lotus japonicus* transgénique.

Dans un mode de réalisation, l'invention concerne un procédé de production d'une plante transgénique tel que défini ci-dessus, comprenant :
**a)** une étape d'introduction d'un acide nucléique contenant la séquence nucléotidique SEQ ID NO : 15, codant le osmiPEP171i consistant en la séquence d'acides aminés SEQ ID NO : 14, dans une plante *Oriza sativa,* ou dans au moins une cellule de ladite plante *Oriza sativa* dans des conditions permettant l'expression du osmiPEP171i,
   ledit osmiPEP171i étant également naturellement présent dans ladite plante *Oriza sativa,* ledit osmiPEPi naturellement présent étant un peptide dont la séquence est codée par un cadre ouvert de lecture situé en 5' sur le transcrit primaire du osmiR171i, ledit miPEP171i étant capable de moduler l'accumulation dudit osmiR171i, lequel osmiR171i régule l'expression d'au moins un gène impliqué dans la symbiose mycorhizienne chez *Oriza sativa,* et
**b)** une étape de culture de la plante, ou d'au moins une cellule de ladite plante, obtenues à l'étape a) dans des conditions permettant l'obtention d'une plante *Oriza sativa* transgénique.

Dans un mode de réalisation, l'invention concerne un procédé de production tel que défini ci-dessus, dans lequel ledit miPEP est introduit dans la plante par le biais d'un acide nucléique codant ledit miPEP, ledit acide nucléique étant introduit dans la plante.

Dans un mode de réalisation, l'invention concerne un procédé de production d'une plante transgénique tel que défini ci-dessus, dans laquelle le pourcentage de colonisation par ledit champignon est augmenté chez la plante dans laquelle a été introduit ledit miPEP par rapport au pourcentage de colonisation par ledit champignon d'une plante identique et de même âge dans laquelle aucun miPEP n'a été introduit, ou bien par rapport au pourcentage de colonisation par ledit champignon d'une plante identique et de même âge dans laquelle ledit miPEP n'a pas été introduit.

Dans un mode de réalisation, l'invention concerne un procédé de production d'une plante transgénique tel que défini ci-dessus, dans laquelle le nombre d'arbuscules dans les zones racinaires est augmenté chez la plante dans laquelle a été introduit ledit miPEP par rapport au nombre d'arbuscules dans les zones racinaires d'une plante identique et de même âge dans laquelle aucun miPEP n'a été introduit, ou bien par rapport au nombre d'arbuscules dans les zones racinaires d'une plante identique et de même âge dans laquelle ledit miPEP n'a pas été introduit.

Dans un mode de réalisation, l'invention concerne un procédé de production d'une plante transgénique tel que défini ci-dessus, dans laquelle l'aire des arbuscules est augmentée chez la plante dans laquelle a été introduit ledit miPEP par rapport à l'aire des arbuscules d'une plante identique et de même âge dans laquelle aucun miPEP n'a été introduit, ou bien par rapport à l'aire des arbuscules d'une plante identique et de même âge dans laquelle ledit miPEP n'a pas été introduit.

Dans un aspect, l'invention concerne également une plante transgénique telle qu'obtenue par le procédé de production tel que défini ci-dessus.

Dans un autre aspect, l'invention concerne une composition, en particulier une composition phytosanitaire, comprenant le miPEP171b en tant que substance active, ledit miPEP171b consistant de préférence en SEQ ID NO : 2.

Dans un autre aspect, l'invention concerne une composition, en particulier une composition phytosanitaire, comprenant le slmiPEP171e en tant que substance active, ledit slmiPEP171e consistant de préférence en SEQ ID NO : 6.

Dans un autre aspect, l'invention concerne une composition, en particulier une composition phytosanitaire, comprenant le ljmiPEP171b en tant que substance active, ledit ljmiPEP171b consistant de préférence en SEQ ID NO : 10.

Dans un autre aspect, l'invention concerne une composition, en particulier une composition phytosanitaire, comprenant le osmiPEP171i en tant que substance active, ledit osmiPEP171i consistant de préférence en SEQ ID NO : 14.

Dans un autre aspect, l'invention concerne une composition telle que définie ci-dessus, dans laquelle ledit miPEP171b est à une concentration de 10⁻⁹ M à 10⁻⁴ M, en particulier 10⁻⁹, 10⁻⁸, 10⁻⁷, 10⁻⁶, 10⁻⁵ ou 10⁻⁴ M.

De préférence, une composition telle que définie ci-dessus a une concentration de 10⁻⁸ M à 10⁻⁵ M pour une application par arrosage ou par pulvérisation sur la plante.

De manière complémentaire, des compositions plus ou moins concentrées peuvent être envisagées pour traiter la plante avec le miPEP. Par exemple, et de manière non limitative, des compositions plus concentrées comprenant 10⁻¹ M à 10⁻³ M, notamment 10⁻² M de miPEP, peuvent être utilisées dans le cas où le miPEP introduit par voie exogène est administré à la plante par épandage.

Dans un autre aspect, l'invention concerne une composition telle que définie ci-dessus, comprenant de plus un excipient, un diluant ou un solvant.

Dans un mode de réalisation, l'invention concerne une composition telle que définie ci-dessus formulée de façon à former un enrobé.

Dans un autre aspect, l'invention concerne une composition comprenant en combinaison une quantité de semences d'une plante et une quantité d'un peptide dont la séquence comprend ou consiste en une séquence identique à celle d'un miPEP naturellement présent chez ladite plante.

Dans un mode de réalisation, l'invention concerne une composition comprenant en combinaison une quantité de semences d'une plante, notamment *M. truncatula,* et une quantité d'un peptide dont la séquence comprend ou consiste en une séquence identique à celle du miPEP171b.

Dans un mode de réalisation, l'invention concerne une composition comprenant en combinaison une quantité de semences d'une plante, notamment *Solanum lycopersicum,* et une quantité d'un peptide dont la séquence comprend ou consiste en une séquence identique à celle du slmiPEP171e.

Dans un mode de réalisation, l'invention concerne une composition comprenant en combinaison une quantité de semences d'une plante, notamment *Lotus japonicus,* et une quantité d'un peptide dont la séquence comprend ou consiste en une séquence identique à celle du ljmiPEP171b.

Dans un mode de réalisation, l'invention concerne une composition comprenant en combinaison une quantité de semences d'une plante, notamment *Oriza sativa,* et une quantité d'un peptide dont la séquence comprend ou consiste en une séquence identique à celle du osmiPEP171i.

Dans un autre aspect, l'invention concerne une composition telle que définie ci-dessus, comprenant de plus un excipient, un diluant ou un solvant.

Dans un mode de réalisation, l'invention concerne une composition telle que définie ci-dessus formulée de façon à former une semence enrobée.

L'enrobage peut être réalisé selon les procédés classiquement utilisées dans l'industrie agroalimentaire et peut obtenu en utilisant un matériau apte à se désagréger dans un solvant ou dans la terre, tel qu'un liant ou de l'argile.

Selon l'invention, l'enrobage peut être utilisé pour par exemple conférer des propriétés particulières à une composition de miPEP, ou encore à une composition de semences en combinaison avec un miPEP.

Dans un autre aspect de l'invention, il est décrit un protocole de production d'un peptide recombinant, dont la séquence comprend ou consiste en une séquence identique à celle d'un miPEP tel que défini ci-dessus, comprenant une étape de transformation d'un organisme avec un vecteur d'expression codant ledit peptide recombinant.

Dans un mode de réalisation, ledit organisme est choisi parmi le groupe comprenant les bactéries, les levures, les champignons (autre que levures), les cellules animales, les plantes et les animaux.

Dans un mode de réalisation, ledit organisme est *Escherichia coli.*

En particulier, il est décrit un protocole de production d'un peptide recombinant tel que défini ci-dessus, comprenant les étapes suivantes :
- l'acide nucléique codant ledit peptide recombinant est lié à un acide nucléique codant une étiquette, telle que la GST,
- le vecteur d'expression contenant ledit acide nucléique codant ledit peptide recombinant est introduit dans la bactérie *E. coli,*
- la bactérie *E. coli* contenant le vecteur d'expression est cultivée dans du milieu LB de préférence jusqu'à une DO comprise entre 0.2 et 0.4,
- la production du peptide recombinant est induite avec de l'IPTG, de préférence pendant 4 à 5 heures,
- les bactéries *E. coli* sont centrifugées et lysées,
- le surnageant est filtré,
- ledit peptide recombinant est purifié sur une colonne d'affinité glutathion sepharose,
- si nécessaire, cliver la GST avec une protéase.

Dans un autre aspect il est décrit un anticorps reconnaissant spécifiquement le miPEP171b, en particulier ledit miPEP171b consistant en SEQ ID NO : 2.

Un tel anticorps peut être obtenu à partir d'un procédé connu de l'homme du métier, comme par exemple en injectant ledit miPEP171b à un animal non humain pour déclencher une réaction d'immunisation et la production d'anticorps par ledit animal.

Dans un autre aspect il est décrit un anticorps reconnaissant spécifiquement le slmiPEP171e, le ljmiPEP171b ou le osmiPEP171i consistant respectivement en SEQ ID NO : 5, SEQ ID NO : 9 et SEQ ID NO : 13.

Dans un autre aspect il est décrit un procédé d'immunolocalisation du miPEP171b comprenant une étape de marquage d'un échantillon biologique d'une plante avec un anticorps reconnaissant spécifiquement ledit miPEP171b.

Dans un autre aspect il est décrit un procédé d'immunolocalisation du slmiPEP171e comprenant une étape de marquage d'un échantillon biologique d'une plante avec un anticorps reconnaissant spécifiquement ledit slmiPEP171e.

Dans un autre aspect il est décrit un procédé d'immunolocalisation du ljmiPEP171b comprenant une étape de marquage d'un échantillon biologique d'une plante avec un anticorps reconnaissant spécifiquement ledit ljmiPEP171b.

Dans un autre aspect il est décrit un procédé d'immunolocalisation du osmiPEP171i comprenant une étape de marquage d'un échantillon biologique d'une plante avec un anticorps reconnaissant spécifiquement ledit osmiPEP171i.

Dans un autre aspect, l'invention concerne un procédé de culture de champignons mycorhiziens, en particulier des champignons mycorhiziens à arbuscules, comprenant une étape de mise en contact desdits champignons :
- avec un mélange comprenant une plante ou une partie de plante, en particulier une culture de racine, et un peptide dont la séquence comprend ou consiste en une séquence identique à celle d'un miPEP naturellement présent dans ladite plante, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la symbiose mycorhizienne chez ladite plante, ou
- avec une plante transgénique telle que définie précédemment,
la plante, la partie de plante, et la plante transgénique étant aptes à former une symbiose mycorhizienne avec ledit champignon.

En particulier, le procédé de culture de champignons mycorhiziens tel que défini ci-dessus est réalisé dans des conditions de culture permettant la croissance, voire l'amélioration de la croissance de la plante, de la partie de plante, et de la plante transgénique, et celle du champignon.

En particulier, le procédé de culture de champignons mycorhiziens tel que défini ci-dessus est réalisé dans des conditions de culture permettant la symbiose mycorhizienne entre le champignon et la plante, la partie de plante ou la plante transgénique.

En particulier, l'invention concerne un procédé de culture de champignons mycorhiziens tel que défini ci-dessus, dans lequel ledit champignon est un Gloméromycète.

Dans un mode de réalisation, le peptide présent dans le mélange est un peptide isolé, un peptide isolé et/ou purifié, un peptide synthétique ou un peptide recombinant.

Dans un mode de réalisation, l'invention concerne un procédé de culture de champignons mycorhiziens tel que défini ci-dessus, dans lequel ledit miR est le miR171b, en particulier, dans laquelle ledit miR171b possède une séquence nucléotidique consistant en SEQ ID NO : 1.

Dans un mode de réalisation, l'invention concerne un procédé de culture de champignons mycorhiziens tel que défini ci-dessus, dans lequel ledit miPEP est le miPEP171b, en particulier, dans laquelle ledit miPEP171b possède une séquence d'acides aminés consistant en SEQ ID NO : 2.

Dans un mode de réalisation, l'invention concerne un procédé de culture de champignons mycorhiziens tel que défini ci-dessus, dans lequel ledit miR est le slmiR171e, en particulier, dans laquelle ledit slmiR171e possède une séquence nucléotidique consistant en SEQ ID NO : 5.

Dans un mode de réalisation, l'invention concerne un procédé de culture de champignons mycorhiziens tel que défini ci-dessus, dans lequel ledit miPEP est le slmiPEP171e, en particulier, dans laquelle ledit slmiPEP171e possède une séquence d'acides aminés consistant en SEQ ID NO : 6.

Dans un mode de réalisation, l'invention concerne un procédé de culture de champignons mycorhiziens tel que défini ci-dessus, dans lequel ledit miR est le ljmiR171b, en particulier, dans laquelle ledit ljmiR171b possède une séquence nucléotidique consistant en SEQ ID NO : 9.

Dans un mode de réalisation, l'invention concerne un procédé de culture de champignons mycorhiziens tel que défini ci-dessus, dans lequel ledit miPEP est le ljmiPEP171b, en particulier, dans laquelle ledit ljmiPEP171b possède une séquence d'acides aminés consistant en SEQ ID NO : 10.

Dans un mode de réalisation, l'invention concerne un procédé de culture de champignons mycorhiziens tel que défini ci-dessus, dans lequel ledit miR est le osmiR171i, en particulier, dans laquelle ledit osmiR171i possède une séquence nucléotidique consistant en SEQ ID NO : 13.

Dans un mode de réalisation, l'invention concerne un procédé de culture de champignons mycorhiziens tel que défini ci-dessus, dans lequel ledit miPEP est le osmiPEP171i, en particulier, dans laquelle ledit osmiPEP171i possède une séquence d'acides aminés consistant en SEQ ID NO : 14.

En particulier, l'invention concerne un procédé de culture de champignons mycorhiziens tel que défini ci-dessus, dans lequel ladite partie de plante est une racine ou un fragment de racines.

Dans un autre aspect il est décrit un procédé de production de spores de champignons mycorhiziens, en particulier des champignons mycorhiziens à arbuscules, comprenant une étape de mise en contact desdits champignons :
- avec un mélange comprenant une plante, ou une partie de plante, et un peptide dont la séquence comprend ou consiste en une séquence identique à celle d'un miPEP naturellement présent dans ladite plante, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la symbiose mycorhizienne chez ladite plante, ou
- avec une plante transgénique telle que définie précédemment,
la plante, la partie de plante, et la plante transgénique étant aptes à former une symbiose mycorhizienne avec ledit champignon.

En particulier, le procédé de production de spores tel que défini ci-dessus est réalisé dans des conditions de culture permettant la croissance de la plante, de la partie de plante, et de la plante transgénique, et celle du champignon.

En particulier, le procédé de production de spores tel que défini ci-dessus est réalisé dans des conditions de culture permettant la symbiose mycorhizienne entre le champignon et la plante, la partie de plante ou la plante transgénique.

En particulier, il est décrit un procédé de production de spores tel que défini ci-dessus, dans lequel ledit champignon est un Gloméromycète.

Dans un mode de réalisation, ledit champignon est *Rhizophagus irregularis.*

Dans un mode de réalisation, il est décrit un procédé de production de spores tel que défini ci-dessus, dans lequel ledit miR est le miR171b, en particulier, dans laquelle ledit miR171b possède une séquence nucléotidique consistant en SEQ ID NO : 1.

Dans un mode de réalisation, il est décrit un procédé de production de spores tel que défini ci-dessus, dans lequel ledit miPEP est le miPEP171b, en particulier, dans laquelle ledit miPEP171b possède une séquence d'acides aminés consistant en SEQ ID NO : 2.

Dans un mode de réalisation, il est décrit un procédé de production de spores tel que défini ci-dessus, dans lequel ledit miR est le slmiR171e en particulier, dans laquelle ledit slmiR171e possède une séquence nucléotidique consistant en SEQ ID NO : 5.

Dans un mode de réalisation, il est décrit un procédé de production de spores tel que défini ci-dessus, dans lequel ledit miPEP est le slmiPEP171e, en particulier, dans laquelle ledit slmiPEP171e possède une séquence d'acides aminés consistant en SEQ ID NO : 6.

Dans un mode de réalisation, il est décrit un procédé de production de spores tel que défini ci-dessus, dans lequel ledit miR est le ljmiR171b, en particulier, dans laquelle ledit ljmiR171b possède une séquence nucléotidique consistant en SEQ ID NO : 9.

Dans un mode de réalisation, il est décrit un procédé de production de spores tel que défini ci-dessus, dans lequel ledit miPEP est le ljmiPEP171b, en particulier, dans laquelle ledit ljmiPEP171b possède une séquence d'acides aminés consistant en SEQ ID NO : 10.

Dans un mode de réalisation, il est décrit un procédé de production de spores tel que défini ci-dessus, dans lequel ledit miR est le osmiR171i, en particulier, dans laquelle ledit osmiR171i possède une séquence nucléotidique consistant en SEQ ID NO : 13.

Dans un mode de réalisation, il est décrit un procédé de production de spores tel que défini ci-dessus, dans lequel ledit miPEP est le osmiPEP171i, en particulier, dans laquelle ledit osmiPEP171i possède une séquence d'acides aminés consistant en SEQ ID NO : 14.

En particulier, il est décrit un procédé de production de spores tel que défini ci-dessus, dans lequel ladite partie de plante est une racine ou un fragment de racines.

Dans un autre aspect, l'invention concerne un procédé pour produire de l'inoculum de champignons mycorhiziens, en particulier de l'inoculum de champignons mycorhiziens à arbuscules, comprenant :
- une étape de co-culture de champignons avec une matière végétale vivante, dite plante hôte, correspondant au moins en partie, à une partie constitutive de racine d'une plante apte à former une symbiose avec lesdits champignons, et
- une étape de mise en contact d'une quantité d'un peptide avec la susdite co-culture,
ledit peptide ayant une séquence comprenant ou consistant en une séquence identique à celle d'un miPEP naturellement présent dans ladite plante hôte, ledit miPEP naturellement présent étant un peptide dont la séquence est codée par un cadre ouvert de lecture situé en

5' sur le transcrit primaire d'un miR, ledit miPEP étant capable de moduler l'accumulation dudit miR, lequel miR régule l'expression d'au moins un gène impliqué dans la symbiose mycorhizienne chez ladite plante hôte.

L'invention concerne également un procédé pour produire de l'inoculum de champignons mycorhiziens, en particulier de l'inoculum de champignons mycorhiziens à arbuscules, comprenant une étape de co-culture de champignons avec une matière végétale vivante, dite plante hôte, correspondant au moins en partie, à une partie constitutive de racine d'une plante apte à former une symbiose avec lesdits champignons, et
ladite plante hôte étant une plante transgénique ou une plante dans laquelle a été introduit un peptide,
ledit peptide ayant une séquence comprenant ou consistant en une séquence identique à celle d'un miPEP naturellement présent dans ladite plante hôte, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la symbiose mycorhizienne chez ladite plante hôte.

L'invention concerne également un procédé pour produire de l'inoculum de champignons mycorhiziens, en particulier de l'inoculum de champignons mycorhiziens à arbuscules, comprenant une étape de mélange rassemblant :
- des champignons,
- une matière végétale vivante, dite plante hôte, correspondant au moins en partie, à une partie constitutive de racine d'une plante apte à former une symbiose avec lesdits champignons, et
- un peptide,
ledit peptide ayant une séquence comprenant ou consistant en une séquence identique à celle d'un miPEP naturellement présent dans ladite plante hôte, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la symbiose mycorhizienne chez ladite plante hôte.

Dans un autre aspect, l'invention concerne un inoculum de champignons mycorhiziens, en particulier un inoculum de champignons mycorhiziens à arbuscules, adapté à l'inoculation d'une plante hôte, comprenant au moins un champignon et un peptide dont la séquence comprend ou consiste en une séquence identique à celle d'un miPEP naturellement présent dans la plante hôte,
ledit miPEP naturellement présent dans la plante hôte étant un peptide dont la séquence est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la symbiose mycorhizienne chez ladite plante hôte.

De préférence, ledit miPEP est le miPEP171b.

Ledit miPEP peut également être choisi parmi le slmiPEP171e, le ljmiPEP171b et le osmiPEP171i.

Le peptide utilisé pour produire de l'inoculum, ou le peptide présent dans l'inoculum, est notamment un peptide isolé, un peptide isolé et/ou purifié, un peptide synthétique ou un peptide recombinant.

De préférence, l'inoculum contient en plus une plante ou une partie de plante, en particulier une racine, une culture de racine ou une partie de racine.

Pour produire de l'inoculum, et au sein de l'inoculum, le champignon utilisé peut être sous forme de spores ou sous forme de mycélium.

Les séquences du miPEP171b, de son cadre ouvert de lecture, du miR171b et des transcrits primaires du miR171b chez *M. truncatula* est indiqué dans le tableau 1.

**Tableau 1.**

| | | |
|---|---|---|
| **miR171b** | UGAUUGAGCCGCGUCAAUAUC | **SEQ ID NO : 1** |
| **miPEP171b** | MLLHRLSKFCKIERDIVYIS | **SEQ ID NO : 2** |
| **miORF171b** | | **SEQ ID NO : 3** |
| **pri-miR171b** | | **SEQ ID NO : 4** |
| | | |

Les séquences du slmiPEP171e, de son cadre ouvert de lecture, du slmiR171e et du transcrit primaire du slmiR171e chez *Solanum lycopersicum* est indiqué dans le tableau 2.

**Tableau 2.**

| | | |
|---|---|---|
| **slmiR171e** | uugagccgcgucaauaucucu | **SEQ ID NO : 5** |
| **slmiPEP171e** | MKLGNIEGTYFIICLGRYI | **SEQ ID NO : 6** |
| **slmiORF171e** | | **SEQ ID NO : 7** |
| **slpri-miR171e** | | **SEQ ID NO : 8** |
| | | |

Les séquences du ljmiPEP171b, de son cadre ouvert de lecture, du ljmiR171b et du transcrit primaire du ljmiR171b chez *Lotus japonicus* est indiqué dans le tableau 3.

**Tableau 3.**

| | | |
|---|---|---|
| **ljmiR171b** | ugauugagccgcgucaauauc | **SEQ ID NO : 9** |
| **ljmiPEP171b** | MYHRSKAKLCQTDGDDGGGSDM | **SEQ ID NO : 10** |
| **ljmiORF171b** | | **SEQ ID NO : 11** |
| **ljpri-miR171b** | | **SEQ ID NO : 12** |
| | | |

Les séquences du osmiPEP171i, de son cadre ouvert de lecture, du osmiR171i et du transcrit primaire du osmiR171i chez *Oryza sativa* est indiqué dans le tableau 4.

**Tableau 4.**

| | | |
|---|---|---|
| **osmiR171i** | ggauugagccgcgucaauauc | **SEQ ID NO : 13** |
| **osmiPEP171i** | | **SEQ ID NO : 14** |
| **osmiORF171i** | | **SEQ ID NO : 15** |
| **ospri-miR171i** | | **SEQ ID NO : 16** |
| | | |

*L'objet de la demande* FR 13 60727 *concerne des micropeptides (peptides codés par des microARNs ou « miPEPs ») et leur utilisation pour moduler l'expression de gènes.*

*Les microARNs (miRs) sont des petits ARNs non codants, d'environ 21 nucléotides après maturation, qui contrôlent l'expression de gènes cibles au niveau post-transcriptionnel, en dégradant l'ARNm cible ou en inhibant sa traduction. Les miRs sont rencontrés chez les plantes et les animaux.*

*Les gènes cibles sont souvent des gènes clés de processus développementaux. Ils codent par exemple des facteurs de transcription ou des protéines du protéasome.*

*La régulation de l'expression des miRs est très peu connue, mais on sait notamment que celle-ci fait intervenir, à l'instar de la plupart des gènes codants, une ARN polymerase II : cette enzyme produit un transcrit primaire, appelé « pri-miR », qui est ensuite maturé par un complexe protéique contenant notamment les enzymes type Dicer. Cette maturation conduit tout d'abord à la formation d'un précurseur de miR appelé « pre-miR », ayant une structure secondaire en forme tige-boucle contenant le miR et sa séquence miR^{∗} complémentaire. Puis le précurseur est maturé, ce qui conduit à la formation d'un ARN double brin plus court contenant le miR et le miR*^{∗}*. Le miR est ensuite pris en charge par le complexe RISC qui clive l'ARNm du gène cible ou bien inhibe sa traduction.*

*Par ailleurs, il a été montré que la présence d'introns dans le transcrit primaire du microARN augmente l'expression du microARN mature (*Schwab et al., EMBO Rep., 14(7):615-21, 2013*). Cependant, du fait de difficultés expérimentales, les transcrits primaires de microARNs, ou pri-miRs, sont très peu étudiés.*

*Environ 50% des gènes eucaryotes possèdent, au sein de leur région 5'UTR (5' UnTranslated Region) en amont de la séquence codante, de petits cadres ouverts de lecture. Ces petits cadres ouverts de lecture (ou « uORFs » pour upstream ORFs) peuvent jouer un rôle de régulateur de la traduction, principalement en cis, en modulant la fixation et la vitesse des ribosomes sur l'ARNm, mais également en trans selon un mécanisme encore inconnu, par l'intermédiaire de peptides codés par lesdits uORFs (*Combier et al., Gene Dev, 22:1549-1559, 2008*). Par définition, les uORFS sont présents en amont de gènes codants.*

*Récemment, il a également été découvert de petits ORFs dans de longs ARN non codants intergéniques (lincRNAs) dont la fonction putative, si elle existe, n'est pas connue (*Ingolia et al., Cell, 147(4):789-802, 2011 ; Guttman & Rinn, Nature, 482(7385):339-46, 2012*).*

*Toutefois, aucun exemple n'a encore été rapporté concernant l'existence d'ORFs codant des peptides au sein de microARNs non codants. Jusqu'à présent, les microARNs, et par extension leur transcrit primaire, ont toujours été considérés, de par leur mode d'action particulier, comme des ARNs régulateurs non codants ne produisant aucun peptide.*

*L'un des aspects de l'objet de la demande* FR 13 60727 *est de proposer des peptides capables de moduler l'expression des microARNs.*

*Un autre aspect de l'objet de la demande* FR 13 60727 *est de proposer un moyen permettant de moduler l'expression d'un ou plusieurs gènes cibles d'un microARN.*

*L'objet de la demande* FR 13 60727 *présente l'avantage de permettre un contrôle plus facile et plus efficace de l'expression de gènes ciblés par les microARNs, à l'aide d'un moyen autre que le microARN.*

*L'objet de la demande* FR 13 60727 *concerne ainsi un procédé de détection et d'identification d'un micropeptide (miPEP) codé par une séquence nucléotidique contenue dans la séquence du transcrit primaire d'un microARN,*
*comprenant :*
- *a) une étape de détection d'un cadre ouvert de lecture de 15 à 303 nucléotides contenu dans la séquence du transcrit primaire dudit microARN, puis*
- *b) une étape de comparaison entre :*
   - *l'accumulation dudit microARN dans une cellule eucaryote déterminée exprimant ledit microARN,*
      *en présence d'un peptide codé par une séquence nucléotidique identique ou dégénérée par rapport à celle dudit cadre ouvert de lecture, ledit peptide étant présent dans la cellule indépendamment de la transcription du transcrit primaire dudit microARN, et*
      • *l'accumulation dudit microARN dans une cellule eucaryote de même type que la susdite cellule eucaryote déterminée exprimant ledit microARN, en absence dudit peptide,*
      *dans lequel, une modulation de l'accumulation dudit microARN en présence dudit peptide par rapport à l'accumulation dudit microARN en absence dudit peptide indique l'existence d'un micropeptide codé par ledit cadre ouvert de lecture.*

*Dans une première étape, le procédé de détection et d'identification d'un micropeptide consiste donc à détecter sur le transcrit primaire d'un microARN l'existence d'un cadre ouvert de lecture codant potentiellement un peptide.*

*La deuxième étape permet, quant à elle, de caractériser ledit peptide, c'est-à-dire de déterminer si ledit peptide correspond a un peptide réellement produit dans la cellule, en recherchant un effet dudit peptide sur l'accumulation dudit microARN.*

*Pour mettre en évidence un effet du peptide sur l'accumulation du microARN, une quantité importante de peptide est introduite dans une première cellule exprimant ledit microARN. L'accumulation du microARN dans cette première cellule est ensuite mesurée et comparée avec l'accumulation du microARN dans une deuxième cellule identique à la première, mais ne contenant pas ledit peptide.*

*L'observation d'une variation des quantités de microARN entre les cellules en présence et en absence du peptide indique ainsi (i) qu'il existe un peptide codé sur le transcrit primaire dudit microARN, (ii) que la séquence de ce peptide est codée par le cadre ouvert de lecture identifié sur le transcrit primaire dudit microARN, et (iii) que ledit peptide agit sur l'accumulation dudit microARN.*

*L'objet de la demande* FR 13 60727 *repose donc sur la double observation inattendue faite par les Inventeurs que d'une part, il existe des cadres ouverts de lecture susceptibles de coder des micropeptides présents sur les transcrits primaires de microARNs, et d'autre part que lesdits micropeptides sont capables de moduler l'accumulation desdits microARNs.*

*Dans la demande* FR 13 60727*, les termes « microARN », « microARN non codant » et « miR » sont équivalents et peuvent être utilisés l'un pour l'autre. Ils définissent des petites molécules d'ARN d'environ 21 nucléotides, qui ne sont pas traduites et ne conduisent pas à un peptide ou une protéine.*

*Cependant, sous cette forme mature, les microARNs assurent une fonction de régulation de certains gènes via des mécanismes post-transcriptionnels, comme par exemple par l'intermédiaire du complexe RISC.*

*Le transcrit primaire du microARN ou « pri-miR » correspond lui à la molécule d'ARN directement obtenue à partir de la transcription de la molécule d'ADN. Généralement, ce transcrit primaire subit une ou plusieurs modifications post-transcriptionnelles, qui entraînent par exemple une structure particulière de l'ARN ou un clivage de certaines parties de l'ARN par des phénomènes d'épissage, et qui conduisent à la forme précurseur du microARN ou « pre-miR », puis à la forme mature du microARN ou « miR ».*

*Les termes « micropeptides » et « miPEPs » (microRNA encoded PEPtides) sont équivalents et peuvent être utilisés l'un pour l'autre. Ils définissent un peptide qui est codé par un cadre ouvert de lecture présent sur le transcrit primaire d'un microARN, et qui est capable de moduler l'accumulation dudit microARN. Les micropeptides au sens de la demande* FR 13 60727 *ne doivent être entendus comme étant nécessairement des peptides de petite taille, dans la mesure où « micro » ne correspond pas à la taille du peptide.*

*Compte tenu de la dégénérescence du code génétique, un même micropeptide peut être codé par plusieurs séquences nucléotidiques. De telles séquences nucléotidiques, différentes entre elles d'au moins un nucléotide mais codant un même peptide, sont appelées « séquences dégénérées ».*

*Les termes « cadre ouvert de lecture » ou « ORF » (open reading frame) sont équivalents et peuvent être utilisés l'un pour l'autre. Ils correspondent à une séquence de nucléotides dans une molécule d'ADN ou d'ARN pouvant potentiellement coder un peptide ou une protéine: ledit cadre ouvert de lecture débute par un codon start, suivi d'une série de codons, et se termine par un codon stop.*

*Dans la demande* FR 13 60727*, les ORFs peuvent être dénommés de manière spécifique « miORFs » lorsque ceux-ci sont présents sur les transcrits primaires de microARN.*

*Dans la demande* FR 13 60727*, par « accumulation », on entend la production d'une molécule, telle qu'un microARN ou un micropeptide, dans la cellule.*

*Ainsi, la « modulation » de l'accumulation d'une molécule dans une cellule correspond à une modification de la quantité de cette molécule présente dans la cellule.*

*Dans un mode de réalisation, l'objet de la demande* FR 13 60727 *concerne un procédé de détection et d'identification d'un miPEP tel que défini ci-dessus, dans lequel la modulation de l'accumulation dudit microARN est une diminution ou une augmentation de l'accumulation dudit microARN, en particulier une augmentation.*

*Une « diminution de l'accumulation » correspond à une baisse de la quantité de ladite molécule dans la cellule.*

*A l'inverse, une « augmentation de l'accumulation » correspond à une hausse de la quantité de ladite molécule dans la cellule.*

*Dans un mode de réalisation avantageux, l'objet de la demande* FR 13 60727 *concerne un procédé de détection et d'identification d'un miPEP tel que défini ci-dessus, dans lequel la modulation de l'accumulation dudit microARN est une augmentation de l'accumulation dudit microARN.*

*Dans un mode de réalisation, l'objet de la demande* FR 13 60727 *concerne un procédé de détection et d'identification d'un miPEP tel que défini ci-dessus, dans lequel la présence dudit peptide dans la cellule résulte :*
- *de l'introduction dans la cellule d'un acide nucléique codant ledit peptide, ou*
- *de l'introduction dans la cellule dudit peptide*

*De manière à caractériser un miPEP, il est nécessaire de disposer d'un modèle cellulaire exprimant un microARN et dans lequel ledit peptide à tester est présent. Pour cela, il est possible d'introduire un peptide dans la cellule, soit en mettant la cellule en contact avec le dit peptide, soit en introduisant dans la cellule un acide nucléique codant ledit peptide, lequel acide nucléique sera alors traduit en peptide à l'intérieur de la cellule.*

*Dans un mode de réalisation, l'objet de la demande* FR 13 60727 *concerne un procédé de détection et d'identification d'un miPEP tel que défini ci-dessus, dans lequel le dit cadre ouvert de lecture de l'étape a) est contenu dans la partie 5' ou 3' dudit transcrit primaire du microARN, de préférence dans la partie 5'.*

*Les parties 5' ou 3' du transcrit primaire du microARN correspondent aux parties terminales de la molécule de l'ARN qui sont clivées au cours de la maturation du microARN.*

*Dans un mode de réalisation, l'objet de la demande* FR 13 60727 *concerne un procédé de détection et d'identification d'un miPEP tel que défini ci-dessus, dans lequel ledit microARN est présent dans une cellule végétale sauvage.*

*Dans la demande* FR 13 60727*, une cellule végétale sauvage correspond à une cellule végétale qui n 'a pas été modifiée génétiquement par l'homme.*

*Dans un mode de réalisation, l'objet de la demande* FR 13 60727 *concerne un procédé de détection et d'identification d'un miPEP tel que défini ci-dessus, dans lequel ladite cellule eucaryote déterminée, et ladite cellule eucaryote de même type que la susdite cellule eucaryote déterminée, utilisées à l'étape b, sont des cellules végétales, de préférence des cellules de Medicago truncatula ou d'Arabidopsis thaliana.*

*Dans le procédé de détection et d'identification d'un micropeptide tel que défini ci-dessus, après avoir identifié un ORF susceptible de coder un peptide sur le transcrit primaire d'un microARN, il est nécessaire de disposer d'un modèle cellulaire possédant ledit microARN et ledit peptide, pour pouvoir démontrer un effet éventuel du peptide sur ledit microARN. Deux options sont donc envisageables :*
- *le modèle cellulaire dans lequel a été identifié le miORF et celui dans lequel est mis en évidence l'effet du peptide sur le miR sont identiques, ou*
- *le modèle cellulaire dans lequel a été identifié le miORF et celui dans lequel est mis en évidence l'effet du peptide sur le miR sont différents.*

*Dans la première option, le modèle cellulaire utilisé pour observer un effet du peptide est le même que celui dans lequel le transcrit primaire dudit microARN a été isolé. Dans ce modèle cellulaire, les cellules eucaryotes déterminées contiennent naturellement ledit microARN et seul le peptide à tester doit être introduit dans ces cellules. Dans ce contexte, ledit microARN est qualifié « d'origine endogène » car celui-ci existe naturellement dans les cellules. Néanmoins, dans une cellule, d'autres copies d'un microARN d'origine endogène peuvent être ajoutées, comme par exemple en introduisant dans la cellule un vecteur codant ledit microARN d'origine endogène.*

*Dans la deuxième option, le modèle cellulaire utilisé pour observer un effet du peptide est différent de celui dans lequel le transcrit primaire dudit microARN a été isolé. Dans ce modèle cellulaire, les cellules eucaryotes déterminées ne contiennent ni le microARN, ni le peptide à tester. Ces deux éléments doivent donc être introduits dans ces cellules. Dans ce contexte, ledit microARN est qualifié « d'origine exogène » car celui-ci n'existe pas naturellement dans les cellules.*

*Dans un mode de réalisation, l'objet de la demande* FR 13 60727*concerne un procédé de détection et d'identification d'un miPEP tel que défini ci-dessus, dans lequel ledit microARN est d'origine endogène dans ladite cellule eucaryote et dans ladite cellule eucaryote de même type que la susdite cellule eucaryote déterminée, utilisées à l'étape b).*

*Dans un mode de réalisation, l'objet de la demande* FR 13 60727 *concerne un procédé de détection et d'identification d'un miPEP tel que défini ci-dessus dans lequel ledit microARN est d'origine exogène dans ladite cellule eucaryote et dans ladite cellule eucaryote de même type que la susdite cellule eucaryote déterminée, utilisées à l'étape b), lesdites cellules eucaryotes contenant un vecteur permettant l'expression dudit microARN.*

*Dans un mode de réalisation, l'objet de la demande* FR 13 60727 *concerne un procédé de détection et d'identification d'un miPEP tel que défini ci-dessus, dans lequel l'accumulation dudit microARN est déterminée en mettant en œuvre une RT-PCR quantitative ou un Northern blot.*

*Dans un mode de réalisation, l'objet de la demande* FR 13 60727 *concerne un procédé de détection et d'identification d'un miPEP tel que défini ci-dessus, dans lequel l'accumulation dudit microARN est déterminée en mettant en œuvre une puce à ADN ou à ARN.*

*L'accumulation dudit microARN peut être déterminée à l'aide des techniques de biologie moléculaire permettant le dosage de molécules d'acides nucléiques spécifiques.*

*Dans un autre aspect, l'objet de la demande* FR 13 60727 *concerne également un procédé de détection et d'identification d'un microARN dont la séquence du transcrit primaire contient une séquence nucléotidique codant un miPEP,*
*comprenant :*
- *a) une étape de détection d'un cadre ouvert de lecture de 15 à 303 nucléotides contenu dans la séquence du transcrit primaire dudit microARN, puis*
- *b) une étape de comparaison entre :*
   - *l'accumulation dudit microARN dans une cellule eucaryote déterminée exprimant ledit microARN,*
      *en présence d'un peptide codé par une séquence nucléotidique identique ou dégénérée par rapport à celle dudit cadre ouvert de lecture, ledit peptide étant présent dans la cellule indépendamment de la transcription du transcrit primaire dudit microARN, et*
   - *l'accumulation dudit microARN dans une cellule eucaryote, de même type que la susdite cellule eucaryote déterminée exprimant ledit microARN, en absence dudit peptide,*
   *dans lequel, une modulation de l'accumulation dudit microARN en présence dudit peptide par rapport à l'accumulation dudit microARN en absence dudit peptide indique l'existence d'un microARN dont le transcrit primaire contient une séquence nucléotidique codant un micropeptide.*

*Dans un mode de réalisation, l'objet de la demande* FR 13 60727 *concerne un procédé de détection et d'identification d'un microARN tel que défini ci-dessus, dans lequel la modulation de l'accumulation dudit microARN est une diminution ou une augmentation de l'accumulation dudit microARN, en particulier une augmentation.*

*Dans un mode de réalisation, l'objet de la demande* FR 13 60727 *concerne un procédé de détection et d'identification d'un microARN tel que défini ci-dessus, dans lequel la présence dudit peptide dans la cellule résulte :*
- *de l'introduction dans la cellule d'un acide nucléique codant ledit peptide, ou*
- *de l'introduction dans la cellule de ledit peptide.*

*Dans un mode de réalisation, l'objet de la demande* FR 13 60727 *concerne un procédé de détection et d'identification d'un microARN tel que défini ci-dessus, dans lequel le dit cadre ouvert de lecture de l'étape a) est contenu dans la partie 5' ou 3' dudit transcrit primaire du microARN, de préférence dans la partie 5'.*

*Dans un mode de réalisation, l'objet de la demande* FR 13 60727 *concerne un procédé de détection et d'identification d'un microARN tel que défini ci-dessus, dans lequel ledit microARN est présent dans une cellule végétale sauvage.*

*Dans un mode de réalisation, l'objet de la demande* FR 13 60727 *concerne un procédé de détection et d'identification d'un microARN tel que défini ci-dessus, dans lequel ladite cellule eucaryote, et ladite cellule eucaryote de même type que la susdite cellule eucaryote déterminée, utilisées à l'étape b) sont des cellules végétales, de préférence des cellules de Medicago truncatula.*

*Dans un mode de réalisation, l'objet de la demande* FR 13 60727 *concerne un procédé de détection et d'identification d'un microARN tel que défini ci-dessus, dans lequel ledit microARN est d'origine endogène dans ladite cellule eucaryote et dans ladite cellule eucaryote de même type que la susdite cellule eucaryote déterminée, utilisées à l'étape b).*

*Dans un mode de réalisation, l'objet de la demande* FR 13 60727 *concerne un procédé de détection et d'identification d'un microARN tel que défini ci-dessus dans lequel ledit microARN est d'origine exogène dans ladite cellule eucaryote et dans ladite cellule eucaryote de même type que la susdite cellule eucaryote déterminée, utilisées à l'étape b), lesdites cellules eucaryotes contenant un vecteur permettant l'expression dudit microARN.*

*Dans un mode de réalisation, l'objet de la demande* FR 13 60727 *concerne un procédé de détection et d'identification d'un microARN tel que défini ci-dessus, dans lequel l'accumulation dudit microARN est déterminée en mettant en œuvre une RT-PCR quantitative ou un Northern blot.*

*Dans un mode de réalisation, l'objet de la demande* FR 13 60727 *concerne un procédé de détection et d'identification d'un microARN tel que défini ci-dessus, dans lequel l'accumulation dudit microARN est déterminée en mettant en œuvre une puce à ADN ou à ARN.*

*Dans un autre aspect, l'objet de la demande* FR 13 60727 *concerne un miPEP tel qu'obtenu par la mise en œuvre du procédé tel que défini ci-dessus.*

*Un autre aspect de l'objet de la demande* FR 13 60727 *concerne également un miPEP de 4 à 100 acides aminés, de préférence de 4 à 40 acides aminés, codé par une séquence nucléotidique contenue dans le transcrit primaire d'un microARN, ledit miPEP étant capable de moduler l'accumulation dudit microARN dans une cellule eucaryote.*

*Par ailleurs, il convient de noter que plusieurs miORFS peuvent être identifiés sur le transcrit primaire d'un microARN, indiquant qu'un transcrit primaire de microARN peut potentiellement coder plusieurs miPEPs.*

*Il convient également de noter que l'effet d'un miPEP est généralement spécifique d'un seul microARN, à savoir celui résultant du transcrit primaire codant ledit miPEP.*

*Dans un mode de réalisation, l'objet de la demande* FR 13 60727 *concerne un miPEP tel que défini ci-dessus, ladite séquence nucléotidique étant contenue dans la partie 5' ou 3' dudit transcrit primaire d'un microARN, de préférence dans la partie 5'.*

*Dans un mode de réalisation, l'objet de la demande* FR 13 60727 *concerne un miPEP tel que défini ci-dessus, ladite séquence nucléotidique correspondant au premier cadre ouvert de lecture présent sur ledit transcrit primaire d'un microARN.*

*Dans un mode de réalisation, l'objet de la demande* FR 13 60727 *concerne un miPEP tel que défini ci-dessus, ledit miPEP possédant un point isoélectrique basique, de préférence supérieur à 8.*

*Dans un autre aspect, l'objet de la demande* FR 13 60727 *concerne une molécule d'acide nucléique codant un miPEP tel que défini ci-dessus.*

*Dans autre aspect, l'objet de la demande* FR 13 60727 *concerne un vecteur comprenant au moins une molécule d'acide nucléique tel que définie ci-dessus.*

*Dans un autre aspect, l'objet de la demande* FR 13 60727 *concerne également l'utilisation d'au moins :*
- *un miPEP tel que défini ci-dessus,*
- *un acide nucléique codant ledit miPEP, ou*
- *un vecteur contenant ledit acide nucléique,*
*pour moduler l'expression d'au moins un gène dans une cellule eucaryote déterminée, ladite cellule eucaryote déterminée étant capable d'exprimer un microARN, dont le transcrit primaire contient au moins une séquence nucléotidique codant ledit au moins un miPEP et dont l'accumulation est modulée par ledit au moins un miPEP, l'expression dudit au moins un gène étant régulée par ledit microARN.*

*Dans un autre aspect, l'objet de la demande* FR 13 60727 *concerne également l'utilisation d'au moins :*
- *un miPEP de 4 à 100 acides aminés, de préférence de 4 à 40 acides aminés, codé par une séquence nucléotidique contenue dans le transcrit primaire d'un microARN, ledit miPEP étant capable de moduler l'accumulation dudit microARN dans une cellule eucaryote,*
- *un acide nucléique codant ledit miPEP, ou*
- *un vecteur contenant ledit acide nucléique,*
*pour moduler l'expression d'au moins un gène dans une cellule eucaryote déterminée, ladite cellule eucaryote déterminée étant capable d'exprimer un microARN, dont le transcrit primaire contient au moins une séquence nucléotidique codant ledit au moins un miPEP et dont l'accumulation est modulée par ledit au moins un miPEP, l'expression dudit au moins un gène étant régulée par ledit microARN.*

*L'objet de la demande* FR 13 60727 *repose sur l'observation surprenante faite par les Inventeurs qu'il est possible de moduler l'expression d'un ou plusieurs gènes cibles d'un même microARN en modulant l'accumulation dudit microARN à l'aide d'un miPEP.*

*Dans un mode de réalisation, l'objet de la demande* FR 13 60727 *concerne l'utilisation telle que définie ci-dessus dans laquelle ladite cellule eucaryote déterminée est une cellule végétale.*

*Dans un mode de réalisation, l'objet de la demande* FR 13 60727 *concerne l'utilisation telle que définie ci-dessus dans laquelle ledit microARN et ledit gène sont d'origine endogène dans ladite cellule eucaryote déterminée.*

*Dans un mode de réalisation, l'objet de la demande* FR 13 60727 *concerne l'utilisation telle que définie ci-dessus dans laquelle ledit microARN et ledit gène sont d'origine exogène dans ladite cellule eucaryote déterminée, ladite cellule eucaryote déterminée contenant au moins un vecteur permettant l'expression dudit microARN et dudit gène.*

*Dans la demande* FR 13 60727*, les expressions « d'origine endogène » et « d'origine exogène » sont utilisées pour distinguer lesdits microARNs et*/*ou les gènes d'espèces différentes, étant donné la conservation des séquences entre espèces.*

*Ainsi, le terme « d'origine endogène » indique que le microARN et*/*ou le gène peuvent être présents de manière naturelle dans la cellule en question. De manière artificielle, d'autres copies du microARN et*/*ou du gène d'origine endogène peuvent néanmoins être ajoutées dans la cellule en question, comme par exemple par clonage.*

*A l'inverse, le terme « d'origine exogène » indique que le microARN et*/*ou le gène ne sont jamais présents naturellement dans la cellule en question. Il s'agit d'un microARN et*/*ou d'un gène identifié dans un autre type cellulaire ou dans un organisme d'une autre espèce, ce microARN et*/*ou ce gène sont donc nécessairement introduits artificiellement dans la cellule en question.*

*Dans la demande* FR 13 60727*, une cellule transformée génétiquement peut donc contenir 2 groupes de microARNs et*/*ou de gènes potentiellement proches en termes de séquence, l'un d'origine endogène et l'autre d'origine exogène.*

*Dans un autre aspect, l'objet de la demande* FR 13 60727 *concerne un procédé de modulation de l'expression d'un gène régulée par un microARN dans une cellule eucaryote,*
*comprenant la mise en œuvre d'une étape d'accumulation d'un miPEP dans ladite cellule eucaryote,*
   *ledit miPEP ayant :*
   - *une taille de 4 à 100 acides aminés, de préférence 4 à 20 acides aminés, et*
   - *une séquence peptidique identique à celle codée par une séquence nucléotidique contenue dans le transcrit primaire d'un microARN régulant l'expression dudit gène, et*
   - *étant capable de moduler l'accumulation dudit microARN,*
*dans lequel, l'accumulation dudit miPEP dans ladite cellule eucaryote induit une modulation de l'expression dudit gène par rapport à l'expression dudit gène sans accumulation dudit miPEP.*

*Dans un mode de réalisation, l'objet de la demande* FR 13 60727 *concerne un procédé de modulation de l'expression d'un gène tel que défini ci-dessus, dans lequel l'accumulation dudit miPEP dans la cellule résulte :*
- *de l'introduction dans la cellule d'un acide nucléique codant ledit miPEP, ou*
- *de l'introduction dans la cellule dudit miPEP.*

*Dans un mode de réalisation, l'objet de la demande* FR 13 60727 *concerne un procédé de modulation de l'expression d'un gène tel que défini ci-dessus dans lequel ladite cellule eucaryote est une cellule végétale.*

*Dans un mode de réalisation, l'objet de la demande* FR 13 60727 *concerne un procédé de modulation de l'expression d'un gène tel que défini ci-dessus dans lequel ledit microARN et ledit gène sont d'origine endogène dans ladite cellule eucaryote.*

*Dans un mode de réalisation, l'objet de la demande* FR 13 60727 *concerne un procédé de modulation de l'expression d'un gène tel que défini ci-dessus dans lequel ledit microARN et ledit gène sont d'origine exogène dans ladite cellule eucaryote, ladite cellule eucaryote contenant au moins un vecteur permettant l'expression dudit microARN et dudit gène.*

*Dans un autre aspect, l'objet de la demande* FR 13 60727 *concerne une cellule eucaryote modifiée contenant un peptide identique à un miPEP tel que défini ci-dessus, lequel peptide est présent dans ladite cellule eucaryote indépendamment de la transcription du transcrit primaire du microARN portant la séquence nucléotidique codant ledit miPEP.*

*Dans la demande* FR 13 60727*, le terme « cellule eucaryote modifiée » signifie que ladite cellule eucaryote contient un miPEP introduit artificiellement dans la cellule, que ce soit en tant que peptide, ou par l'intermédiaire d'un vecteur codant ledit miPEP.*

*Dans un mode de réalisation, l'objet de la demande* FR 13 60727 *concerne une cellule eucaryote modifiée telle que définie ci-dessus, dans laquelle ledit microARN est d'origine endogène.*

*Dans un autre mode de réalisation, l'objet de la demande* FR 13 60727 *concerne une cellule eucaryote modifiée telle que définie ci-dessus dans laquelle ledit microARN est d'origine exogène, ladite cellule eucaryote modifiée contenant un vecteur permettant l'expression dudit microARN.*

*Dans un mode de réalisation, l'objet de la demande* FR 13 60727 *concerne une cellule eucaryote modifiée telle que définie ci-dessus, ladite cellule étant une cellule végétale.*

*Dans un autre aspect, l'objet de la demande* FR 13 60727 *concerne une plante comprenant au moins une cellule eucaryote modifiée telle que définie ci-dessus.*

*Dans un autre aspect, l'objet de la demande* FR 13 60727 *concerne une composition comprenant au moins :*
- *un miPEP tel que défini ci-dessus,*
- *un acide nucléique codant ledit miPEP, ou*
- *un vecteur contenant ledit acide nucléique.*

*Dans un autre aspect, l'objet de la demande* FR 13 60727 *concerne une composition pesticide comprenant au moins :*
- *un miPEP tel que défini ci-dessus,*
- *un acide nucléique codant ledit miPEP, ou*
- *un vecteur contenant ledit acide nucléique.*

*Dans un autre aspect, l'objet de la demande* FR 13 60727 *concerne une composition phytopharmaceutique comprenant au moins :*
- *un miPEP tel que défini ci-dessus,*
- *un acide nucléique codant ledit miPEP, ou*
- *un vecteur contenant ledit acide nucléique.*

*Dans un autre aspect, l'objet de la demande* FR 13 60727 *concerne une composition élicitrice comprenant au moins :*
- *un miPEP tel que défini ci-dessus,*
- *un acide nucléique codant ledit miPEP, ou*
- *un vecteur contenant ledit acide nucléique.*

*Par « composition élicitrice », on désigne une composition capable de conférer à la plante une meilleure aptitude à la symbiose ou une meilleure résistance à différents stress, qu'ils soient de nature thermiques, hydriques ou chimiques.*

*A cet effet, l'objet de la demande* FR 13 60727 *concerne également des compositions agissant sur la croissance (inhibition de la croissance ou au contraire augmentation de la croissance) et la physiologie (meilleure aptitude à mycorhizer, noduler, meilleure tolérance à différents stress) de la plante.*

*Dans un autre aspect, l'objet de la demande* FR 13 60727 *concerne une composition herbicide comprenant au moins :*
- *un miPEP tel que défini ci-dessus,*
- *un acide nucléique codant ledit miPEP, ou*
- *un vecteur contenant ledit acide nucléique.*

*Dans un autre aspect, l'objet de la demande* FR 13 60727 *concerne une composition insecticide comprenant au moins :*
- *un miPEP tel que défini ci-dessus,*
- *un acide nucléique codant ledit miPEP, ou*
- *un vecteur contenant ledit acide nucléique.*

*Dans un autre aspect, l'objet de la demande* FR 13 60727 *concerne l'utilisation d'une composition telle que définie ci-dessus, en tant qu 'herbicide pour éliminer les plantes ou ralentir leur croissance, de préférence en tant qu'herbicide spécifique d'une espèce ou d'un genre de plantes.*

*Dans un autre aspect, l'objet de la demande* FR 13 60727 *concerne l'utilisation d'une composition telle que définie ci-dessus, en tant qu'agent phytopharmaceutique,*
- *pour favoriser la croissance et*/*ou le développement des plantes,*
   *notamment pour la modulation des paramètres physiologiques d'une plante, en particulier la biomasse, la surface foliaire, la floraison, le calibre du fruit, la production et*/*ou la sélection de semence végétales, en particulier pour contrôler la parthénocarpie ou la monoecie d'une plante, ou pour la modification des paramètres physiologiques de semences végétales, en particulier la germination, l'implantation racinaire et la résistance au stress hydrique,*
- *ou pour prévenir ou traiter les maladies des plantes,*
*en particulier pour favoriser la résistance aux maladies infectieuses.*

*Dans un autre aspect, l'objet de la demande* FR 13 60727 *concerne l'utilisation d'une composition telle que définie ci-dessus, pour moduler les paramètres physiologiques d'une plante, en particulier la biomasse, la surface foliaire, ou le calibre du fruit.*

*Dans un mode de réalisation, l'objet de la demande* FR 13 60727 *concerne l'utilisation d'une composition telle que définie ci-dessus, pour l'éclaircissage de vergers afin d'augmenter le calibre des fruits.*

*Dans un mode de réalisation, l'objet de la demande* FR 13 60727 *concerne l'utilisation d'une composition telle que définie ci-dessus, pour la production et*/*ou la sélection de semences végétales, ladite composition étant utilisée pour contrôler la parthénocarpie ou la monœcie d'une plante.*

*Dans un mode de réalisation, l'objet de la demande* FR 13 60727 *concerne l'utilisation d'une composition telle que définie ci-dessus, ladite composition étant administrée à ladite plante par la voie foliaire ou par la voie racinaire.*

*Dans un mode de réalisation, l'objet de la demande* FR 13 60727 *concerne l'utilisation d'une composition telle que définie ci-dessus, pour la production et*/*ou la sélection de semences végétales.*

*Dans un mode de réalisation, l'objet de la demande* FR 13 60727 *concerne l'utilisation d'une composition telle que définie ci-dessus, dans laquelle ladite composition est utilisée pour modifier les paramètres physiologiques desdites semences végétales, en particulier l'implantation racinaire, la germination et la résistance au stress hydrique.*

*Dans un mode de réalisation, l'objet de la demande* FR 13 60727 *concerne l'utilisation d'une composition telle que définie ci-dessus, dans laquelle ladite composition est appliquée par enrobage ou pelliculage sur lesdites semences végétales.*

*Dans un autre aspect, l'objet de la demande* FR 13 60727 *concerne l'utilisation d'une composition telle que définie ci-dessus, en tant que pesticide, pour éliminer les organismes nuisibles des plantes ou susceptibles d'être classés comme tels, notamment en tant qu'insecticide, arachnicide, limacide ou rodonticide.*

*Dans un mode de réalisation, l'objet de la demande* FR 13 60727 *concerne l'utilisation d'une composition telle que définie ci-dessus, en tant qu'insecticide.*

*Dans un mode de réalisation, l'objet de la demande* FR 13 60727 *concerne l'utilisation d'une composition telle que définie ci-dessus, pour éliminer les insectes ravageurs.*

*Dans un mode de réalisation, l'objet de la demande* FR 13 60727 *concerne l'utilisation d'une composition telle que définie ci-dessus, pour éliminer les espèces animales classées nuisibles ou susceptibles d'être classées comme telles, en particulier les muridae, notamment le rat.*

*Dans un autre aspect, l'objet de la demande* FR 13 60727 *concerne l'utilisation d'une composition telle que définie ci-dessus, dans laquelle ladite composition est appliquée sur une plante pour la protéger d'insectes ravageurs.*

Les figures et les exemples suivants illustreront mieux l'invention, sans pour autant en limiter sa portée.

### LEGENDES DES FIGURES

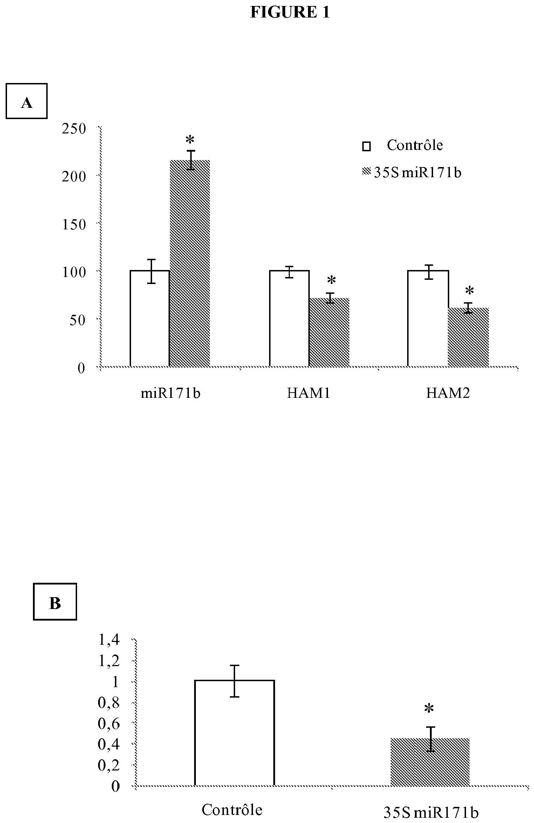

**FIGURE 1****. Effets de la surexpression du miR171b (miR171b identifié chez *Medicago truncatula)* sur l'expression des gènes *HAM1* et *HAM2* (A) ou sur le nombre de racines latérales (B) chez *M. truncatula.***
   (A) L'axe des ordonnées indique l'expression relative du miR171b (colonnes de gauche), de *HAM1* (colonnes du milieu) ou de *HAM2* (colonnes de droite) chez une plante témoin (colonnes blanches) ou chez une plante dans laquelle est surexprimé miR171b (colonnes noires). La barre d'erreur correspond à l'erreur standard à la moyenne (nombre d'individus = 10). La surexpression de miR171b induit une diminution de l'expression des gènes *HAM1* et *HAM2.*
   (B) L'axe des ordonnées indique le nombre moyen de racines latérales observées chez une plante témoin (colonne blanche) ou chez une plante dans laquelle est surexprimé miR171b (colonne noire). La barre d'erreur correspond à l'erreur standard à la moyenne (nombre d'individus = 100). La surexpression de miR171b entraîne une réduction du nombre de racines latérales.

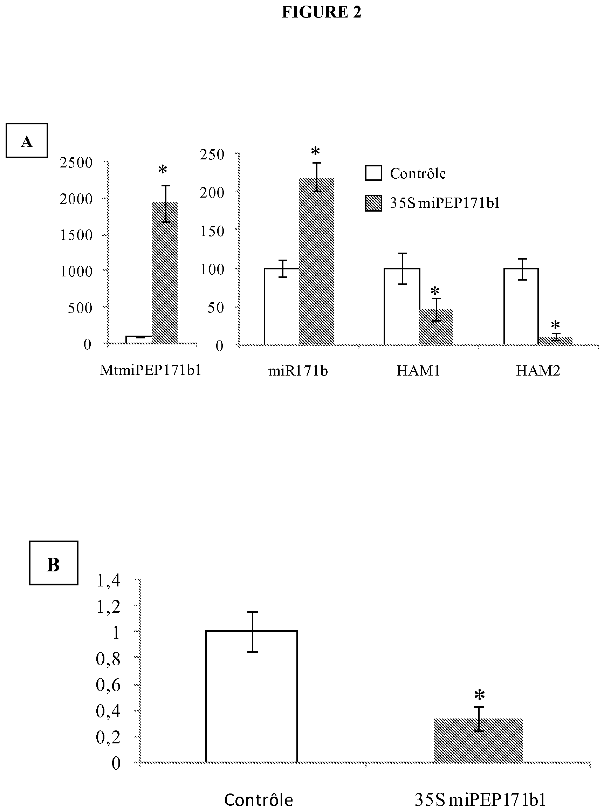

**FIGURE 2****. Effets de la surexpression du miPEP171b sur l'expression du miR171b et des gènes *HAM1* et *HAM2* (A) ou sur le nombre racines latérales (B) chez *M. truncatula.***
   (A) L'axe des ordonnées indique l'expression relative du miPEP171b (graphique de gauche), du miR171b (graphique de droite, colonnes de gauche), de *HAM1* (accession n°MtGI9- TC114268) (graphique de droite, colonnes du milieu) ou de *HAM2* (accession n° MtGI9- TC120850) (graphique de droite, colonnes de droite) chez une plante témoin (colonnes blanches) ou chez une plante dans laquelle est surexprimé miPEP171b (colonnes noires). La barre d'erreur correspond à l'erreur standard à la moyenne (nombre d'individus = 10). La surexpression de miPEP171b induit une augmentation de l'accumulation de miR171b, ainsi qu'une diminution de l'expression des gènes *HAM1* et *HAM2.*
   (B) L'axe des ordonnées indique le nombre moyen de racines latérales observées chez une plante témoin (colonne blanche) ou chez une plante dans laquelle est surexprimé miPEP171b (colonne noire). La barre d'erreur correspond à l'erreur standard à la moyenne (nombre d'individus = 100). La surexpression de miPEP171b entraîne une réduction du nombre de racines latérales.

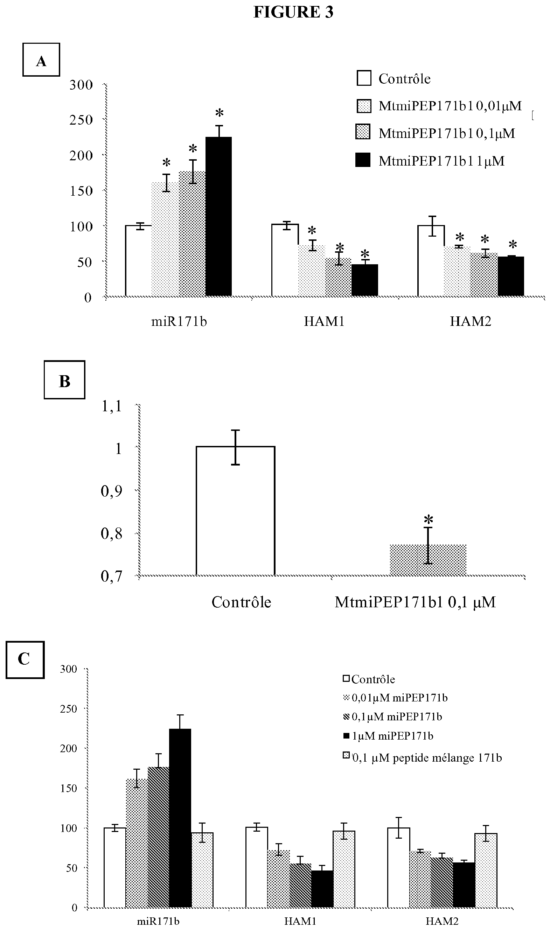

**FIGURE 3****. Effets du miPEP171b sur l'expression du miR171b et des gènes *HAM1* et *HAM2* (A) et sur le nombre racines latérales (B) chez *M. truncatula.***
   (A) L'axe des ordonnées indique l'expression relative du miR171b (colonnes de gauche), de *HAM1* (colonnes du milieu) ou de *HAM2* (colonnes de droite) chez une plante témoin (colonnes blanches) ou chez une plante cultivée sur milieu contenant le miPEP171b à 0,01 µM (colonnes gris clair), 0,1 µM (colonnes gris foncé) ou 1µM (colonnes noires). La barre d'erreur correspond à l'erreur standard à la moyenne (nombre d'individus = 10). L'application du miPEP171b à différentes concentrations induit une augmentation de l'accumulation de miR171b, ainsi qu'une diminution de l'expression des gènes *HAM1* et *HAM2.*
   (B) L'axe des ordonnées indique le nombre moyen de racines latérales observées chez une plante témoin (colonne blanche) ou chez une plante cultivée sur milieu contenant le miPEP171b à 0,1 µM pendant 5 jours et 1 fois par jour (colonne noire). La barre d'erreur correspond à l'erreur standard à la moyenne (nombre d'individus = 100). L'application du miPEP 171b à 0,1 µM entraîne une réduction du nombre de racines latérales.
   (C) L'axe des ordonnées indique l'expression relative du MtmiR171b (colonnes de gauche), de *HAM1* (colonnes du milieu) ou de *HAM2* (colonnes de droite) chez une plante témoin (colonnes blanches) ou chez une plante traitée par arrosage pendant 5 jours et 1 fois par jour avec le MtmiPEP171bl à 0,01 µM (colonnes gris), 0,1 µM (colonnes gris foncé) ou 1µM (colonnes noires) ou avec 0,01 µM d'un peptide mélange (colonnes gris clair) dont la composition en acides aminés est identique au miPEP171b mais dont la séquence est différente. La barre d'erreur correspond à l'erreur standard à la moyenne (nombre d'individus = 10).

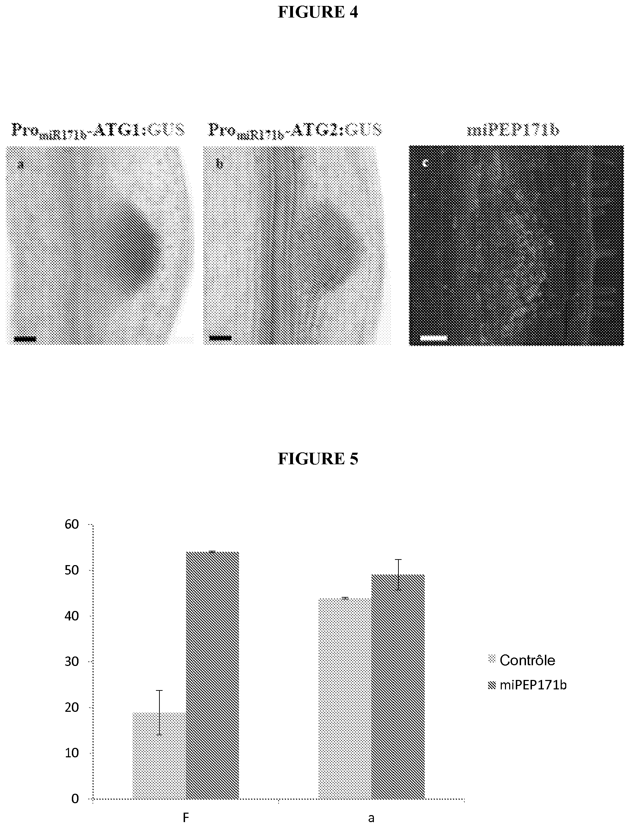

**FIGURE 4****. Immunolocalisation**
   Les racines de *Medicago truncatula* ont été transformées pour exprimer des fusions entre la protéine GUS (en bleu) et l'ATG du miPEP171b (Pro_{miR171b}-ATG1:GUS) ou bien l'ATG2 (deuxième ATG se trouvant sur le précurseur, après le miPEP) (Pro_{miR171b}-ATG2:GUS). Un marquage a été également réalisé avec un anticorps anti-miPEP171b (miPEP171b). L'immunolocalisation du miPEP171b dans les racines de *M. truncatula* révèle la présence du miPEP171b dans les sites d'initiation des racines latérales, montrant une co-localisation entre le microARN et le miPEP correspondant.
**FIGURE 5****. Effets du miPEP171b sur la colonisation de *M. truncatula* par le champignon *Rhizophagus irregularis***
   L'axe des ordonnées indique le pourcentage de colonisation (à gauche) et l'abondance des arbuscules (à droite) dans des racines de *M. truncatula* traitées par un solvant (contrôle, barres claires) ou par un solvant contenant 0,1 µM de miPEP171b (miPEP171b, barres foncées).
   La barre d'erreur correspond à l'erreur standard à la moyenne (nombre d'individus = 15).

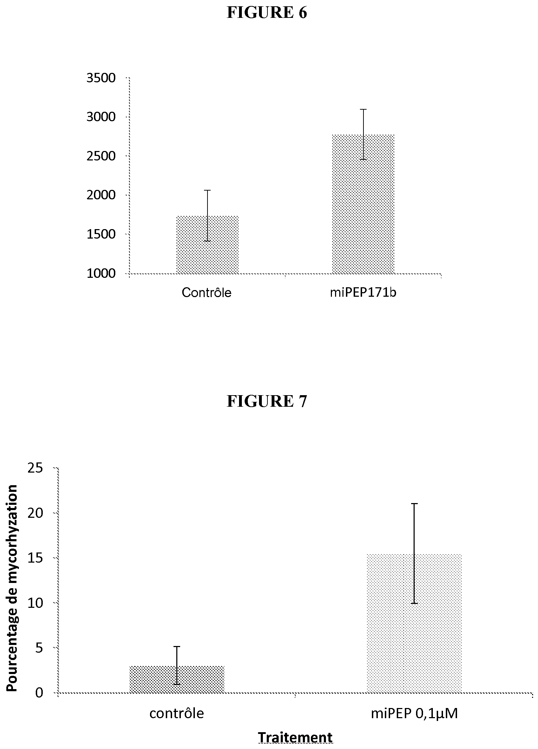

**FIGURE 6****. Effets du miPEP171b sur l'aire des arbuscules formées par le champignon *Rhizophagus irregularis* chez *M. truncatula***
   L'axe des ordonnées indique l'aire des arbuscules (mesurées en unités arbitraires) dans des racines de *M. truncatula* traitées par un solvant (contrôle) ou par un solvant contenant 0,1 µM de miPEP171b (miPEP171b).
   La barre d'erreur correspond à l'erreur standard à la moyenne (nombre d'individus = 15).
**FIGURE 7****. Effet du miPEP171b sur le taux de de mycorhization de *M. truncatula* par le champignon *Rhizophagus irregularis***
   L'axe des ordonnées indique le taux de mycorhization dans des racines de *M. truncatula* traitées 5 semaines post inoculation par un solvant (barre de gauche) ou par un solvant contenant 0,1 µM de miPEP171b (miPEP, barre de droite).
   La barre d'erreur correspond à l'erreur standard à la moyenne (nombre d'individus = 12).

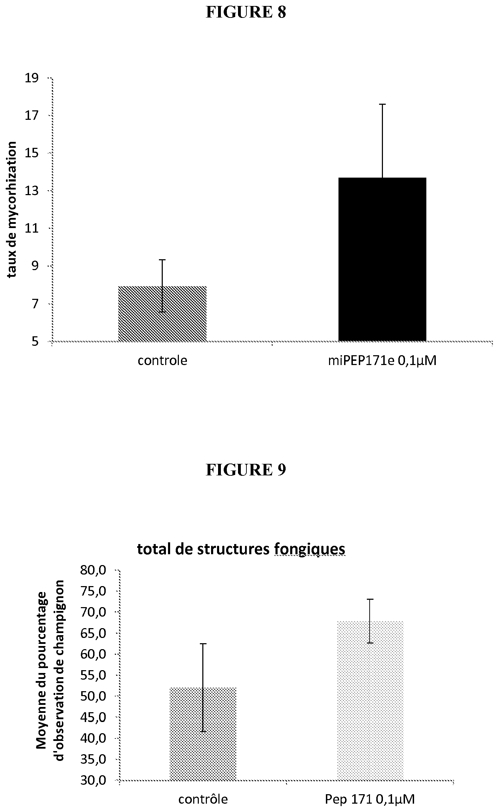

**FIGURE 8****. Effet du slmiPEP171e sur le taux de mycorhization de *Solanum lycopersicum***
   L'axe des ordonnées indique le taux de mycorhization de plantes de *Solanum lycopersicum* traitées 12 semaines post inoculation par un solvant (barre de gauche) ou par un solvant contenant 0,1 µM de slmiPEP171e (miPEP, barre de droite).
   La barre d'erreur correspond à l'erreur standard à la moyenne (nombre d'individus = 12).
**FIGURE 9****. Effets du ljmiPEP171b sur le nombre de structures fongiques chez *Lotus japonicus***
   L'axe des ordonnées indique le nombre de structures fongiques chez une plante traitée 5 semaines post inoculation par un solvant (barre de gauche) ou par un solvant contenant 0,1 µM de ljmiPEP171b (Pep 171, barre de droite).
   La barre d'erreur correspond à l'erreur standard à la moyenne (nombre d'individus = 10 plantes contrôles, 12 plantes traitées).

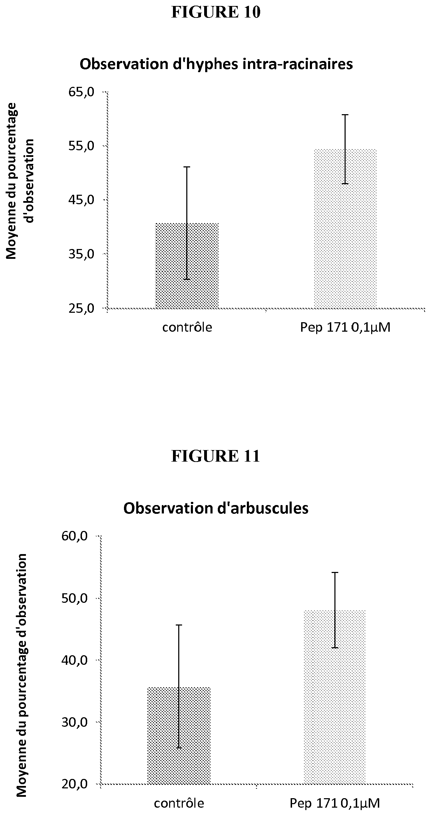

**FIGURE 10****. Effets du ljmiPEP171b sur le nombre d'hyphes intra-racinaires chez *Lotus japonicus***
   L'axe des ordonnées indique le nombre d'hyphes intraracinaires chez une plante traitée 5 semaines post inoculation par un solvant (barre de gauche) ou par un solvant contenant 0,1 µM de ljmiPEP171b (Pep 171, barre de droite).
   La barre d'erreur correspond à l'erreur standard à la moyenne (nombre d'individus = 10 plantes contrôles, 12 plantes traitées).
**FIGURE 11****. Effets du limiPEP171b sur le nombre d'arbuscules chez *Lotus japonicus***
   L'axe des ordonnées indique le nombre d'arbuscules chez une plante traitée 5 semaines post inoculation par un solvant (barre de gauche) ou par un solvant contenant 0,1 µM de ljmiPEP171b (Pep 171, barre de droite).
   La barre d'erreur correspond à l'erreur standard à la moyenne (nombre d'individus = 10 plantes contrôles, 12 plantes traitées).

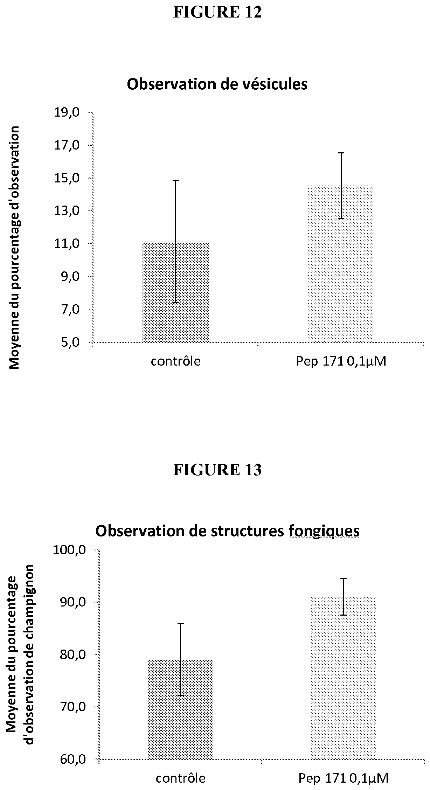

**FIGURE 12****. Effets du ljmiPEP171b sur le nombre de vésicules chez *Lotus japonicus***
   L'axe des ordonnées indique le nombre de vésicules chez une plante traitée 5 semaines post inoculation par un solvant (barre de gauche) ou par un solvant contenant 0,1 µM de ljmiPEP171b (Pep 171, barre de droite).
   La barre d'erreur correspond à l'erreur standard à la moyenne (nombre d'individus = 10 plantes contrôles, 12 plantes traitées).
**FIGURE 13****. Effets du osmiPEP171i sur le nombre de structures fongiques chez *Oryza sativa***
   L'axe des ordonnées indique le nombre de structures fongiques chez une plante traitée 5 semaines post inoculation par un solvant (barre de gauche) ou par un solvant contenant 0,1 µM de ljmiPEP171b (Pep 171, barre de droite).
   La barre d'erreur correspond à l'erreur standard à la moyenne (nombre d'individus = 5 plantes contrôles, 5 plantes traitées).

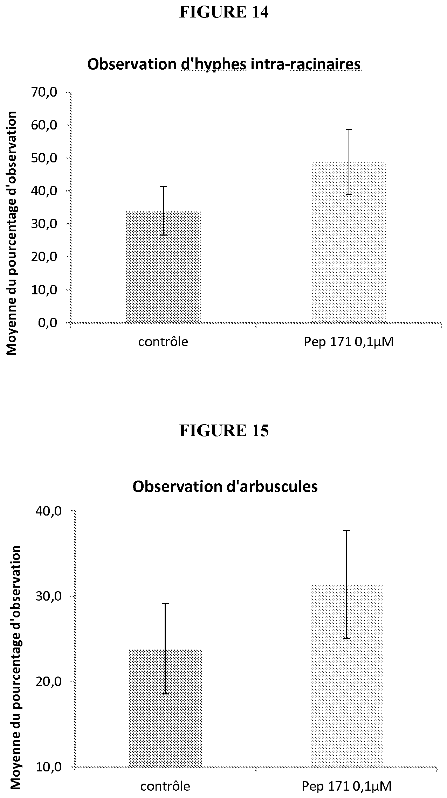

**FIGURE 14****. Effets du osmiPEP171i sur le nombre d'hyphes intra-racinaires chez *Oryza sativa***
   L'axe des ordonnées indique le nombre d'hyphes intraracinaires chez une plante traitée 5 semaines post inoculation par un solvant (barre de gauche) ou par un solvant contenant 0,1 µM de ljmiPEP171b (Pep 171, barre de droite).
   La barre d'erreur correspond à l'erreur standard à la moyenne (nombre d'individus = 5 plantes contrôles, 5 plantes traitées).
**FIGURE 15****. Effets du osmiPEP171i sur le nombre d'arbuscules chez *Oryza sativa***
   L'axe des ordonnées indique le nombre d'arbuscules chez une plante traitée 5 semaines post inoculation par un solvant (barre de gauche) ou par un solvant contenant 0,1 µM de ljmiPEP171b (Pep 171, barre de droite).
   La barre d'erreur correspond à l'erreur standard à la moyenne (nombre d'individus = 5 plantes contrôles, 5 plantes traitées).

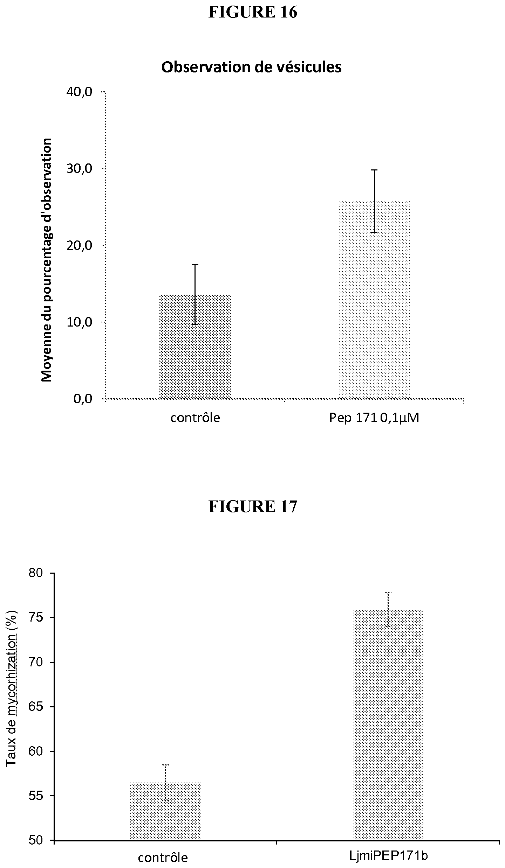

**FIGURE 16****. Effets du osmiPEP171i sur le nombre de vésicules chez *Oryza sativa***
   L'axe des ordonnées indique le nombre de vésicules chez une plante traitée 5 semaines post inoculation par un solvant (barre de gauche) ou par un solvant contenant 0,1 µM de OsmiPEP171i (Pep 171, barre de droite).
   La barre d'erreur correspond à l'erreur standard à la moyenne (nombre d'individus = 5 plantes contrôles, 5 plantes traitées).
**FIGURE 17****. Effets du slmiPEP171e sur le taux de de mycorhization de *Solanum lycopersicum***
   L'axe des ordonnées indique le taux de mycorhization des plantes de *Solanum lycopersicum* traitées 12 semaines post inoculation par un solvant (barre de gauche) ou par un solvant contenant 0,1 µM de slmiPEP171e (barre de droite).
   La barre d'erreur correspond à l'erreur standard à la moyenne (nombre d'individus = 12).

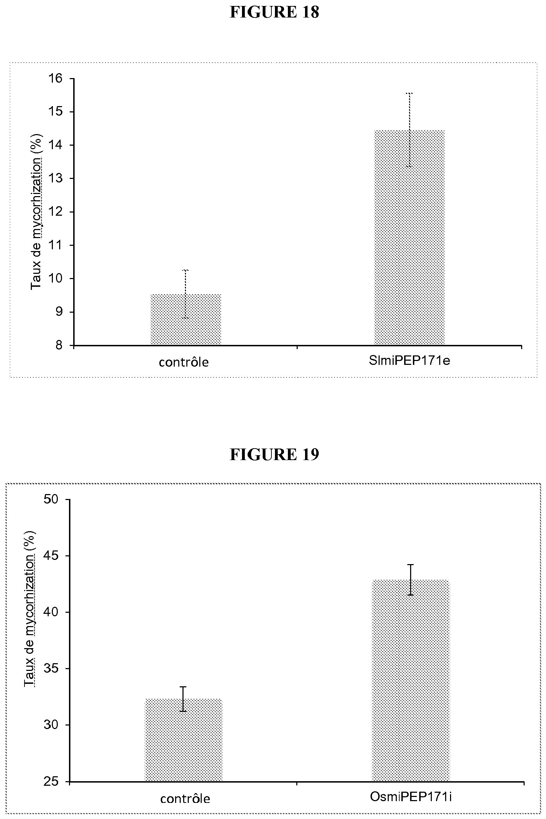

**FIGURE 18****. Effets du ljPEP171b sur le taux de de mycorhization de *Lotus japonicus***
   L'axe des ordonnées indique le taux de mycorhization des plantes de *Lotus japonicus* traitées 5 semaines post inoculation par un solvant (barre de gauche) ou par un solvant contenant 0,1 µM de ljmiPEP171b (barre de droite).
   La barre d'erreur correspond à l'erreur standard à la moyenne (nombre d'individus = 12).
**FIGURE 19****. Effets du OsPEP171i sur le taux de de mycorhization d'*Oriza sativa***
   L'axe des ordonnées indique le taux de mycorhization des plantes d'*Oryza sativa* traitées 5 semaines post inoculation par un solvant (barre de gauche) ou par un solvant contenant 0,1 µM de OsmiPEP171i (barre de droite).
   La barre d'erreur correspond à l'erreur standard à la moyenne (nombre d'individus = 12).

### EXEMPLES

### Exemple 1 - Identification et caractérisation du miPEP171b

Le miR171b est exprimé dans la région méristématique des racines, ainsi que dans les sites d'initiation des racines latérales. La surexpression de ce miR conduit notamment à une réduction de l'expression des gènes *HAM1* (Accession n°. MtGI9- TC114268) et *HAM2* (Accession n°. MtGI9- TC120850) (Figure 1A), ainsi qu'à une réduction du nombre de racines latérales (Figure 1B).

La séquence du transcrit primaire du miR171b a été déterminée en utilisant la technique de RACE-PCR. L'analyse de la séquence du transcrit primaire a permis d'identifier la présence de plusieurs petits cadres ouverts de lecture (ORF) complètement inattendus.

La surexpression du premier ORF, nommé miORF171b, conduit à une augmentation de l'accumulation de miR171b et une réduction de l'expression des gènes *HAM1* et *HAM2* (voir Figure 2A), ainsi qu'à une réduction du nombre de racines latérales (Figure 2B), comme cela a déjà été observé lors de la surexpression du miR171b.

Pour déterminer si le miORF171b conduit à la production réelle d'un peptide une immunolocalisation du peptide avec un anticorps spécifique a été effectuée et a révélé la présence du peptide dans les sites d'initiation des racines latérales, montrant ainsi une co-localisation entre le microARN et le miPEP correspondant. Pour déterminer si la fonction de régulation observée ci-dessus est bien portée par ledit peptide, un peptide synthétique, dont la séquence est identique à celle potentiellement codée par miORF171b, a été appliqué sur les racines de *Medicago truncatula.* L'application de ce peptide entraîne le phénotype déjà observé plus haut lors de la surexpression du miORF171b, c'est-à-dire qu'il entraîne une augmentation de l'accumulation de miR171b et une réduction de l'expression des gènes *HAM1* et *HAM2* (voir Figure 3A), ainsi qu'une réduction du nombre de racines latérales (Figure 3B). Les résultats de ces expériences démontrent que le miORF171b code un peptide capable de moduler l'accumulation du miR171b, et l'expression des gènes cibles de miR171b: *HAM1* et *HAM2.* Ledit peptide a été dénommé miPEP171b.

Par ailleurs, l'immunolocalisation du miPEP171b1 dans les racines de *M*. *truncatula* révèle la présence du miPEP171b1 dans les sites d'initiation des racines latérales, montrant une co-localisation entre le microARN et le miPEP correspondant.

### Exemple 2 - Effets du miPEP171b sur la mycorhization chez M. truncatula

Des plantes *Medicago truncatula* ont été traitées pendant 5 semaines, par arrosage tous les 2 jours, avec de faibles concentrations (0.1 µM) d'un peptide synthétique dont la séquence est identique à celle du miPEP171b et le pourcentage de colonisation de la plante a été mesuré.

Les résultats de ces expériences indiquent que le traitement avec le miPEP171b augmente significativement la mycorhization chez *M. truncatula* (Figure 5).

Par ailleurs, il a été observé que les arbuscules obtenus dans les racines traitées par le miPEP171b ont une taille plus importante que ceux présents dans les racines contrôle non traitées avec miPEP171b (Figure 6).

Il a également été observé que le taux de mycorhization est plus important chez les plantes traitées avec le miPEP171b par rapport aux plantes contrôle non traitées avec le miPEP171b (Figure 7).

### Exemple 3 - Effets du miPEP171b sur la mycorhization chez Medicago sativa

L'homologue du miPEP171b de *M. truncatula* a été identifié chez *M. sativa* par blast et RACE PCR.

Un traitement par différentes doses de miPEP171b (0.01 µM à 10 µM) est effectué en parallèle de la mycorhization de *M. sativa.*

### Exemple 4 - Effets du miPEP171b sur la mycorhization chez Glycine max (soja)

L'homologue du miPEP171b de *M. truncatula* a été identifié chez le soja par blast et RACE PCR.

Un traitement par différentes doses de miPEP171b (0.01 µM à 10 µM) est effectué en parallèle de la mycorhization du soja.

### Exemple 5 - Effets du slmiPEP171e sur la mycorhization chez Solanumlycopersicum (tomate)

L'homologue du miPEP171e de *M. truncatula* a été identifié chez la tomate par blast et RACE PCR.

Les effets du slmiPEP171e sur la mycorhization de la tomate ont été analysés en traitant les plantes avec slmiPEP171e en parallèle de la mycorhization.

Les résultats sont présentés dans les figures 8 et 17.

Ces résultats indiquent que le slmiPEP171e favorise la mycorhization de la tomate.

### Exemple 6 - Effets du LjmiPEP171b sur la mycorhization chez Lotus japonicus (lotier)

L'homologue du miPEP171b de *M. truncatula* a été identifié chez le lotier par blast et RACE PCR.

Les effets du LjmiPEP171b sur la mycorhization du lotier ont été analysés en traitant les plantes avec LjmiPEP171b en parallèle de la mycorhization.

Les résultats sont présentés dans les figures 9, 10, 11, 12 et 18.

Ces résultats indiquent que le LjmiPEP171b favorise la mycorhization du lotier.

### Exemple 7 - Effets du osmiPEP171i sur la mycorhization chez Oryza sativa (riz)

L'homologue du miPEP171i de *M. truncatula* a été identifié chez le riz par blast et RACE PCR.

Les effets du osmiPEP171i sur la mycorhization du soja ont été analysés en traitant les plantes avec osmiPEP171i en parallèle de la mycorhization.

Les résultats sont présentés dans les figures 13, 14, 15, 16 et 19. Ces résultats indiquent que le osmiPEP171i favorise la mycorhization du lotier.

### MATERIEL ET METHODES

### Medicago truncatula

### Matériel biologique

La surface des graines de *M. truncatula* a été stérilisée et celles-ci ont été mises à germer sur des plaques d'agar pendant 5 jours à 4°C dans l'obscurité. Les jeunes pousses ont ensuite été cultivées sur des plaques carrées de 12 cm remplies de milieu Fahraeus sans nitrogène et contenant du phosphate 7,5 µM (Lauresergues et al., Plant J., 72(3):512-22, 2012). Les racines latérales ont été dénombrées chaque jour. En pots, les plantes ont été arrosées tous les deux jours avec du milieu Long Ashton modifié contenant peu de phosphore (Balzergue et al., 2011 Journal of experimental botany, 62 :1049-1060.*)*.

Les peptides ont été synthétisés par Eurogentec ou Smartox-Biotech. Le miPEP171b a été remis en suspension dans une solution d'eau 40%/ acétonitrile 50%/ acide acétique 10% (v/v/v).

### Transcription inverse des microRNAs

L'ARN a été extrait en utilisant le réactif Tri-Reagent (MRC) selon les instructions du fabricant, à l'exception de la précipitation de l'ARN qui a été réalisée avec 3 volumes d'éthanol. La transcription inverse de l'ARN a été réalisée en utilisant l'amorce spécifique RTprimerl71b tige boucle en combinaison avec des hexamères pour effectuer la transcription inverse de l'ARN de haut poids moléculaire.

En bref, 1 µg de RNA a été ajouté à l'amorce tige boucle MIR171b (0,2 µM), l'héxamère (500 ng), le tampon RT (1X), l'enzyme SuperScript Reverse transcriptase (SSIII) (une unité), les dNTPs (0,2 mM chacun), le DTT (0,8 mM) dans un mélange réactionnel final de 25 µl. Pour effectuer la transcription inverse, une réaction de transcription inverse pulsée a été réalisée (40 répétitions du cycle suivant : 16°C pendant 2 minutes, 42°C pendant une minute et 50°C pendant une seconde, suivies d'une inactivation finale de la transcription inverse à 85°C pendant 5 minutes).

### Analyses par RT-PCR quantitative (qRT-PCR)

L'ARN total des racines de *M. truncatula* a été extrait en utilisant le kit d'extraction RNeasy Plant Mini Kit (Qiagen). La transcription inverse a été réalisée en utilisant la transcriptase inverse SuperScript II (Invitrogen) à partir de 500 ng d'ARN total. Trois répétitions (n=3) ont été réalisées avec deux répétitions techniques chacune. Chaque expérience a été répétée de deux à trois fois. Les amplifications par qPCR ont été réalisées en utilisant un thermocycleur LightCycler 480 System (Roche Diagnostics) selon la méthode décrite dans Lauressergues et al. (Plant J., 72(3):512-22, 2012).

### Analyses statistiques

Les valeurs moyennes de l'expression relative des gènes ou de la production de racines latérales ont été analysées en utilisant le test de Student ou le test de Kruskal-Wallis. Les barres d'erreurs représentent l'écart type de la moyenne (SEM, Standard Error of the Mean). Les astérisques indiquent une différence significative (p<0,05).

### Constructions plasmidiques

Les fragments d'ADN d'intérêt ont été amplifiés avec la Pfu polymerase (Promega). Les fragments d'ADN ont été clonés à l'aide des enzymes XhoI et NotI dans un plasmide pPEX-DsRED pour une surexpression sous le contrôle du promoteur fort constitutif 35S, et à l'aide des enzymes KpnI-NcoI dans un plasmide pPEX GUS pour les gènes rapporteurs, selon la méthode décrite dans Combier et al. (Genes & Dev, 22: 1549-1559, 2008).

### Transformation des plantes

Les plantes composites possédant des racines transformées avec *Agrobacterium Rhizogenes* ont été obtenues par la méthode décrite dans Boisson-Dernier et al. (Mol Plant-Microbe Interact, 18:1269-1276, 2005). Les racines transformées ont été vérifiées et sélectionnées par observations du DsRED avec une loupe binoculaire à fluorescence. Les racines témoins correspondent à des racines transformées avec *A. rhizogenes* ne contenant pas le vecteur pPEX-DsRED.

### Mycorhization

Des spores stériles de *Rhizophagus irregularis* (anciennement dénommé *Glomus intraradices*) DAOM197198 ont été achetées auprès d'Agronutrition (Carbonne, France). Des graines de *M. truncatula* (Gaertn 'Jemalong' génotype A17) ont été stérilisées en surface et mises à germer sur des plaques d'agar dans le noir pendant 5 jours à 4°C. Les plantes ont ensuite été cultivées sur un substrat Oil-Dri US-special (Damolin, Danemark) pendant 5 à 12 semaines dans une chambre de culture et arrosées tous les 2 jours avec du milieu Long Ashton modifié contenant une faible concentration de phosphate (Balzergue et al., J Exp Bot, 62(3):1049-60, 2011).

Pour l'inoculation des plantes avec *R. irregularis*, 450 spores ont été utilisées par plante. Après lavage dans du KOH 10% (poids/volume) et rinçage dans de l'eau stérile, les racines mycorhizées ont été traitées pendant 30 minutes avec de l'agglutinine de germe de blé conjuguée à la fluorescéine (Invitrogen) qui fixe la chitine fongique, puis lavées 3 fois dans un tampon de PBS. Les racines ont ensuite été observées avec un microscopique optique inversé ou un microscope confocal (Leica, France).

De manière alternative, les racines ont été marquées avec de l'encre noire Schaeffer selon le protocole décrit dans Vierheilig et al., Appl Environ Microbiol, 64:5004-5007, 1998.

Le pourcentage de mycorhization a été mesuré par grilles selon le protocole décrit dans Giovannetti and Mosse, New Phytol, 84:489-500, 1980.

Le phénotypage précis de la mycorhization a aussi été réalisée selon la méthode de Trouvelot et al., Physiological and Genetical Aspects ofMycorrhizae, pp 217-221, 1986. La fréquence de mycorhization (F) dans le système racinaire et l'abondance des arbuscules (A) (en pourcentage) ont été calculées dans des sections de racines colonisées en utilisant le logiciel Mycocalc.

La taille des arbuscules a été mesurée en utilisant le logiciel ImageJ.

Chaque expérience de mycorhization a été réalisée au moins 2 fois en utilisant 12 plantes pour chaque condition, chacune correspondant à une transformation indépendante avec *A*. *rhizogenes.*

### Immunolocalisation

Des racines ou des plantules de tissus de Medicago ont été fixés pendant 2 heures dans 4% de formol (v / v) à 50 mM de tampon phosphate (pH 7,2), puis inclus dans de l'agarose LMP 5% dans de l'eau (à bas point de fusion). Des coupes fines (100 um) ont été obtenues et placées dans du Pbi (tampon phosphate pour l'immunologie) sur des lames coatées avec du téflon, bloquées dans Pbi, 2% Tween et 1% d'albumine de sérum bovin pendant 2 heures (PbiT-BSA), puis marquées épendant une nuit (12 h) à 4 ° C avec l'anticorps primaire dilué dans du BSA-PbiT. Les coupes ont été lavées avec du PBiT et incubées à température ambiante pendant 2 h avec un anticorps secondaire dilué dans PbiT-BSA. Les lames ont ensuite été lavées dans du Pbi pendant 30 min et montées dans du citifluor (milieu de montage). Les anticorps primaires et les dilutions ont été les suivants: 1716a (1:500, v/v). L'anticorps secondaire était un anticorps de chèvre anti-IgG de lapin couplé à la sonde fluorescente Alexa Fluor 633 (Molecular Probes), et a été utilisé à une dilution de 1:1000 (v/v).

### S. lycopersicum

### • Germination des graines de S. lycopersicum

Les graines sont stérilisées 2 minutes dans l'eau de javel dilué au ¼, puis rinçées au moins 8 fois avec de l'eau stérile.

Les graines sont déposées sur une boite de Pétri contenant de l'eau + agar 1%. Les boites sont placées à 4°C pendant une nuit, puis elles sont placées à température ambiante pendant 4 jours pour faire germer les graines.

### • Inoculation des plantules germées :

Les graines germées sont transférées dans du substrat oil-dry^{®} contenant des spores de *Rhizophagus irregularis* à la concentration de 400spores/L. Après deux jours d'adaptation sous mini serre les plantules sont transférées en chambre de culture ou serre. Les plantes seront arrosées régulièrement avec une solution de Long Ashton + azote *(*Lauressergues et al., Plant J. 72(3):512-22, 2012).

### • Traitements des plantes avec les miPEP :

Le traitement s'effectue par arrosage des plantes avec 12,5 ml d'eau + la solution concentrée de miPEP ou l'équivalent de solution de solvant (acetonitrile 50%) pour le traitement contrôle. Les traitements sont réalisés tous les deux jours.

### • Protocole de coloration des racines de tomate :

Les racines sont décolorées au KOH 1% 7 minutes à 90°C, puis colorées 10 minutes dans une solution d'encre (5% d'encre Sheaffer ; 5% acide acétique) à 90°C.

### L. japonicus

### • Germination des graines de L. japonicus:

Les graines sont stérilisées à la javel pendant 20 min puis rincées plusieurs fois à l'eau.

### • Inoculation des plantules germées :

Mise en culture des lotiers dans pot contenant de la vermiculite avec du coton de fibre de verre au fond. 5000 spores de *Rhizophagus irregularis* sont ajoutées par pot. Les spores en mélanges dans un substrat sont disposées en couche sur la vermiculite à environ les deux tiers de la hauteur du pot. Le reste du pot est ensuite rempli de vermiculite et 10 graines de lotier pré-germées sont placées dans le substrat mouillé. 3 pots sont préparés pour chaque traitement. Les pots sont ensuite placés dans une chambre de culture à 25°C.

### • Traitements des plantes avec les miPEP :

▪ LjmiPEP 171e : 0,1 µM (final)
▪ Contrôle : eau.

Les traitements sont réalisés le lundi et vendredi en mélangeant la solution de miPEP ou contrôle avec de la solution nutritive (milieu B&D adapté) et le mercredi la solution de miPEP ou contrôle dans de d'eau. Chaque pot est arrosé avec 10ml sauf le vendredi avec 30ml (sans changer la quantité de miPEP), pour tenir le weekend.

### • Protocole de coloration des racines de Lotier:

Protocole adapté de Vierheilg et al. (1998) (Ink vinegar a simple staining technique for arbuscular-mycorrhizal fungi. Environ. Microbiol. 64 (12) : 5004-7)
▪ Couper les racines des plantes inoculées avec le champignon mycorhizien, laver et les placer dans un tube de 2ml.
▪ Couvrir les échantillons avec une solution de KOH 10% et décolorer les tissus pendant 15 min à 95°C dans un bloc chauffant. Porter des gants lorsqu'on manipule le KOH chaud.
▪ Vider le KOH et rincer les racines une fois avec de l'eau et une fois avec de l'acide acétique 10%. Attention à ne pas toucher les racines car elles sont très fragiles.
▪ Couvrir les racines avec une solution d'encre noire (pelikan) 5% dans une solution d'acide acétique 5%. Chauffer 5 minutes à 95°C.
▪ Retirer minutieusement la solution d'encre sans abimer les racines avec une pipette 200µL. Rincer deux fois les racines avec de l'eau.
▪ Décolorer les racines dans une solution acide acétique 5% pendant 20 minutes sous agitation.
▪ Stocker à 4°C jusqu'à utilisation.
▪ Monter les racines entre lame et lamelle dans l'eau ou dans du glycerol 50%.

### O. sativa

### • Germination des graines de O. sativa:

Les graines sont stérilisées 1min avec de l'éthanol 70%, puis 30 min avec de la javel avant rinçages.

### • Inoculation des plantules germées :

Mise en culture d'une graine de riz préalablement germée dans un pot contenant de la vermiculite avec du coton de fibre de verre au fond. 500 spores de *Rhizophagus irregularis* sont ajoutées par pot. Les spores en mélanges dans un substrat sont disposées en couche sur la vermiculite à environ les deux tiers de la hauteur du pot. Le reste du pot est ensuite remplie de vermiculite et un trou est réalisé pour y placer une graine de riz germée. Le substrat est mouillé. Les pots sont ensuite placés dans une chambre de culture à 25°C.

### • Traitements des plantes avec les miPEP :

▪ OsmiPEP171i : 0,1µM (final)
▪ Contrôle : équivalent de la solution ACN 50%.

Les traitements sont réalisés le lundi et vendredi en mélangeant la solution de miPEP ou contrôle dans 60 ml de solution nutritive (1/2 Hoagland avec 25 µM Pi et de Sequestren) et le mercredi la solution de miPEP ou contrôle dans 60ml d'eau. Chaque plante est arrosée avec 10ml par pot.

### • Protocole de coloration des racines de riz :

Les racines peuvent être stockées dans le 10% KOH plusieurs mois avant d'être colorées.
▪ Dans un tube de 2 ml incubation des racines pendant 30 minutes à 96°C dans le KOH (avec une faible agitation).
▪ Enlever le KOH
▪ Rincer trois fois avec de l'eau
▪ Incuber dans du HCl 0.3M pendant 15 min à 2h.
▪ Enlever le HCl
▪ Ajouter environ 1ml de 0.1% trypan blue et incubé les échantillons pendant 5 min à 96°C
▪ Enlever le trypan blue
▪ Laver les échantillons dans le glycerol acide 50% et monter les sections de racines entre lame et lamelle.

## Revendications

1. Utilisation d'un miPEP introduit par voie externe dans une plante pour favoriser la symbiose mycorhizienne, en particulier la symbiose mycorhizienne à arbuscules, entre ladite plante et un champignon, ledit miPEP étant également naturellement présent dans ladite plante,
ledit miPEP introduit par voie externe étant un peptide comprenant, ou consistant, en une séquence identique à celle dudit miPEP naturellement présent,
lequel miPEP naturellement présent est un peptide de 3 à 100 acides aminés dont la séquence est codée par un cadre ouvert de lecture situé en 5' sur le transcrit primaire d'un miR,
ledit miPEP étant capable de moduler l'accumulation dudit miR dans la dite plante, lequel miR régule l'expression d'au moins un gène impliqué dans la symbiose mycorhizienne à arbuscules chez ladite plante,
la somme de la quantité dudit miPEP introduit par voie externe et de celle dudit miPEP naturellement présent étant strictement supérieure à la quantité dudit miPEP naturellement présent,
ledit gène impliqué dans la symbiose mycorhizienne codant un facteur de transcription de la famille GRAS,
ledit gène impliqué dans la symbiose mycorhizienne étant choisi parmi le groupe consistant en : *HAM1* et *HAM2*
ledit miR étant le miR171b ou les miR ayant au moins 90% d'identité avec le miR171b ou ledit miR étant le slmiR171e ou les miR ayant au moins 90% d'identité avec le slmiR171e.

2. Utilisation selon la revendication 1, dans laquelle ledit miARN est :
- le miR171b, dans laquelle ledit miR171b possède une séquence nucléotidique consistant en SEQ ID NO : 1,
- le slmiR171e, dans laquelle ledit slmiR171e possède une séquence nucléotidique consistant en SEQ ID NO : 5,
- le ljmiR171b, en particulier dans laquelle ledit ljmiR171b possède une séquence nucléotidique consistant en SEQ ID NO : 9, ou
- le osmiR171i, en particulier dans laquelle ledit osmiR171i possède une séquence nucléotidique consistant en SEQ ID NO : 13.

3. Utilisation selon l'une quelconque des revendications précédentes, dans laquelle ledit miPEP est :
- le miPEP171b, en particulier, dans laquelle ledit miPEP171b possède une séquence d'acides aminés consistant en SEQ ID NO : 2,
- le slmiPEP171e, en particulier dans laquelle ledit slmiPEP171e possède une séquence d'acides aminés consistant en SEQ ID NO : 6,
- le ljmiPEP171b, en particulier dans laquelle ledit ljmiPEP171b possède une séquence d'acides aminés consistant en SEQ ID NO : 10, ou
- le osmiPEP171i, en particulier dans laquelle ledit osmiPEP171i possède une séquence d'acides aminés consistant en SEQ ID NO : 14.

4. Utilisation selon l'une quelconque des revendications précédentes, dans laquelle ladite plante est une plante Monocotylédone, une plante Dicotylédone, une plante solanacée ou une plante légumineuse, notamment choisie parmi : *Medicago truncatula, Medicago sativa* (luzerne), *Solanum lycopersicum* (tomate), *Lotus japonicus* (lotier) ou *Oryza sativa* (riz).

5. Utilisation selon l'une quelconque des revendications précédentes, dans laquelle ledit champignon est un Gloméromycète ou Basidiomycète ou Ascomycète, de préférence un Gloméromycète.

6. Utilisation selon l'une quelconque des revendications 1 à 5 pour favoriser la symbiose mycorhizienne à arbuscules :
entre une plante *Medicago truncatula* et un champignon gloméromycète, dans laquelle le miPEP171b est introduit par voie externe dans ladite plante *M. truncatula,* ledit miPEP 171b étant également naturellement présent dans ladite plante *M. truncatula,* ledit miPEP171b introduit par voie externe étant un peptide dont la séquence comprend ou consiste en une séquence identique à celle dudit miPEP171b naturellement présent, ladite séquence du miPEP171b naturellement présent étant codée par un cadre ouvert de lecture situé en 5' sur le transcrit primaire du miR171b, lequel miR171b régule l'expression d'au moins un gène impliqué dans la symbiose mycorhizienne chez *M. truncatula,*
la somme de la quantité dudit miPEP171b introduit par voie externe et de celle dudit miPEP171b naturellement présent étant strictement supérieure à la quantité dudit miPEP 171b naturellement présent chez ladite plante *Medicago truncatula,*
ou
entre une plante *Solanum lycopersicum* et un champignon gloméromycète, dans laquelle le slmiPEP171e est introduit par voie externe dans ladite plante *Solanum lycopersicum*, ledit slmiPEP171e étant également naturellement présent dans ladite plante *Solanum lycopersicum,*
ledit slmiPEP171e introduit par voie externe étant un peptide dont la séquence comprend ou consiste en une séquence identique à celle dudit slmiPEP171e naturellement présent, ladite séquence du slmiPEP171e naturellement présent étant codée par un cadre ouvert de lecture situé en 5' sur le transcrit primaire du slmiR171e, lequel slmiR171e régule l'expression d'au moins un gène impliqué dans la symbiose mycorhizienne chez *Solanum lycopersicum,*
la somme de la quantité dudit slmiPEP171e introduit par voie externe et de celle dudit slmiPEP171e naturellement présent étant strictement supérieure à la quantité dudit slmiPEP171e naturellement présent chez ladite plante *Solanum lycopersicum,*
ou
entre une plante *Lotus japonicus* et un champignon gloméromycète, dans laquelle le ljmiPEP171b est introduit par voie externe dans ladite plante *Lotus japonicus,* ledit ljmiPEP171b étant également naturellement présent dans ladite plante *Lotus japonicus,* ledit ljmiPEP171b introduit par voie externe étant un peptide dont la séquence comprend ou consiste en une séquence identique à celle dudit ljmiPEP171b naturellement présent, ladite séquence du ljmiPEP171b naturellement présent étant codée par un cadre ouvert de lecture situé en 5' sur le transcrit primaire du ljmiR171b, lequel ljmiR171b régule l'expression d'au moins un gène impliqué dans la symbiose mycorhizienne chez *Lotus japonicus*,
la somme de la quantité dudit ljmiPEP171b introduit par voie externe et de celle dudit ljmiPEP171b naturellement présent étant strictement supérieure à la quantité dudit ljmiPEP171b naturellement présent chez ladite plante *Lotus japonicus,*
ou
entre une plante *Oryza sativa* et un champignon gloméromycète, dans laquelle le osmiPEP171i est introduit par voie externe dans ladite plante *Oryza sativa*, ledit osmiPEP171i étant également naturellement présent dans ladite plante *Oryza sativa*,ledit osmiPEP171i introduit par voie externe étant un peptide dont la séquence comprend ou consiste en une séquence identique à celle dudit osmiPEP171i naturellement présent, ladite séquence du osmiPEP171i naturellement présent étant codée par un cadre ouvert de lecture situé en 5' sur le transcrit primaire du osmiR171i, lequel miR171i régule l'expression d'au moins un gène impliqué dans la symbiose mycorhizienne chez *Oryza sativa*, la somme de la quantité dudit osmiPEP171i introduit par voie externe et de celle dudit osmiPEP171i naturellement présent étant strictement supérieure à la quantité dudit osmiPEP171i naturellement présent chez ladite plante *Oryza sativa*
ledit gène impliqué dans la symbiose mycorhizienne codant un facteur de transcription de la famille GRAS,
ledit gène impliqué dans la symbiose mycorhizienne étant choisi parmi le groupe consistant en : *HAM1* et *HAM2.*

7. Procédé pour favoriser la symbiose mycorhizienne entre une plante et un champignon, et en particulier la symbiose mycorhizienne à arbuscules (AM), comprenant une étape d'introduction d'un miPEP dans une plante par voie externe, ledit miPEP étant également présent naturellement dans ladite plante,
ledit miPEP introduit par voie externe étant un peptide de 3 à 100 acides aminés dont la séquence comprend ou consiste en une séquence identique à celle dudit miPEP naturellement présent, laquelle séquence du miPEP naturellement présent est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la symbiose mycorhizienne chez ladite plante,
la somme de la quantité dudit miPEP introduit par voie externe et de celle dudit miPEP naturellement présent étant strictement supérieure à la quantité dudit miPEP naturellement présent,
ledit gène impliqué dans la symbiose mycorhizienne codant un facteur de transcription de la famille GRAS,
ledit gène impliqué dans la symbiose mycorhizienne étant choisi parmi le groupe consistant en : *HAM1* et *HAM2,*
ledit miR étant le miR171b ou les miR ayant au moins 90% d'identité avec le miR171b ou ledit miR étant le slmiR171e ou les miR ayant au moins 90% d'identité avec le slmiR171e.

8. Procédé selon la revendication 7, dans lequel ledit miPEP est introduit dans la plante :
- par voie externe, de préférence par arrosage, par pulvérisation ou par l'ajout d'un engrais, ledit miPEP étant notamment administré à la plante sous la forme d'une composition comprenant 10⁻⁹ M à 10⁻⁴ M dudit miPEP, en particulier 10⁻⁹, 10⁻⁸, 10⁻⁷, 10⁻⁶, 10⁻⁵ ou 10⁻⁴ M dudit miPEP,
- par voie externe, de préférence par arrosage, par pulvérisation ou par l'ajout d'un engrais, ledit miPEP étant notamment administré à une graine ou à une semence sous la forme d'une composition comprenant 10⁻⁹ M à 10⁻⁴ M dudit miPEP, en particulier 10⁻⁹, 10⁻⁸, 10⁻⁷, 10⁻⁶, 10⁻⁵ ou 10⁻⁴ M dudit miPEP, ou
- par le biais d'un acide nucléique codant ledit miPEP, ledit acide nucléique étant introduit dans la plante.

9. Procédé de production d'une plante transgénique comprenant :
**a)** une étape d'introduction d'un acide nucléique codant un miPEP de 3 à 100 acides aminés dans une plante, mais ledit acide nucléique ne comprenant pas la séquence complète du miR correspondant ou dans au moins une cellule de ladite plante, dans des conditions permettant l'expression dudit miPEP,
ledit miPEP étant également naturellement présent dans ladite plante, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR dans la plante, lequel miR régule l'expression d'au moins un gène impliqué dans la symbiose mycorhizienne, en particulier la symbiose mycorhizienne à arbuscules, et
**b)** une étape de culture de la plante, ou d'au moins une cellule de ladite plante, obtenues à l'étape a) dans des conditions permettant l'obtention d'une plante transgénique,
ledit gène impliqué dans la symbiose mycorhizienne étant choisi parmi le groupe consistant en : *HAM1* et *HAM2*,
ledit miR étant le miR171b ou les miR ayant au moins 90% d'identité avec le miR171b ou ledit miR étant le slmiR171e ou les miR ayant au moins 90% d'identité avec le slmiR171e.

10. Procédé de production d'une plante transgénique tel que décrit dans la revendication 9 dans lequel,
- ledit miPEP étant le miPEP171b, ledit miPEP171b possédant en particulier une séquence d'acides aminés consistant en SEQ ID NO : 2, ou
- ledit miPEP étant le slmiPEP171e, ledit slmiPEP171e possédant en particulier une séquence d'acides aminés consistant en SEQ ID NO : 6, ou
- ledit miPEP étant le ljmiPEP171b, ledit ljmiPEP171b possédant en particulier une séquence d'acides aminés consistant en SEQ ID NO : 10, ou
- ledit miPEP étant le osmiPEP171i, ledit osmiPEP171i possédant en particulier une séquence d'acides aminés consistant en SEQ ID NO : 14.

11. Plante transgénique telle qu'obtenue par le procédé tel que défini selon la revendication 9 ou 10.

12. Composition comprenant :
- le miPEP171b en tant que substance active, ledit miPEP171b consistant de préférence en SEQ ID NO : 2, ledit miPEP171b étant à une concentration 10⁻⁹, 10⁻⁵ ou 10⁻⁴ M,
- le slmiPEP171e en tant que substance active, ledit slmiPEP171e consistant de préférence en SEQ ID NO : 6, ledit slmiPEP171e étant notamment à une concentration de 10⁻⁹ M à 10⁻⁴ M, en particulier 10⁻⁹, 10⁻⁸, 10⁻⁷, 10⁻⁶, 10⁻⁵ ou 10⁻⁴ M,
- le ljmiPEP171b en tant que substance active, ledit ljmiPEP171b consistant de préférence en SEQ ID NO : 10, ledit ljmiPEP171b étant notamment à une concentration de 10⁻⁹ M à 10⁻⁴ M, en particulier 10⁻⁹, 10⁻⁸, 10⁻⁷, 10⁻⁶, 10⁻⁵ ou 10⁻⁴ M, ou
- le osmiPEP171i en tant que substance active, ledit osmiPEP171i consistant de préférence en SEQ ID NO : 14, ledit osmiPEP171i étant notamment à une concentration de 10⁻⁹ M à 10⁻⁴ M, en particulier 10⁻⁹, 10⁻⁸, 10⁻⁷, 10⁻⁶, 10⁻⁵ ou 10⁻⁴ M,
- en particulier, ladite composition comprenant de plus un excipient, un diluant ou un solvant,
- en particulier, ladite composition étant formulée de façon à former un enrobé.

13. Composition comprenant en combinaison une quantité de semences d'une plante et une quantité d'un peptide dont la séquence comprend ou consiste en une séquence identique à celle d'un miPEP naturellement présent chez ladite plante,
ledit peptide ayant notamment une séquence comprenant ou consistant en une séquence identique à celle du miPEP171b, du slmiPEP171e, du LjmiPEP171b ou du osmiPEP171i,
en particulier, ladite composition étant formulée de façon à former une semence enrobée.

14. Procédé de culture de champignons mycorhiziens, en particulier des champignons mycorhiziens à arbuscules, comprenant une étape de mise en contact desdits champignons avec :
- un mélange comprenant une plante ou une partie de plante, en particulier une culture de racine, et un peptide dont la séquence comprend ou consiste en une séquence identique à celle d'un miPEP naturellement présent dans ladite plante, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la symbiose mycorhizienne chez ladite plante, ou
- avec une plante transgénique telle que définie précédemment,
la plante, la partie de plante, et la plante transgénique étant aptes à former une symbiose mycorhizienne avec ledit champignon,
ledit gène impliqué dans la symbiose mycorhizienne codant un facteur de transcription de la famille GRAS,
ledit gène impliqué dans la symbiose mycorhizienne étant choisi parmi le groupe consistant en : *HAM1* et *HAM2,*
ledit miR étant le miR171b ou les miR ayant au moins 90% d'identité avec le miR171b ou ledit miR étant le slmiR171e ou les miR ayant au moins 90% d'identité avec le slmiR171e.

15. Procédé pour produire de l'inoculum de champignons mycorhiziens, en particulier de l'inoculum de champignons mycorhiziens à arbuscules, comprenant :
- une étape de co-culture de champignons avec une matière végétale vivante, dite plante hôte, correspondant au moins en partie, à une partie constitutive de racine d'une plante apte à former une symbiose avec lesdits champignons, et
- une étape de mise en contact d'une quantité d'un peptide avec la susdite co-culture,
ledit peptide ayant une séquence comprenant ou consistant en une séquence identique à celle d'un miPEP naturellement présent dans ladite plante, ledit miPEP naturellement présent étant un peptide dont la séquence est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la symbiose mycorhizienne chez ladite plante
ledit gène impliqué dans la symbiose mycorhizienne codant un facteur de transcription de la famille GRAS,
ledit gène impliqué dans la symbiose mycorhizienne étant choisi parmi le groupe consistant en : *HAM1* et *HAM2,*
ledit miR étant le miR171b ou les miR ayant au moins 90% d'identité avec le miR171b ou ledit miR étant le slmiR171e ou les miR ayant au moins 90% d'identité avec le slmiR171e.

16. Inoculum de champignons mycorhiziens, en particulier un inoculum de champignons mycorhiziens à arbuscules, adapté à l'inoculation d'une plante hôte, comprenant au moins : un champignon et un peptide dont la séquence comprend ou consiste en une séquence identique à celle d'un miPEP naturellement présent dans la plante hôte, ledit miPEP naturellement présent dans la plante hôte étant un peptide dont la séquence est codée par un cadre ouvert de lecture situé en 5' sur le transcrit primaire d'un miR, ledit miPEP étant capable de moduler l'accumulation dudit miR, lequel miR régule l'expression d'au moins un gène impliqué dans la symbiose mycorhizienne chez ladite plante hôte
ledit gène impliqué dans la symbiose mycorhizienne codant un facteur de transcription de la famille GRAS,
ledit gène impliqué dans la symbiose mycorhizienne étant choisi parmi le groupe consistant en : *HAM1* et *HAM2,*
ledit miR étant le miR171b ou les miR ayant au moins 90% d'identité avec le miR171b ou ledit miR étant le slmiR171e ou les miR ayant au moins 90% d'identité avec le slmiR171e.

## Patentansprüche

1. Verwendung eines miPEP, das auf externem Weg in eine Pflanze eingeführt wird, um die Mykorrhiza-Symbiose, insbesondere die arbukuläre Mykorrhiza-Symbiose, zwischen der Pflanze und einem Pilz zu fördern, wobei das genannte miPEP auch natürlich in der genannten Pflanze vorkommt,
wobei das genannte miPEP, das auf externem Weg eingeführt wird, ein Peptid ist, umfassend eine, oder bestehend aus einer, Sequenz, die identisch ist mit jener des genannten natürlich vorkommenden miPEP,
wobei das genannte natürlich vorkommende miPEP ein Peptid mit 3 bis 100 Aminosäuren ist, dessen Sequenz von einem offenen Leserahmen codiert wird, der in 5' auf dem primären Transkript einer miR angeordnet ist,
wobei das genannte miPEP in der Lage ist, die Akkumulation der genannten miR in der genannten Pflanze zu modulieren,
wobei die genannte miR die Expression mindestens eines Gens reguliert, das in die arbukuläre Mykorrhiza-Symbiose bei der genannten Pflanze impliziert ist,
wobei die Summe der Menge des genannten miPEP, das auf externem Weg eingeführt wird, und jener des genannten natürlich vorkommenden miPEP streng größer ist als die Menge des genannten natürlich vorkommenden miPEP,
wobei das genannte Gen, das in die Mykorrhiza-Symbiose impliziert ist, einen Transkriptionsfaktor der Familie GRAS codiert,
wobei das genannte Gen, das in die Mykorrhiza-Symbiose impliziert ist, ausgewählt wird aus der Gruppe bestehend aus: *HAM1* und *HAM2,*
wobei die genannte miR die miR171b ist oder die miRs eine Identität von mindestens 90 % mit der miR171b aufweisen oder die genannte miR die slmiR171e ist oder die miRs eine Identität von mindestens 90 % mit der slmiR171e aufweisen.

2. Verwendung nach Anspruch 1, wobei die genannte miRNA ist:
- die miR171b, wobei die genannte miR171b eine Nucleotidsequenz bestehend aus der SEQ ID NR: 1 besitzt,
- die slmiR171e, wobei die genannte slmiR171e eine Nucleotidsequenz bestehend aus der SEQ ID NR: 5 besitzt,
- die ljmiR171b, wobei insbesondere die genannte ljmiR171b eine Nucleotidsequenz bestehend aus der SEQ ID NR: 9 besitzt, oder
- die osmiR171i, wobei insbesondere die genannte osmiR171i eine Nucleotidsequenz bestehend aus der SEQ ID NR: 13 besitzt.

3. Verwendung nach einem der vorhergehenden Ansprüche, wobei das genannte miPEP ist:
- das miPEP171b, wobei insbesondere das genannte miPEP171b eine Aminosäuresequenz bestehend aus der SEQ ID NR: 2 besitzt,
- das slmiPEP171e, wobei insbesondere das genannte slmiPEP171e eine Aminosäuresequenz bestehend aus der SEQ ID NR: 6 besitzt,
- das ljmiPEP171b, wobei insbesondere das genannte ljmiPEP171b eine Aminosäuresequenz bestehend aus der SEQ ID NR: 10 besitzt, oder
- das osmiPEP171i, wobei insbesondere das genannte osmiPEP171i eine Aminosäuresequenz bestehend aus der SEQ ID NR: 14 besitzt.

4. Verwendung nach einem der vorhergehenden Ansprüche, wobei die genannte Pflanze eine einkeimblättrige Pflanze, eine zweikeimblättrige Pflanze, ein Nachtschattengewächs oder eine Leguminosenpflanze ist, insbesondere ausgewählt aus: *Medicago truncatula, Medicago sativa* (Luzerne), *Solanum lycopersicum* (Tomate), *Lotus japonicus* (Hornklee) oder *Oryza sativa* (Reis) ist.

5. Verwendung nach einem der vorhergehenden Ansprüche, wobei der genannte Pilz ein Glomeromycetes oder Basidiomycetes oder Ascomycetes, vorzugsweise ein Glomeromycetes ist.

6. Verwendung nach einem der Ansprüche 1 bis 5, zur Förderung der arbuskulären Mykorrhiza-Symbiose:
zwischen einer *Medicago truncatula-Pflanze* und einem Glomeromycetes-Pilz, wobei das miPEP171b auf externem Weg in die genannte *M. truncatula-Pflanze* eingeführt wird, wobei das genannte miPEP171b auch natürlich in der genannten *M. truncatula-Pflanze* vorkommt,
wobei das genannte miPEP171b, das auf externem Weg eingeführt wird, ein Peptid ist, dessen Sequenz eine Sequenz umfasst oder daraus besteht, die identisch ist mit jener des genannten natürlich vorkommenden miPEP171b, wobei die genannte Sequenz des natürlich vorkommenden miPEP171b von einem offenen Leserahmen codiert wird, der in 5' auf dem primären Transkript der miR171b angeordnet ist,
wobei die genannte miR171b die Expression mindestens eines Gens reguliert, das in die Mykorrhiza-Symbiose bei *M. truncatula* impliziert ist,
wobei die Summe der Menge des genannten miPEP171b, das auf externem Weg eingeführt wird, und jener des genannten natürlich vorkommenden miPEP171b streng größer ist als die Menge des genannten miPEP171b, das natürlich in der genannten *Medicago truncatula-Pflanze* vorkommt,
oder
zwischen einer *Solanum lycopersicum-Pflanze* und einem Glomeromycetes-Pilz, wobei das slmiPEP171e auf externem Weg in die genannte *Solanum lycopersicum-Pflanze* eingeführt wird, wobei das genannte slmiPEP171e auch natürlich in der genannten *Solanum lycopersicum-Pflanze* vorkommt,
wobei das genannte slmiPEP171e, das auf externem Weg eingeführt wird, ein Peptid ist, dessen Sequenz eine Sequenz umfasst oder daraus besteht, die identisch ist mit jener des genannten natürlich vorkommenden slmiPEP171e, wobei die genannte Sequenz des natürlich vorkommenden slmiPEP171e von einem offenen Leserahmen codiert wird, der in 5' auf dem primären Transkript der slmiR171e angeordnet ist,
wobei die genannte slmiR171e die Expression mindestens eines Gens reguliert, das in die Mykorrhiza-Symbiose bei *Solanum lycopersicum* impliziert ist,
wobei die Summe der Menge des genannten slmiPEP171e, das auf externem Weg eingeführt wird, und jener des genannten natürlich vorkommenden slmiPEP171b streng größer ist als die Menge des genannten slmiPEP171b, das natürlich in der genannten *Solanum lycopersicum-Pflanze* vorkommt,
oder
zwischen einer *Lotus japonicus*-Pflanze und einem Glomeromycetes-Pilz, wobei das ljmiPEP171b auf externem Weg in die genannte *Lotus japonicus-Pflanze* eingeführt wird, wobei das genannte ljmiPEP171b auch natürlich in der genannten *Lotus japonicus-Pflanze* vorkommt,
wobei das genannte ljmiPEP171b, das auf externem Weg eingeführt wird, ein Peptid ist, dessen Sequenz eine Sequenz umfasst oder daraus besteht, die identisch ist mit jener des genannten natürlich vorkommenden ljmiPEP171b, wobei die genannte Sequenz des natürlich vorkommenden ljmiPEP171b von einem offenen Leserahmen codiert wird, der in 5' auf dem primären Transkript der ljmiR171b angeordnet ist,
wobei die genannte ljmiR171b die Expression mindestens eines Gens reguliert, das in die Mykorrhiza-Symbiose bei *Lotus japonicus* impliziert ist,
wobei die Summe der Menge des genannten ljmiPEP171b, das auf externem Weg eingeführt wird, und jener des genannten natürlich vorkommenden ljmiPEP171b streng größer ist als die Menge des genannten ljmiPEP171b, das natürlich in der genannten *Lotus japonicus-Pflanze* vorkommt,
oder
zwischen einer *Oryza* sativa-Pflanze und einem Glomeromycetes-Pilz, wobei das osmiPEP171i auf externem Weg in die genannte *Oryza* sativa-Pflanze eingeführt wird, wobei das genannte osmiPEP171i auch natürlich in der genannten *Oryza* sativa-Pflanze vorkommt, wobei das genannte osmiPEP171i, das auf externem Weg eingeführt wird, ein Peptid ist, dessen Sequenz eine Sequenz umfasst oder daraus besteht, die identisch ist mit jener des genannten natürlich vorkommenden osmiPEP171i, wobei die genannte Sequenz des natürlich vorkommenden osmiPEP171i von einem offenen Leserahmen codiert wird, der in 5' auf dem primären Transkript der osmiR171i angeordnet ist,
wobei die genannte miR171i die Expression mindestens eines Gens reguliert, das in die Mykorrhiza-Symbiose bei *Oryza sativa* impliziert ist, wobei die Summe der Menge des genannten osmiPEP171i, das auf externem Weg eingeführt wird, und jener des genannten natürlich vorkommenden osmiPEP171i streng größer ist als die Menge des genannten osmiPEP171i, das natürlich in der genannten *Lotus japonicus-Pflanze* vorkommt,
wobei das genannte Gen, das in die Mykorrhiza-Symbiose impliziert ist, einen Transkriptionsfaktor der Familie GRAS codiert,
wobei das genannte Gen, das in die Mykorrhiza-Symbiose impliziert ist, ausgewählt wird aus der Gruppe bestehend aus: *HAM1* und *HAM2.*

7. Verfahren zur Förderung der Mykorrhiza-Symbiose zwischen einer Pflanze und einem Pilz, und insbesondere der arbuskulären Mykorrhiza-Symbiose (AM), umfassend einen Schritt des Einführens eines miPEP in eine Pflanze auf externem Weg, wobei das genannte miPEP auch natürlich in der genannten Pflanze vorkommt,
wobei das genannte miPEP, das auf externem Weg eingeführt wird, ein Peptid mit 3 bis 100 Aminosäuren ist, dessen Sequenz eine Sequenz umfasst oder daraus besteht, die identisch ist mit jener des genannten natürlich vorkommenden miPEP, wobei die Sequenz des natürlich vorkommenden miPEP von einem offenen Leserahmen codiert wird, der in 5' auf dem primären Transkript einer miR angeordnet ist, wobei das genannte miPEP in der Lage ist, die Akkumulation der genannten miR zu modulieren, wobei die miR die Expression mindestens eines Gens reguliert, das in die Mykorrhiza-Symbiose bei der genannten Pflanze impliziert ist,
wobei die Summe der Menge des genannten miPEP, das auf externem Weg eingeführt wird, und jener des genannten natürlich vorkommenden miPEP streng größer ist als die Menge des genannten natürlich vorkommenden miPEP,
wobei das genannte Gen, das in die Mykorrhiza-Symbiose impliziert ist, einen Transkriptionsfaktor der Familie GRAS codiert,
wobei das genannte Gen, das in die Mykorrhiza-Symbiose impliziert ist, ausgewählt wird aus der Gruppe bestehend aus: *HAM1* und *HAM2,*
wobei die genannte miR die miR171b ist oder die miR eine Identität von mindestens 90 % mit der miR171b aufweisen oder die genannte miR die slmiR171e ist oder die miR eine Identität von mindestens 90 % mit der slmiR171e aufweisen.

8. Verfahren nach Anspruch 7, wobei das genannte miPEP in die Pflanze eingeführt wird:
- auf externem Weg, vorzugsweise durch Berieselung, durch Zerstäubung oder durch Zusatz eines Düngemittels, wobei das genannte miPEP insbesondere der Pflanze in der Form einer Zusammensetzung verabreicht wird, die 10⁻⁹ M bis 10⁻⁴ M des genannten miPEP, insbesondere 10⁻⁹, 10⁻⁸, 10⁻⁷, 10⁻⁶, 10⁻⁵ oder 10⁻⁴ M des genannten miPEP, umfasst,
- auf externem Weg, vorzugsweise durch Berieselung, durch Zerstäubung oder durch Zusatz eines Düngemittels, wobei das genannte miPEP insbesondere einem Korn oder einem Samen in der Form einer Zusammensetzung verabreicht wird, die 10⁻⁹ M bis 10⁻⁴ M des genannten miPEP, insbesondere 10⁻⁹, 10⁻⁸, 10⁻⁷, 10⁻⁶, 10⁻⁵ oder 10⁻⁴ M des genannten miPEP, umfasst,
- mit Hilfe einer Nucleinsäure, die für das genannte miPEP codiert, wobei die genannte Nucleinsäure in die Pflanze eingeführt wird.

9. Verfahren zur Herstellung einer transgenen Pflanze, umfassend:
a) einen Schritt des Einführens einer Nucleinsäure, die ein miPEP mit 3 bis 100 Aminosäuren codiert, in eine Pflanze, wobei jedoch die genannte Nucleinsäure keine vollständige Sequenz der entsprechenden miR umfasst, oder in mindestens eine Zelle der genannten Pflanze, unter Bedingungen, welche die Expression des genannten miPEP gestatten,
wobei das genannte miPEP auch natürlich in der genannten Pflanze vorkommt, wobei das natürlich vorkommende miPEP ein Peptid ist, dessen Sequenz von einem offenen Leserahmen codiert wird, der in 5' auf dem primären Transkript einer miR angeordnet ist, wobei das genannte miPEP in der Lage ist, die Akkumulation der genannten miR in der Pflanze zu modulieren, wobei die miR die Expression mindestens eines Gens reguliert, das in die Mykorrhiza-Symbiose, insbesondere die arbuskuläre Mykorrhiza-Symbiose, impliziert ist, und
b) einen Schritt des Kultivierens der Pflanze, oder mindestens einer Zelle der genannten Pflanze, die in Schritt a) erhalten werden, unter Bedingungen, welche das Erhalten einer transgenen Pflanze gestatten,
wobei das genannte Gen, das in die Mykorrhiza-Symbiose impliziert ist, ausgewählt wird aus der Gruppe bestehend aus: *HAM1* und *HAM2,*
wobei die genannte miR die miR171b ist oder die miR eine Identität von mindestens 90 % mit der miR171b aufweisen oder die genannte miR die slmiR171e ist oder die miR eine Identität von mindestens 90 % mit der slmiR171e aufweisen.

10. Verfahren zur Herstellung einer transgenen Pflanze, wie in Anspruch 9 beschrieben, wobei
- das genannte miPEP das miPEP171b ist, wobei das genannte miPEP171b insbesondere eine Aminosäuresequenz bestehend aus der SEQ ID NR: 2 besitzt, oder
- das genannte miPEP das slmiPEP171e ist, wobei das genannte slmiPEP171e insbesondere eine Aminosäuresequenz bestehend aus der SEQ ID NR: 6 besitzt, oder
- das genannte miPEP das ljmiPEP171b ist, wobei das genannte ljmiPEP171b insbesondere eine Aminosäuresequenz bestehend aus der SEQ ID NR: 10 besitzt, oder
- das genannte miPEP das osmiPEP171i ist, wobei das genannte osmiPEP171i insbesondere eine Aminosäuresequenz bestehend aus der SEQ ID NR: 14 besitzt.

11. Transgene Pflanze, wie durch das Verfahren, wie nach Anspruch 9 oder 10 definiert, erhalten.

12. Zusammensetzung, umfassend:
- das miPEP171b als aktive Substanz, wobei das genannte miPEP171b vorzugsweise aus der SEQ ID NR: 2 besteht, wobei das genannte miPEP171b eine Konzentration von 10⁻⁹, 10⁻⁵ oder 10⁻⁴ M aufweist,
- das slmiPEP171e als aktive Substanz, wobei das genannte slmiPEP171e vorzugsweise aus der SEQ ID NR: 6 besteht, wobei das genannte slmiPEP171e insbesondere eine Konzentration von 10⁻⁹ bis 10⁻⁴ M, insbesondere 10⁻⁹, 10⁻⁸, 10⁻⁷, 10⁻⁶, 10⁻⁵ oder 10⁻⁴ M, aufweist,
- das ljmiPEP171b als aktive Substanz, wobei das genannte ljmiPEP171b vorzugsweise aus der SEQ ID NR: 10 besteht, wobei das genannte ljmiPEP171b insbesondere eine Konzentration von 10⁻⁹ bis 10⁻⁴ M, insbesondere 10⁻⁹, 10⁻⁸, 10⁻⁷, 10⁻⁶, 10⁻⁵ oder 10⁻⁴ M, aufweist,
- das osmiPEP171i als aktive Substanz, wobei das genannte osmiPEP171i vorzugsweise aus der SEQ ID NR: 14 besteht, wobei das genannte osmiPEP171i insbesondere eine Konzentration von 10⁻⁹ bis 10⁻⁴ M, insbesondere 10⁻⁹, 10⁻⁸, 10⁻⁷, 10⁻⁶, 10⁻⁵ oder 10⁻⁴ M, aufweist,
- wobei insbesondere die genannte Zusammensetzung außerdem einen Exzipienten, ein Verdünnungsmittel oder ein Lösungsmittel umfasst,
- wobei insbesondere die genannte Zusammensetzung formuliert ist, um eine Beschichtung zu bilden.

13. Zusammensetzung, umfassend eine Kombination einer Menge von Samen einer Pflanze und einer Menge eines Peptids, dessen Sequenz eine Sequenz umfasst oder daraus besteht, die identisch ist mit jener eines miPEP, das natürlich in der genannten Pflanze vorkommt,
wobei das genannte Peptid insbesondere eine Sequenz aufweist, die eine Sequenz umfasst oder daraus besteht, welche identisch ist mit jener des miPEP171b, des slmiPEP171e, des LjmiPEP171b oder des osmiPEP171i,
wobei insbesondere die genannte Zusammensetzung formuliert ist, um einen beschichteten Samen zu bilden.

14. Verfahren zum Kultivieren von Mykorrhiza-Pilzen, insbesondere arbuskulären Mykorrhiza-Pilzen, umfassend einen Schritt des Inberührungbringens der genannten Pilze mit:
- einer Mischung, umfassend eine Pflanze oder einen Teil einer Pflanze, insbesondere eine Wurzelkultur, und ein Peptid, dessen Sequenz eine Sequenz umfasst oder daraus besteht, welche identisch ist mit jener eines miPEP, das natürlich in der genannten Pflanze vorkommt, wobei das genannte natürlich vorkommende miPEP ein Peptid ist, dessen Sequenz von einem offenen Leserahmen codiert wird, der in 5' auf dem primären Transkript einer miR angeordnet ist, wobei das genannte miPEP in der Lage ist, die Akkumulation der genannten miR in der Pflanze zu modulieren, wobei die miR die Expression mindestens eines Gens reguliert, das in die Mykorrhiza-Symbiose bei der genannten Pflanze impliziert ist, oder
- mit einer transgenen Pflanze, wie im Vorstehenden definiert,
wobei die Pflanze, der Teil der Pflanze und die transgene Pflanze dafür geeignet sind, um eine Mykorrhiza-Symbiose mit dem genannten Pilz zu bilden,
wobei das genannte Gen, das in die Mykorrhiza-Symbiose impliziert ist, einen Transkriptionsfaktor der Familie GRAS codiert,
wobei das genannte Gen, das in die Mykorrhiza-Symbiose impliziert ist, ausgewählt wird aus der Gruppe bestehend aus: *HAM1* und *HAM2,*
wobei die genannte miR die miR171b ist oder die miR eine Identität von mindestens 90 % mit der miR171b aufweisen oder die genannte miR die slmiR171e ist oder die miR eine Identität von mindestens 90 % mit der slmiR171e aufweisen.

15. Verfahren zur Herstellung eines Inokulums von Mykorrhiza-Pilzen, insbesondere eines Inokulums von arbuskulären Mykorrhiza-Pilzen, umfassend:
- einen Schritt des Co-Kultivierens der Pilze mit einem lebenden pflanzlichen Material, das als Wirtspflanze bezeichnet wird, und mindestens teilweise einem konstituierenden Teil der Wurzel einer Pflanze entspricht, die dafür geeignet ist, um eine Symbiose mit den genannten Pilzen zu bilden, und
- einen Schritt des Inberührungbringens einer Menge eines Peptids mit der im Vorstehenden gekannten Co-Kultur,
wobei das genannte Peptid eine Sequenz aufweist, die eine Sequenz umfasst oder daraus besteht, welche identisch ist mit jener eines miPEP, das natürlich in der genannten Pflanz vorkommt, wobei das genannte natürlich vorkommende miPEP ein Peptid ist, dessen Sequenz von einem offenen Leserahmen codiert wird, der in 5' auf dem primären Transkript einer miR angeordnet ist, wobei das genannte miPEP in der Lage ist, die Akkumulation der genannten miR zu modulieren, wobei die miR die Expression mindestens eines Gens reguliert, das in die Mykorrhiza-Symbiose bei der genannten Pflanze impliziert ist,
wobei das genannte Gen, das in die Mykorrhiza-Symbiose impliziert ist, einen Transkriptionsfaktor der Familie GRAS codiert,
wobei das genannte Gen, das in die Mykorrhiza-Symbiose impliziert ist, ausgewählt wird aus der Gruppe bestehend aus: *HAM1* und *HAM2,*
wobei die genannte miR die miR171b ist oder die miR eine Identität von mindestens 90 % mit der miR171b aufweisen oder die genannte miR die slmiR171e ist oder die miR eine Identität von mindestens 90 % mit der slmiR171e aufweisen.

16. Inokulum von Mykorrhiza-Pilzen, insbesondere ein Inokulum von arbuskulären Mykorrhiza-Pilzen, welches für die Inokulation einer Wirtspflanze geeignet ist, mindestens umfassend: einen Pilz und ein Peptid, dessen Sequenz eine Sequenz umfasst oder daraus besteht, welche identisch ist mit jener eines miPEP, das natürlich in der Wirtspflanze vorkommt,
wobei das genannte miPEP, das natürlich in der Wirtspflanze vorkommt, ein Peptid ist, dessen Sequenz von einem offenen Leserahmen codiert wird, der in 5' auf dem primären Transkript einer miR angeordnet ist, wobei das genannte miPEP in der Lage ist, die Akkumulation der genannten miR zu modulieren, wobei die miR die Expression mindestens eines Gens reguliert, das in die Mykorrhiza-Symbiose bei der genannten Wirtspflanze impliziert ist,
wobei das genannte Gen, das in die Mykorrhiza-Symbiose impliziert ist, einen Transkriptionsfaktor der Familie GRAS codiert,
wobei das genannte Gen, das in die Mykorrhiza-Symbiose impliziert ist, ausgewählt ist aus der Gruppe bestehend aus: *HAM1* und *HAM2,*
wobei die genannte miR die miR171b ist oder die miR eine Identität von mindestens 90 % mit der miR171b aufweisen oder die genannte miR die slmiR171e ist oder die miR eine Identität von mindestens 90 % mit der slmiR171e aufweisen.

## Claims

1. Use of a miPEP introduced externally in a plant for promoting mycorrhizal symbiosis, in particular the arbuscular mycorrhizal symbiosis, between said plant and a fungus, a said miPEP being also naturally present in said plant,
said miPEP introduced externally being a peptide comprising, or consisting of, a sequence identical to that of a naturally present miPEP,
said naturally present miPEP is a peptide of 3 to 100 amino acids, the sequence of which is encoded by an open reading frame situated at 5' on the primary transcript of a miR, said miPEP being capable of modulating the accumulation of said miR in said plant, which miR regulates the expression of at least one gene involved in the arbuscular mycorrhizal symbiosis,
the sum of the quantity of said miPEP introduced externally and that of said naturally present miPEP being strictly greater than the quantity of present naturally present miPEP,
said gene involved in the mycorrhizal symbiosis encoding a transcription factor of the GRAS family,
said gene involved in the mycorrhizal symbiosis being selected from the group consisting of: *HAM1* and *HAM2*
said miR being the miR171b or the miRs having at least 90% identity with the miR171b or said miR being the slmiR171e or the miRs having at least 90% identity with the slmiR171e.

2. Use according claim 1, wherein said miR is:
- miR171b, wherein said miR171b has a nucleotide sequence consisting of SEQ ID NO: 1,
- slmiR171e, wherein said slmiR171e has a nucleotide sequence consisting of SEQ ID NO: 5,
- ljmiR171b, in particular wherein said ljmiR171b has a nucleotide sequence consisting of SEQ ID NO: 9, or
- osmiR171i, in partciular wherein said osmiR171i has a nucleotide sequence consisting of SEQ ID NO: 13.

3. Use according to any one of the preceding claims, wherein said miPEP is:
- miPEP171b, in particular, wherein said miPEP171b has an amino acid sequence consisting of SEQ ID NO: 2,
- slmiPEP171e, in particular wherein said slmiPEP171e has an amino acid sequence consisting of SEQ ID NO: 6,
- ljmiPEP171b, in particular wherein said ljmiPEP171b has an amino acid sequence consisting of SEQ ID NO: 10, ou
- osmiPEP171i, in particular wherein said osmiPEP171i has an amino acid sequence consisting of SEQ ID NO: 14.

4. Use according to any one of the preceding claims, wherein said plant is a Monocotyledon plant, a Dicotyledon plant, a solanaceous plant or a leguminous plant, in particular selected from: *Medicago truncatula, Medicago sativa* (alfalfa), *Solanum lycopersicum* (tomato), *Lotus japonicus* (birdsfoot trefoil) or *Oryza sativa* (rice).

5. Use according to any one of the preceding claims, wherein said said fungus is a Glomeromycete or Basidiomycete or Ascomycete fungus, preferably Glomeromycete.

6. Use according to any one claims 1 to 5 for promoting the arbuscular mycorrhizal symbiosis:
between a *Medicago truncatula* plant and a glomeromycete fungus, in which the miPEP171b is introduced exogenously into said M. *truncatula* plant, said miPEP171b also being naturally present in said *M. truncatula* plant,
said miPEP171b introduced exogenously being a peptide, the sequence of which comprises or consists of a sequence identical to that of said naturally present miPEP171b, said sequence of the naturally present miPEP171b being encoded by an open reading frame situated at 5' on the primary transcript of the miR171b, which miR171b regulates the expression of at least one gene involved in the mycorrhizal symbiosis in *M. truncatula,*
the sum of the quantity of said miPEP171b introduced exogenously and that of said naturally present miPEP171b being strictly greater than the quantity of said miPEP171b naturally present in said *Medicago truncatula* plant,
or
between a *Solanum lycopersicum* plant and a glomeromycete fungus, in which the slmiPEP171e is introduced exogenously into said *Solanum lycopersicum* plant, said slmiPEP171e also being naturally present in said *Solanum lycopersicum* plant,
said slmiPEP171e introduced exogenously being a peptide, the sequence of which comprises or consists of a sequence identical to that of said naturally present slmiPEP171e, said sequence of the naturally present slmiPEP171e being encoded by an open reading frame situated at 5' on the primary transcript of the slmiR171e, said slmiR171e regulating the expression of at least one gene involved in the mycorrhizal symbiosis in *Solanum lycopersicum,*
the sum of the quantity of said slmiPEP171e introduced exogenously and that of said naturally present slmiPEP171e being strictly greater than the quantity of said slmiPEP171e naturally present in said *Solanum lycopersicum* plant,
or
between a *Lotus japonicus* plant and a glomeromycete fungus, in which the ljmiPEP171b is introduced exogenously into said plant *Lotus japonicus,* said ljmiPEP171b also being naturally present in said *Lotus japonicus* plant,
said ljmiPEP171b introduced exogenously being a peptide, the sequence of which comprises or consists of a sequence identical to that of said naturally present ljmiPEP171b, said sequence of the naturally present ljmiPEP171b being encoded by an open reading frame situated at 5' on the primary transcript of the ljmiR171b, said ljmiR171b regulating the expression of at least one gene involved in the mycorrhizal symbiosis in *Lotus japonicus,*
the sum of the quantity of said ljmiPEP171b introduced exogenously and that of said naturally present ljmiPEP171b being strictly greater than the quantity of said ljmiPEP171b naturally present in said *Lotus japonicus* plant,
or
between an *Oryza sativa* plant and a glomeromycete fungus, in which the osmiPEP171i is introduced exogenously into said *Oryza sativa* plant, said osmiPEP171i also being naturally present in said *Oryza sativa* plant,
said osmiPEP171i introduced exogenously being a peptide, the sequence of which comprises or consists of a sequence identical to that of said naturally present osmiPEP171i, said sequence of the naturally present osmiPEP171i being encoded by an open reading frame situated at 5' on the primary transcript of the osmiR171i, said miR171i regulating the expression of at least one gene involved in the mycorrhizal symbiosis in *Oryza sativa,*
the sum of the quantity of said osmiPEP171i introduced exogenously and that of said naturally present osmiPEP171i being strictly greater than the quantity of said osmiPEP171i naturally present in said plant *Oryza sativa*
said gene involved in the mycorrhizal symbiosis encoding a transcription factor of the GRAS family,
said gene involved in the mycorrhizal symbiosis being selected from the group consisting of: *HAM1* and *HAM2.*

7. Process for promoting the mycorrhizal symbiosis between a plant and a fungus, and in particular the arbuscular mycorrhizal symbiosis (AM), comprising a step of introducing a miPEP into a plant externally, said miPEP also being naturally present in said plant, said miPEP introduced exogenously being a peptide of 3 to 100 amino acids, the sequence of which comprises or consists of a sequence identical to that of said naturally present miPEP, said sequence of the naturally present miPEP being encoded by an open reading frame situated at 5' on the primary transcript of a miR, said miPEP being capable of modulating the accumulation of said miR, which miR regulates the expression of at least one gene involved in the mycorrhizal symbiosis in said plant, the sum of the quantity of said miPEP introduced externally and that of said naturally present miPEP being strictly greater than the quantity of the miPEP naturally present, said gene involved in the mycorrhizal symbiosis encoding a transcription factor of the GRAS family,
said gene involved in the mycorrhizal symbiosis being selected from the group consisting of: *HAM1* and *HAM2,*
said miR being the miR171b or the miRs having at least 90% identity with the miR171b or said miR being the slmiR171e or the miRs having at least 90% identity with the slmiR171e.

8. Process according claim 7, wherein said miPEP is introduced into the plant:
- externally, preferably by watering, by spraying or by adding a fertilizer, said miPEP being in particuliar administered to the plant in the form of a composition comprising 10⁻⁹ M to 10⁻⁴ M of said miPEP, in particular 10⁻⁹, 10⁻⁸, 10⁻⁷, 10⁻⁶, 10⁻⁵ or 10⁻⁴ M of said miPEP,
- externally, preferably by watering, by spraying or by adding a fertilizer, said miPEP being in particuliar administered to the plant in the form of a composition comprising 10⁻⁹ M to 10⁻⁴ M of said miPEP, in particular 10⁻⁹, 10⁻⁸, 10⁻⁷, 10⁻⁶, 10⁻⁵ or 10⁻⁴ M of said miPEP, or
- by means of a nucleic acid encoding said miPEP, said nucleic acid being introduced into the plant.

9. Process for the production of a transgenic plant comprising:
a) a step of introducing a nucleic acid encoding for a miPEP of 3 to 100 amino acids in said plant, said nucleic acid does not comprise the complete sequence of said miR, or in at least one cell of said plant, under conditions allowing the expression of said miPEP,
said miPEP also being naturally present in said plant, said naturally present miPEP is a peptide, the sequence of which is encoded by an open reading frame situated at 5' on the primary transcript of a miR, said miPEP being capable of modulating the accumulation of said miR in the plant, which miR regulates the expression of at least one gene involved in the mycorrhizal symbiosis, in particular the arbuscular mycorrhizal symbiosis, and
b) a step of growing the plant, or of at least one cell of said plant, obtained in step a) under conditions allowing a transgenic plant to be obtained,
said gene involved in the mycorrhizal symbiosis being selected from the group consisting of: *HAM1* and *HAM2,*
said miR being the miR171b or the miRs having at least 90% identity with the miR171b or said miR being the slmiR171e or the miRs having at least 90% identity with the slmiR171e.

10. Process for the production of a transgenic plant according to claim 9 wherein,
- said miPEP being the miPEP171b, said miPEP171b having in particular an amino acid sequence consisting of SEQ ID NO: 2,
- said miPEP being the slmiPEP171e, said slmiPEP171e having in particular an amino acid sequence consisting of SEQ ID NO: 6,
- said miPEP being the ljmiPEP171b, said ljmiPEP171b having in particular an amino acid sequence consisting of SEQ ID NO: 10, or
- said miPEP being the osmiPEP171i, said osmiPEP171i having in particular an amino acid sequence consisting of SEQ ID NO: 14.

11. Transgenic plant such as obtained by the process as defined according to claim 9 or 10.

12. Composition comprising:
- miPEP171b as the active ingredient, said miPEP171b preferably consisting of SEQ ID NO: 2, said miPEP171b is at a concentration of 10⁻⁹, 10⁻⁵ or 10⁻⁴ M,
- comprising slmiPEP171e as the active ingredient, said slmiPEP171e preferably consisting of SEQ ID NO: 6, said slmiPEP171e being in particular at a concentration of 10⁻⁹ M to 10⁻⁴ M, in particular 10⁻⁹, 10⁻⁸, 10⁻⁷, 10⁻⁶, 10⁻⁵ or 10⁻⁴ M,
- comprising ljmiPEP171b as the active ingredient, said ljmiPEP171b preferably consisting of SEQ ID NO: 10, said ljmiPEP171b being in particular at a concentration of 10⁻⁹ M to 10⁻⁴ M, in particular 10⁻⁹, 10⁻⁸, 10⁻⁷, 10⁻⁶, 10⁻⁵ or 10⁻⁴ M, or
- comprising osmiPEP171i as the active ingredient, said osmiPEP171i preferably consisting of SEQ ID NO: 14, said osmiPEP171i being in particular at a concentration of 10⁻⁹ M to 10⁻⁴ M, in particular 10⁻⁹, 10⁻⁸, 10⁻⁷, 10⁻⁶, 10⁻⁵ or 10⁻⁴ M,
- in particular, said composition further comprising an excipient, a diluent or a solvent,
- in particular, said composition being formulated so as to form a coating.

13. composition comprising, in combination, a quantity of seeds from a plant and a quantity of a peptide, the sequence of which comprises or consists of a sequence identical to that of a naturally present miPEP in said plant,
said peptide having in particular a sequence comprising or consisting of a sequence identical to that of miPEP171b, slmiPEP171e, LjmiPEP171b or osmiPEP171i,
in particular, said composition being formulated so as to form a coated seed.

14. Process for culturing mycorrhizal fungi, in particular arbuscular mycorrhizal fungi, comprising a step of contacting said fungi with:
- a mixture comprising a plant or a part of a plant, in particular a root culture, and a peptide, the sequence of which comprises or consists of a sequence identical to that of a naturally present miPEP in said plant, said naturally present miPEP is a peptide, the sequence of which is encoded by an open reading frame situated at 5' on the primary transcript of a miR, said miPEP being capable of modulating the accumulation of said miR, which miR regulates the expression of at least one gene involved in the mycorrhizal symbiosis in said plant, or
- with a transgenic plant as defined above,
the plant, the part of a plant, and the transgenic plant being suitable for forming a mycorrhizal symbiosis with said fungus,
said gene involved in the mycorrhizal symbiosis encoding a transcription factor of the GRAS family,
said gene involved in the mycorrhizal symbiosis being selected from the group consisting of: *HAM1* and *HAM2,*
said miR being the miR171b or the miRs having at least 90% identity with the miR171b or said miR being the slmiR171e or the miRs having at least 90% identity with the slmiR171e.

15. Process for producing inoculum of mycorrhizal fungi, in particular inoculum of arbuscular mycorrhizal fungi, comprising:
- a step of co-culturing fungi with a living plant material, called plant host, corresponding at least partially to a constitutive part of a plant root suitable for forming a symbiosis with said fungi, and
- a step of contacting a quantity of a peptide with the aforementioned co-culture, said peptide having a sequence comprising or consisting of a sequence identical to that of a naturally present miPEP in said plant host, said naturally present miPEP being a peptide, the sequence of which is encoded by an open reading frame situated at 5' on the primary transcript of a miR, said miPEP being capable of modulating the accumulation of said miR, which miR regulates the expression of at least one gene involved in the mycorrhizal symbiosis in said plant host
said gene involved in the mycorrhizal symbiosis encoding a transcription factor of the GRAS family,
said gene involved in the mycorrhizal symbiosis being selected from the group consisting of: *HAM1* and *HAM2,*
said miR being the miR171b or the miRs having at least 90% identity with the miR171b or said miR being the slmiR171e or the miRs having at least 90% identity with the slmiR171e.

16. Inoculum of mycorrhizal fungi, in particular an inoculum of arbuscular mycorrhizal fungi, suitable for the inoculation of a plant host, comprising at least one fungus and a peptide, the sequence of which comprises or consists of a sequence identical to that of a naturally present miPEP in the plant host,
said naturally present miPEP in the plant host being a peptide, the sequence of which is encoded by an open reading frame situated at 5' on the primary transcript of a miR, said miPEP being capable of modulating the accumulation of said miR, which miR regulates the expression of at least one gene involved in the mycorrhizal symbiosis in said plant host
said gene involved in the mycorrhizal symbiosis encoding a transcription factor of the GRAS family,
said gene involved in the mycorrhizal symbiosis being selected from the group consisting of: *HAM1* and *HAM2,*
said miR being the miR171b or the miRs having at least 90% identity with the miR171b or said miR being the slmiR171e or the miRs having at least 90% identity with the slmiR171e.
